(19) Europäisches Patentamt
European Patent Office
Office européen des brevets

(11) Publication number: **0 643 057 A1**

# (12) EUROPEAN PATENT APPLICATION

(21) Application number: **94113800.0**

(22) Date of filing: **02.09.94**

(51) Int. Cl.[6]: **C07D 401/04**, C07D 211/58, C07D 295/10, C07D 295/18, C07D 401/12, C07D 401/14, C07D 405/12, C07D 417/12, C07D 409/12, A61K 31/445

(30) Priority: **03.09.93 US 117362**

(43) Date of publication of application: **15.03.95 Bulletin 95/11**

(84) Designated Contracting States: **AT BE CH DE DK ES FR GB GR IE IT LI LU MC NL PT SE**

(71) Applicant: **BRISTOL-MYERS SQUIBB COMPANY**
**P.O. Box 4000**
**Princeton, NJ 08543-4000 (US)**

(72) Inventor: **Wetterau II, John R.**
**190 Rugby Drive**
**Langhorne, PA. (US)**
Inventor: **Sharp, Daru Young**
**893 Perrineville Road**
**Perrineville NJ (US)**
Inventor: **Gregg, Richard E.**
**7 Linden Lane**
**Pennington NJ (US)**
Inventor: **Biller, Scott A.**
**136 Nancy Lane**
**Pennington NJ (US)**
Inventor: **Dickson, John K.**
**105 Dawn Drive**
**Mount Holly NJ (US)**
Inventor: **Lawrence, Michael R.**
**48 W. Crown Terrace**
**Yardley PA (US)**
Inventor: **Lawson, John E.**
**5 Old Pasture Court**
**Wallingford CT (US)**
Inventor: **Holava, Henry M.**
**13 Meadow Way**
**Meriden, CT., (US)**
Inventor: **Partyka, Richard A.**
**618 Van Liew Court**
**Neshanic, NJ., (US)**

(74) Representative: **VOSSIUS & PARTNER**
**Siebertstrasse 4**
**D-81675 München (DE)**

## (54) Inhibitors of microsomal triglyceride transfer protein.

(57) Compounds are provided which inhibit microsomal triglyceride transfer protein and thus are useful for lowering serum lipids and treating atherosclerosis and related diseases. The compounds have the structure

EP 0 643 057 A1

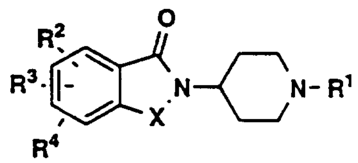 , 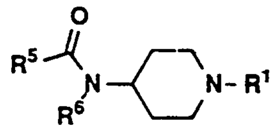 or

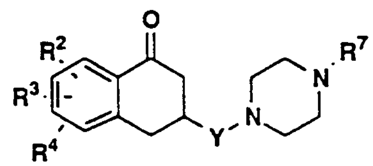

wherein $R^1$ to $R^7$, X and Y are as defined herein.

This invention relates to novel compounds which inhibit microsomal triglyceride transfer protein, and to pharmaceutical compositions decreasing serum lipids and treating atherosclerosis comprising such compounds.

The microsomal triglyceride transfer protein (MTP) catalyzes the transport of triglyceride (TG), cholesteryl ester (CE), and phosphatidylcholine (PC) between small unilamellar vesicles (SUV). Wetterau & Zilversmit, Chem. Phys. Lipids 38, 205-22 (1985). When transfer rates are expressed as the percent of the donor lipid transferred per time, MTP expresses a distinct preference for neutral lipid transport (TG and CE), relative to phospholipid transport. The protein from bovine liver has been isolated and characterized. Wetterau & Zilversmit, Chem. Phys. Lipids 38, 205-22 (1985). Polyacrylamide gel electrophoresis (PAGE) analysis of the purified protein suggests that the transfer protein is a complex of two subunits of apparent molecular weights 58,000 and 88,000, since a single band was present when purified MTP was electrophoresed under nondenaturing condition, while two bands of apparent molecular weights 58,000 and 88,000 were identified when electrophoresis was performed in the presence of sodium dodecyl sulfate (SDS). These two polypeptides are hereinafter referred to as 58 kDa and 88 kDa, respectively, or the 58 kDa and the 88 kDa component of MTP, respectively, or the low molecular weight subunit and the high molecular weight subunit of MTP, respectively.

Characterization of the 58,000 molecular weight component of bovine MTP indicates that it is the previously characterized multifunctional protein, protein disulfide isomerase (PDI). Wetterau et al., J. Biol. Chem. 265, 9800-7 (1990). The presence of PDI in the transfer protein is supported by evidence showing that (1) the amino terminal 25 amino acids of the bovine 58,000 kDa component of MTP is identical to that of bovine PDI, and (2) disulfide isomerase activity was expressed by bovine MTP following the dissociation of the 58 kDa - 88 kDa protein complex. In addition, antibodies raised against bovine PDI, a protein which by itself has no TG transfer activity, were able to immunoprecipitate bovine TG transfer activity from a solution containing purified bovine MTP.

PDI normally plays a role in the folding and assembly of newly synthesized disulfide bonded proteins within the lumen of the endoplasmic reticulum. Bulleid & Freedman, Nature 335, 649-51 (1988). It catalyzes the proper pairing of cysteine residues into disulfide bonds, thus catalyzing the proper folding of disulfide bonded proteins. In addition, PDI has been reported to be identical to the beta subunit of human prolyl 4-hydroxylase. Koivu et al., J. Biol. Chem. 262, 6447-9 (1987). The role of PDI in the bovine transfer protein is not clear. It does appear to be an essential component of the transfer protein as dissociation of PDI from the 88 kDa component of bovine MTP by either low concentrations of a denaturant (guanidine HCl), a chaotropic agent (sodium perchlorate), or a nondenaturing detergent (octyl glucoside) results in a loss of transfer activity. Wetterau et al., Biochemistry 30, 9728-35 (1991). Isolated bovine PDI has no apparent lipid transfer activity, suggesting that either the 88 kDa polypeptide is the transfer protein or that it confers transfer activity to the protein complex.

The tissue and subcellular distribution of MTP activity in rats has been investigated. Wetterau & Zilversmit, Biochem. Biophys. Acta 875, 610-7 (1986). Lipid transfer activity was found in liver and intestine. Little or no transfer activity was found in plasma, brain, heart, or kidney. Within the liver, MTP was a soluble protein located within the lumen of the microsomal fraction. Approximately equal concentrations were found in the smooth and rough microsomes.

Abetalipoproteinemia is an autosomal recessive disease characterized by a virtual absence of plasma lipoproteins which contain apolipoprotein B (apoB). Kane & Havel in The Metabolic Basis of Inherited Disease, Sixth edition, 1139-64 (1989). Plasma TG levels may be as low as a few mg/dL, and they fail to rise after fat ingestion. Plasma cholesterol levels are often only 20-45 mg/dL. These abnormalities are the result of a genetic defect in the assembly and/or secretion of very low density lipoproteins (VLDL) in the liver and chylomicrons in the intestine. The molecular basis for this defect has not been previously determined. In subjects examined, triglyceride, phospholipid, and cholesterol synthesis appear normal. At autopsy, subjects are free of atherosclerosis. Schaefer et al., Clin. Chem. 34, B9-12 (1988). A link between the apoB gene and abetalipoproteinemia has been excluded in several families. Talmud et al., J. Clin. Invest. 82, 1803-6 (1988) and Huang et al., Am. J. Hum. Genet. 46, 1141-8 (1990).

Subjects with abetalipoproteinemia are afflicted with numerous maladies. Kane & Havel, supra. Subjects have fat malabsorption and TG accumulation in their enterocytes and hepatocytes. Due to the absence of TG-rich plasma lipoproteins, there is a defect in the transport of fat-soluble vitamins such as vitamin E. This results in acanthocytosis of erythrocytes, spinocerebellar ataxia with degeneration of the fasciculus cuneatus and gracilis, peripheral neuropathy, degenerative pigmentary retinopathy, and ceroid myopathy. Treatment of abetalipoproteinemic subjects includes dietary restriction of fat intake and dietary supplementation with vitamins A, E and K.

To date, the physiological role of MTP has not been demonstrated. In vitro, it catalyzes the transport of lipid molecules between phospholipid membranes. Presumably, it plays a similar role in vivo, and thus plays some role in lipid metabolism. The subcellular (lumen of the microsomal fraction) and tissue distribution (liver and intestine) of MTP have led to speculation that it plays a role in the assembly of plasma lipoproteins, as these are the sites of plasma lipoprotein assembly. Wetterau & Zilversmit, Biochem. Biophys. Acta 875, 610-7 (1986). The ability of MTP to catalyze the transport of TG between membranes is consistent with this hypothesis, and suggests that MTP may catalyze the transport of TG from its site of synthesis in the endoplasmic reticulum (ER) membrane to nascent lipoprotein particles within the lumen of the ER.

Olofsson and colleagues have studied lipoprotein assembly in HepG2 cells. Bostrom et al., J. Biol. Chem, 263, 4434-42 (1988). Their results suggest small precursor lipoproteins become larger with time. This would be consistent with the addition or transfer of lipid molecules to nascent lipoproteins as they are assembled. MTP may play a role in this process. In support of this hypothesis, Howell and Palade, J. Cell Biol. 92, 833-45 (1982), isolated nascent lipoproteins from the hepatic Golgi fraction of rat liver. There was a spectrum of sizes of particles present with varying lipid and protein compositions. Particles of high density lipoprotein (HDL) density, yet containing apoB, were found. Higgins and Hutson, J. Lipid Res. 25, 1295-1305 (1984), reported lipoproteins isolated from Golgi were consistently larger than those from the endoplasmic reticulum, again suggesting the assembly of lipoproteins is a progressive event. However, there is no direct evidence in the prior art demonstrating that MTP plays a role in lipid metabolism or the assembly of plasma lipoprotein.

Canadian Patent Application No. 2,091,102 published March 2, 1994 (corresponding to U.S. application Serial No. 117,362, filed September 3, 1993 (file DC21b)) which is incorporated herein by reference, discloses a method for identifying the MTP inhibitors

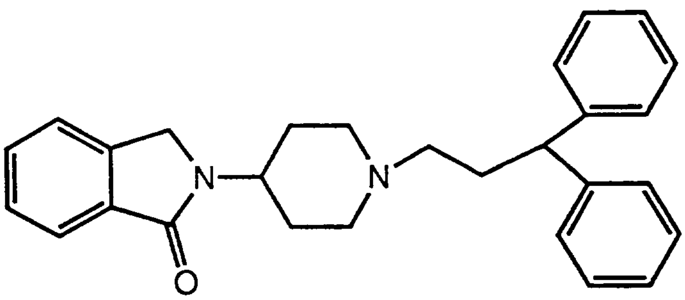

which has the name 2-[1-(3,3-diphenylpropyl)-4-piperidinyl]-2,3-dihydro-3-oxo-1H-isoindole hydrochloride and

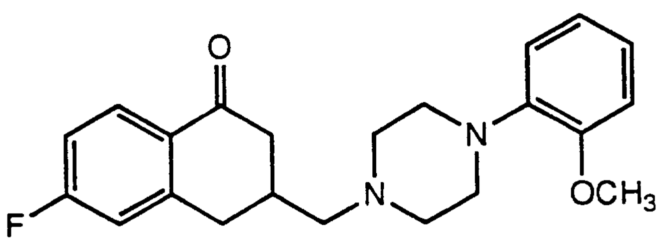

which has the name 1-[3-(6-fluoro-1-tetralanyl)methyl]-4-O-methoxyphenyl piperazine

In accordance with the present invention, novel compounds are provided which are inhibitors of MTP and have the structure

I

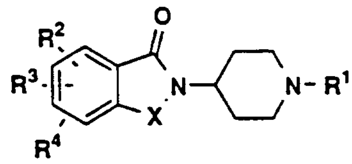 ;

or

II

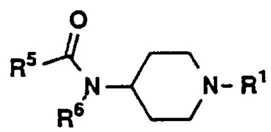 ;

or

III

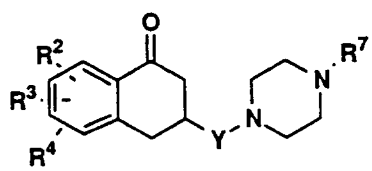

where X is: $CHR^8$,

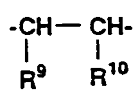 or 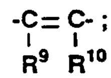

$R^8$, $R^9$ and $R^{10}$ are independently hydrogen, alkyl, alkenyl, alkynyl, aryl, arylalkyl, heteroaryl, heteroarylalkyl, cycloalkyl, or cycloalkylalkyl;

Y is $-(CH_2)_m-$ or

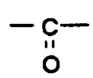

where m is 2 or 3;

$R^1$ is alkyl, alkenyl, alkynyl, aryl, heteroaryl, arylalkyl (wherein alkyl has at least 2 carbons), diarylalkyl, arylalkenyl, diarylalkenyl, arylalkynyl, diarylalkynyl, diarylalkylaryl, heteroarylalkyl (wherein alkyl has at least 2 carbons), cycloalkyl, or cycloalkylalkyl (wherein alkyl has at least 2 carbons); all of the aforementioned $R^1$ groups being optionally substituted through available carbon atoms with 1, 2, or 3 groups selected from halo, haloalkyl, alkyl, alkenyl, alkoxy, aryloxy, aryl, arylalkyl, alkylmercapto, arylmercapto, cycloalkyl, cycloalkylalkyl, heteroaryl, fluorenyl, heteroarylalkyl, hydroxy or oxo; or

$R^1$ is a group of the structure

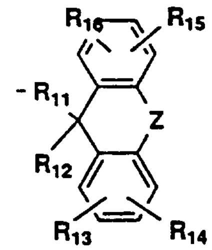

$R^{11}$ is a bond, alkylene, alkenylene or alkynylene of up to 6 carbon atoms, arylene (for example

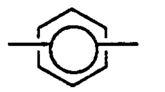 )

,

or mixed arylene-alkylene (for example

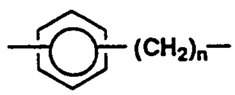 )

where n is 1 to 6;

$R^{12}$ is hydrogen, alkyl, alkenyl, aryl, heteroaryl, haloalkyl, arylalkyl, arylalkenyl, cycloalkyl, aryloxy, alkoxy, arylalkoxy, heteroarylalkyl or cycloalkylalkyl;

Z is a bond, O, S, N-alkyl, N-aryl, or alkylene or alkenylene of from 1 to 5 carbon atoms;

$R^{13}$, $R^{14}$, $R^{15}$, and $R^{16}$ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, carboxy, aminocarbonyl, alkylcarbonyloxy, alkylcarbonylamino, arylalkyl, heteroaryl, heteroarylalkyl, or aryloxy;

or $R^1$ is

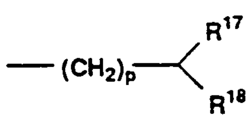

wherein p is 1 to 8 and $R^{17}$ and $R^{18}$ are each independently H, alkyl, alkenyl, aryl, arylalkyl, heteroaryl, heteroarylalkyl, cycloalkyl or cycloalkylalkyl, at least one of $R^{17}$ and $R^{18}$ being other than H;

or $R^1$ is

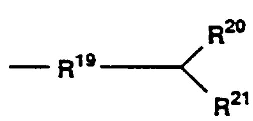

wherein

$R^{19}$ is aryl or heteroaryl;

$R^{20}$ is aryl or heteroaryl;

$R^{21}$ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;

$R^2$, $R^3$, $R^4$ are independently hydrogen, halo, alkyl, haloalkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl;

$R^5$ is alkyl of at least 2 carbons, alkenyl, alkynyl, aryl, heteroaryl, arylalkyl, heteroarylalkyl, cycloalkyl, cycloalkylalkyl, polycycloalkyl, polycycloalkylalkyl, cycloalkenyl, cycloalkenylalkyl, polycycloalkenyl, polycycloalkenylalkyl, heteroarylcarbonyl, all of the $R^5$ and $R^6$ substituents being optionally substituted through

available carbon atoms with 1, 2, or 3 groups selected from hydrogen, halo, alkyl, haloalkyl, alkoxy, haloalkoxy, alkenyl, alkynyl, cycloalkyl, cycloalkylalkyl, cycloheteroalkyl, cycloheteroalkylalkyl, aryl, heteroaryl, arylalkyl, arylcycloalkyl, arylalkynyl, aryloxy, aryloxyalkyl, arylalkoxy, arylazo, heteroaryloxo, heteroarylalkyl, heteroarylalkenyl, heteroaryloxy, hydroxy, nitro, cyano, amino, substituted amino (wherein the amino includes 1 or 2 substituents which are alkyl, or aryl or any of the other aryl compounds mentioned in the definitions), thiol, alkylthio, arylthio, heteroarylthio, arylthioalkyl, alkylcarbonyl, arylcarbonyl, arylaminocarbonyl, alkoxycarbonyl, aminocarbonyl, alkynylaminocarbonyl, alkylaminocarbonyl, alkenylaminocarbonyl, alkylcarbonyloxy, arylcarbonyloxy, alkylcarbonylamino, arylcarbonylamino, arylsulfinyl, arylsulfinylalkyl, arylsulfonyl, alkylsulfonyl, arylsulfonylamino; with the proviso that when $R^5$ is $CH_3$, $R^6$ is not H; and where $R^5$ is phenyl, the phenyl preferably includes an ortho hydrophobic substituent such as alkyl, haloalkyl, aryl, aryloxy or arylalkyl;

$R^6$ is hydrogen or $C_1$-$C_4$ alkyl or $C_1$-$C_4$ alkenyl;

$R^7$ is alkyl, aryl or arylalkyl wherein alkyl or the alkyl portion is optionally substituted with oxo; and

including pharmaceutically acceptable salts thereof such as alkali metal salts such as lithium sodium or potassium, alkaline earth metal salts such as calcium or magnesium, as well as zinc or aluminum and other cations such as ammonium, choline, diethanolamine, ethylenediamine, t-butylamine, t-octylamine, dehydroabietylamine, as well as pharmaceutically acceptable anions such as chloride, bromide, iodide, tartrate, acetate, methanesulfonate, maleate, succinate, glutarate, and salts of naturally occurring amino acids such as arginine, lysine, alanine and the like, and prodrug esters thereof.

In the formula I compounds, where X is $CH_2$ and $R^2$, $R^3$ and $R^4$ are each H, $R^1$ will be other than 3,3-diphenylpropyl.

In the formula III compounds, where one of $R^2$, $R^3$ and $R^4$ is 6-fluoro, and the others are H, $R^7$ will be other than 4-O-methoxyphenyl.

Thus, the compounds of formula I of the invention encompass compounds of the structure

Ia

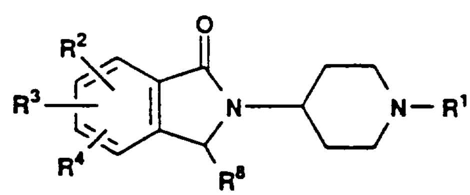

Ib

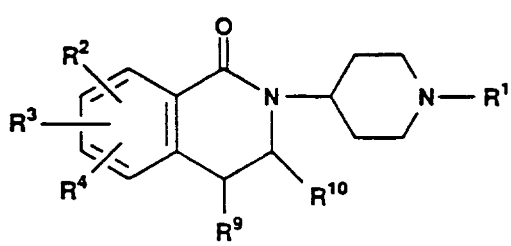

Ic

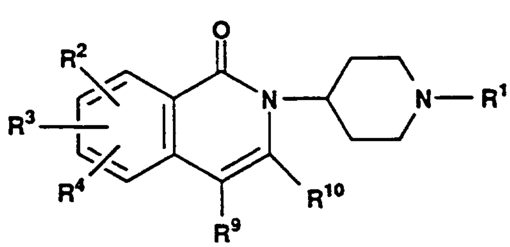

The compounds of formula III of the invention encompass compounds of the structure

IIIa

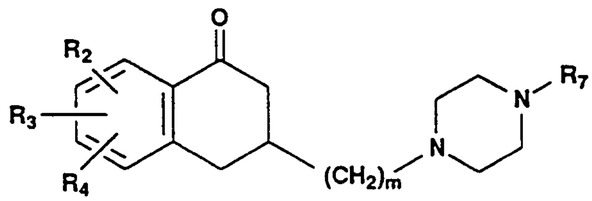

IIIb

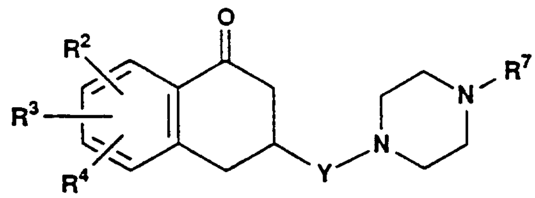

In addition, in accordance with the present invention the use of a compound of formula I, II or III as defined hereinbefore wherein $R^1$ also includes arylmethyl, heteroarylmethyl and cycloalkylmethyl and Y also includes $-CH_2$-for the preparation of a pharmaceutical composition for preventing, inhibiting or treating atherosclerosis, pancreatitis or obesity is provided by decreasing the activity of microsomal triglyceride transfer protein.

Furthermore, in accordance with the present invention, the use of a compound of formula I, II or III as defined hereinbefore wherein $R^1$ also includes arylmethyl, heteroarylmethyl, and cycloalkylmethyl, and Y also includes - $CH_2$-for the preparation of a pharmaceutical composition is provided for lowering serum lipid levels, cholesterol and/or triglycerides, or inhibiting and/or treating hyperlipemia, hyperlipidemia, hyperlipoproteinemia, hypercholesterolemia and/or hypertriglyceridemia, by decreasing the activity of microsomal triglyceride transfer protein.

The following definitions apply to the terms as used throughout this specification, unless otherwise limited in specific instances.

The term "MTP" refers to a polypeptide or protein complex that (1) if obtained from an organism (e.g., cows, humans, etc.), can be isolated from the microsomal fraction of homogenized tissue; and (2) stimulates the transport of triglycerides, cholesterol esters, or phospholipids from synthetic phospholipid vesicles, membranes or lipoproteins to synthetic vesicles, membranes, or lipoproteins and which is distinct from the cholesterol ester transfer protein [Drayna et al., Nature 327, 632-634 (1987)] which may have similar catalytic properties. However, the MTP molecules of the present invention do not necessarily need to be catalytically active. For example, catalytically inactive MTP or fragments thereof may be useful in raising antibodies to the protein.

The phrase "stabilizing" atherosclerosis as used in the present application refers to slowing down the development of and/or inhibiting the formation of new atherosclerotic lesions.

The phrase "causing the regression of" atherosclerosis as used in the present application refers to reducing and/or eliminating atherosclerotic lesions.

Unless otherwise indicated, the term "lower alkyl", "alkyl" or "alk" as employed herein alone or as part of another group includes both straight and branched chain hydrocarbons, containing 1 to 40 carbons, preferably 1 to 20 carbons, more preferably 1 to 12 carbons, in the normal chain,such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1 to 4 substituents such as halo, for example F, Br, Cl or I or $CF_3$, alkoxy, aryl, aryloxy, aryl(aryl) or diaryl, arylalkyl, arylalkyloxy, alkenyl, cycloalkyl, cycloalkylalkyl, cycloalkylalkyloxy, amino, hydroxy, acyl, heteroaryl, heteroaryloxy, heteroarylalkyl, heteroarylalkoxy, aryloxyalkyl, aryloxyaryl, alkylamido, alkanoylamino, arylcarbonylamino, nitro, cyano, thiol, haloalkyl, and/or alkylthio, as well as any of the other substituents as defined for $R^1$, $R^5$ and $R^6$.

Unless otherwise indicated, the term "cycloalkyl" as employed herein alone or as part of another group includes saturated or partially unsaturated (containing 1 or 2 double bonds) cyclic hydrocarbon groups containing 1 to 3 rings, including monocyclicalkyl, bicyclicalkyl and tricyclicalkyl, containing a total of 3 to 20 carbons forming the rings, preferably 4 to 12 carbons, forming the ring and which may be fused to 1 aromatic ring as described for aryl, which include cyclopropyl, cyclobutyl, cyclopentyl, cyclohexyl, cycloheptyl, cyclooctyl, cyclodecyl and cyclododecyl, cyclohexenyl,

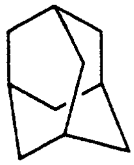 , 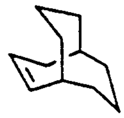 , 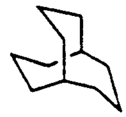 , 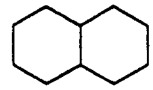 ,

any of which groups may be optionally substituted with 1 to 4 substituents such as halogen, alkyl, alkoxy, hydroxy, aryl, aryloxy, arylalkyl, cycloalkyl, alkylamido, alkanoylamino, oxo, acyl, arylcarbonylamino, amino, nitro, cyano, thiol and/or alkylthio, as well as any of the other substituents as defined for $R^1$, $R^5$ or $R^6$.

The term "cycloalkenyl" as employed herein alone or as part of another group refers to cyclic hydrocarbons containing 5 to 20 carbons, preferably 6 to 12 carbons and 1 or 2 double bonds. Exemplary cycloalkenyl groups include cyclopentenyl, cyclohexenyl, cycloheptenyl, cyclooctenyl, cyclohexadienyl, and cycloheptadienyl, which may be optionally substituted as defined for cycloalkyl.

The term "polycycloalkyl" as employed herein alone or as part of another group refers to a bridged multicyclic group containing 5 to 20 carbons and containing 0 to 3 bridges, preferably 6 to 12 carbons and 1 or 2 bridges. Exemplary polycycloalkyl groups include [3.3.0]-bicyclooctanyl, adamantanyl, [2.2.1]-bicycloheptanyl, [2.2.2]-bicyclooctanyl and the like and may be optionally substituted as defined for cycloalkyl.

The term "polycycloalkenyl" as employed herein alone or as part of another group refers to a bridged multicyclic group containing 5 to 20 carbons and containing 0 to 3 bridges and containing 1 or 2 double bonds, preferably 6 to 12 carbons and 1 or 2 bridges. Exemplary polycycloalkyl groups include [3.3.0]-bicyclooctenyl, [2.2.1]-bicycloheptenyl, [2.2.2]-bicyclooctenyl and the like and may be optionally substituted as defined for cycloalkyl.

The term "aryl" or "Ar" as employed herein alone or as part of another group refers to monocyclic and bicyclic aromatic groups containing 6 to 10 carbons in the ring portion (such as phenyl or naphthyl) and may optionally include one to three additional rings fused to Ar (such as aryl, cycloalkyl, heteroaryl or cycloheteroalkyl rings) and may be optionally substituted through available carbon atoms with 1, 2, or 3 groups selected from hydrogen, halo, haloalkyl, alkyl, haloalkyl, alkoxy, haloalkoxy, alkenyl, trifluoromethyl, trifluoromethoxy, alkynyl, cycloalkylalkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl or any of the other aryl compounds mentioned in the definitions), thiol, alkylthio, arylthio, heteroarylthio, arylthioalkyl, alkoxyarylthio, alkylcarbonyl, arylcarbonyl, alkylaminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino or arylsulfonaminocarbonyl.

The term "aralkyl", "aryl-alkyl" or "aryllower alkyl" as used herein alone or as part of another group refers to alkyl groups as discussed above having an aryl substituent, such as benzyl or phenethyl, or naphthylpropyl, or an aryl as defined above.

The term "lower alkoxy", "alkoxy", "aryloxy" or "aralkoxy" as employed herein alone or as part of another group includes any of the above alkyl, aralkyl or aryl groups linked to an oxygen atom.

The term "amino" as employed herein alone or as part of another group may optionally be substituted with one or two substituents such as alkyl and/or aryl.

The term "lower alkylthio", alkylthio", "arylthio" or "aralkylthio" as employed herein alone or as part of another group includes any of the above alkyl, aralkyl or aryl groups linked to a sulfur atom.

The term "lower alkylamino", "alkylamino", "arylamino", or "arylalkylamino" as employed herein alone or as part of another group includes any of the above alkyl, aryl or arylalkyl groups linked to a nitrogen atom.

The term "acyl" as employed herein by itself or part of another group refers to alkyl, alkenyl, aryl or aralkyl, as defined herein, each linked to a carbonyl

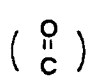

group.

The term "alkanoyl" as used herein alone or as part of another group refers to alkyl linked to a carbonyl group.

Unless otherwise indicated, the term "lower alkenyl" or "alkenyl" as used herein by itself or as part of another group refers to straight or branched chain radicals of 2 to 20 carbons, preferably 3 to 12 carbons, and more preferably 1 to 8 carbons in the normal chain, which include one to six double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, 4,8,l2-tetradecatrienyl, and the like, and which may be optionally substituted with l to 4 substituents, namely, halogen, haloalkyl, alkyl, alkoxy, alkenyl, alkynyl, aryl, arylalkyl, cycloalkyl, amino, hydroxy, heteroaryl, cycloheteroalkyl, alkanoylamino, alkylamido, arylcarbonylamino, nitro, cyano, thiol and/or alkylthio, as well as any of the other substituents as defined for $R^1$, $R^5$ or $R^6$.

Unless otherwise indicated, the term "lower alkynyl" or "alkynyl" as used herein by itself or as part of another group refers to straight or branched chain radicals of 2 to 20 carbons, preferably 2 to 12 carbons and more preferably 2 to 8 carbons in the normal chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl,3-undecynyl, 4-dodecynyl and the like, and which may be optionally substituted with l to 4 substituents, namely, halogen, haloalkyl, alkyl, alkoxy, alkenyl, alkynyl, aryl, arylalkyl, cycloalkyl, amino, heteroaryl, cycloheteroalkyl, hydroxy, alkanoylamino, alkylamido, arylcarbonylamino, nitro, cyano, thiol, and/or alkylthio, as well as any of the other substituents as defined for $R^1$, $R^5$ or $R^6$.

The term "alkylene" as employed herein alone or as part of another group refers to alkyl groups as defined above having single bonds for attachment to other groups at two different carbon atoms and may optionally be substituted as defined above for "alkyl".

Ther terms "alkenylene" and "alkynylene" as employed herein alone or as part of another group refer to alkenyl groups as defined above and alkynyl groups as defined above, respectively, having single bonds for attachment at two different carbon atoms.

Suitable alkylene, alkenylene or alkynylene groups or $(CH_2)_m$, $(CH_2)_n$ or $(CH_2)_p$ (which may include alkylene, alkenylene or alkynylene groups) as defined herein, may optionally include 1,2, or 3 alkyl, alkoxy, aryl, heteroaryl, cycloheteroalkyl, alkenyl, alkynyl, aryloxy, hydroxy, halogen substituents as well as any of the substituents defined for $R^1$, $R^5$ or $R^6$.

Examples include

$-CH=CH-CH_2-$, $-CH_2CH=CH-$, $-C\equiv C-CH_2-$, $-CH_2C\equiv CCH_2-$,

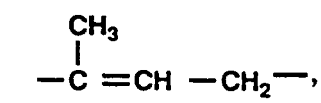

$-(CH_2)_2-$, $-(CH_2)_3-$, $-(CH_2)_4-$,

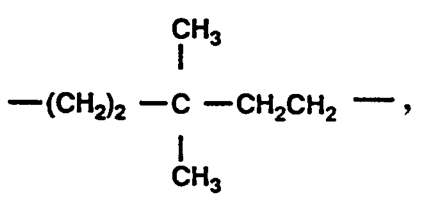

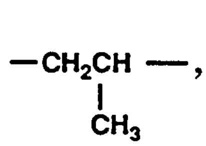

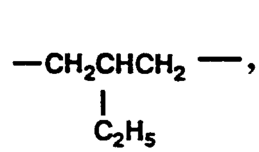

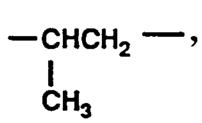

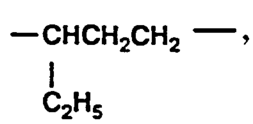

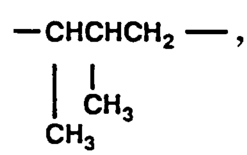

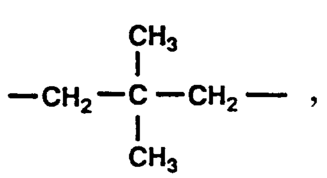

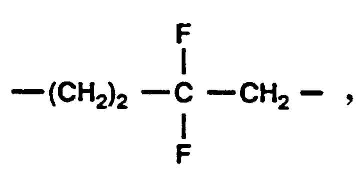

$-(CH_2)_5-$,

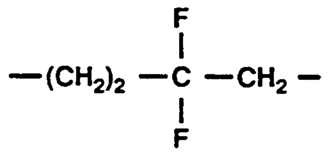,

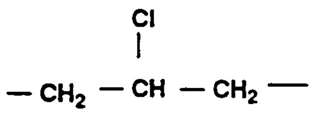, 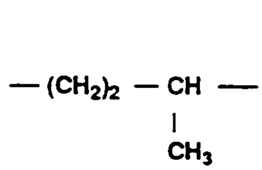, 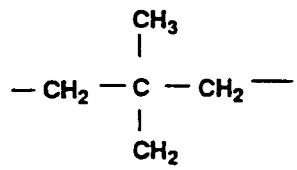,

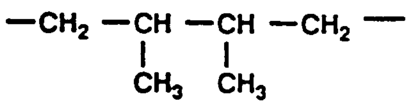, 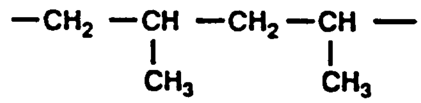,

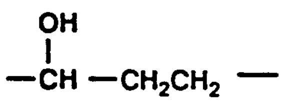, 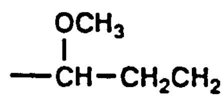,

$-CH_2OCH_2-$, $-OCH_2CH_2-$, $-CH_2NHCH_2-$, $-NHCH_2CH_2-$,

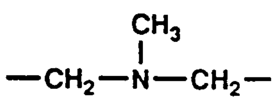, or 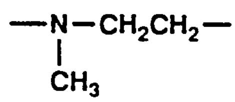.

The term "halogen" or "halo" as used herein alone or as part of another group refers to chlorine, bromine, fluorine, and iodine as well as $CF_3$, with chlorine or fluorine being preferred.

The term "metal ion" refers to alkali metal ions such as sodium, potassium or lithium and alkaline earth metal ions such as magnesium and calcium, as well as zinc and aluminum.

The term "cycloheteroalkyl" as used herein alone or as part of another group refers to a 5-, 6- or 7-membered saturated or partially unsaturated ring which includes 1 to 2 hetero atoms such as nitrogen, oxygen and/or sulfur, linked through a carbon atom or a heteroatom, where possible, optionally via the linker $(CH_2)_p$ (which is defined above), such as

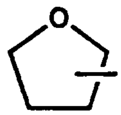 , 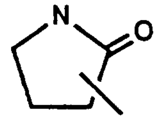 , 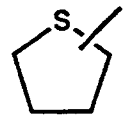 ,

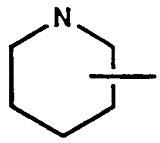 , 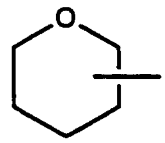 ,

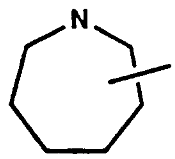 , 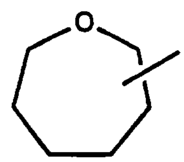

and the like. The above groups may include l to 3 substituents such as any of the $R^1$, $R^5$ or $R^6$ groups as defined above. In addition, any of the above rings can be fused to a cycloalkyl, aryl, heteroaryl or cycloheteroalkyl ring.

The term "heteroaryl" as used herein alone or as part of another group refers to a 5- or 6- membered aromatic ring which includes 1, 2, 3 or 4 hetero atoms such as nitrogen, oxygen or sulfur,and such rings fused to an aryl, cycloalkyl, heteroaryl or cycloheteroalkyl ring (e.g. benzothiophenyl, indolyl), linked through a carbon atom or a heteroatom, where possible, optionally via the linker $(CH_2)_p$ (which is defined above), such as

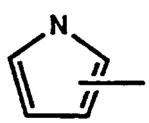 , 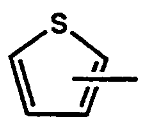 , 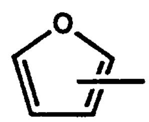 ,

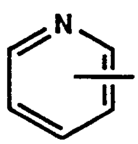 , 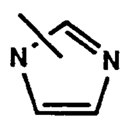 ,

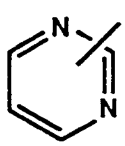 , 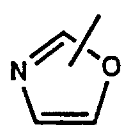 , 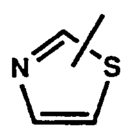

and the like. The heteroaryl groups including the above groups may optionally include l to 3 substituents such as any of the $R^1$, $R^5$ or $R^6$ groups as defined above. In addition, any of the above rings can be fused to a cycloalkyl, aryl, heteroaryl or cycloheteroalkyl ring.

The term cycloheteroalkylalkyl" as used herein alone or as part of another group refers to cycloheteroalkyl groups as defined above linked through a C atom or heteroatom to a $(CH_2)_p$ chain.

The term "heteroarylalkyl" or "heteroarylalkenyl" as used herein alone or as part of another group refers to a heteroaryl group as defined above linked through a C atom or heteroatom to a $-(CH_2)_p-$ chain, alkylene or alkenylene as defined above.

Preferred are compounds of formulae I and II wherein

$R^1$ is arylalkyl, arylalkenyl, heteroarylalkyl, heteroarylalkenyl,

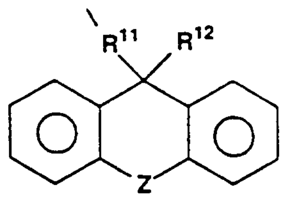

(where $R^{11}$ is alkylene or alkenylene, $R^{12}$ is H, alkyl, alkenyl, aralkyl, aralkenyl, Z is O or a bond); or

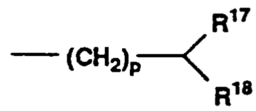

(wherein $R^{17}$ and $R^{18}$ are each independently alkyl, alkenyl, aryl, arylalkyl, heteroaryl, heteroarylalkyl, cycloalkyl or cycloalkylalkyl); or

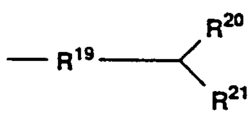

wherein

$R^{19}$ is aryl or heteroaryl;

$R^{20}$ is aryl or heteroaryl;

$R^{21}$ is alkyl, aryl, alkylaryl, arylalkyl aryloxy, arylalkoxy, heteroaryl, heteroarylalkyl, heteroarylalkoxy, cycloalkyl, cycloalkylalkyl or cycloalkylalkoxy.

In structure I, it is preferred that $R^2$, $R^3$ and $R^4$ are each H and X is $CH_2$, $CH_2CH_2$, or $CH=CH$.

In structure II, it is preferred that $R^6$ is H and $R^5$ is cycloalkyl or phenyl having an ortho hydrophobic substituent which is alkyl, alkoxy, haloalkyl, aryl, aryloxy, arylalkyl or arylalkoxy.

In structure II, it is also preferred that $R^1$ is arylalkyl or heteroarylalkyl wherein alkyl of each has at least 2 carbons and $R^5$ and $R^6$ may be as defined hereinbefore and may or may not be the preferred groups set out above.

In structure III, it is preferred that Y is $CH_2$, $R^2$, $R^3$ and $R^4$ are each H or halo and $R^7$ is aryl.

The compounds of formulae I, II, III and IV may be prepared by the exemplary processes described in the following reaction schemes. Exemplary reagents and procedures for these reactions appear hereinafter and in the working Examples.

Scheme I. Routes to Isoindolinone Piperidines I

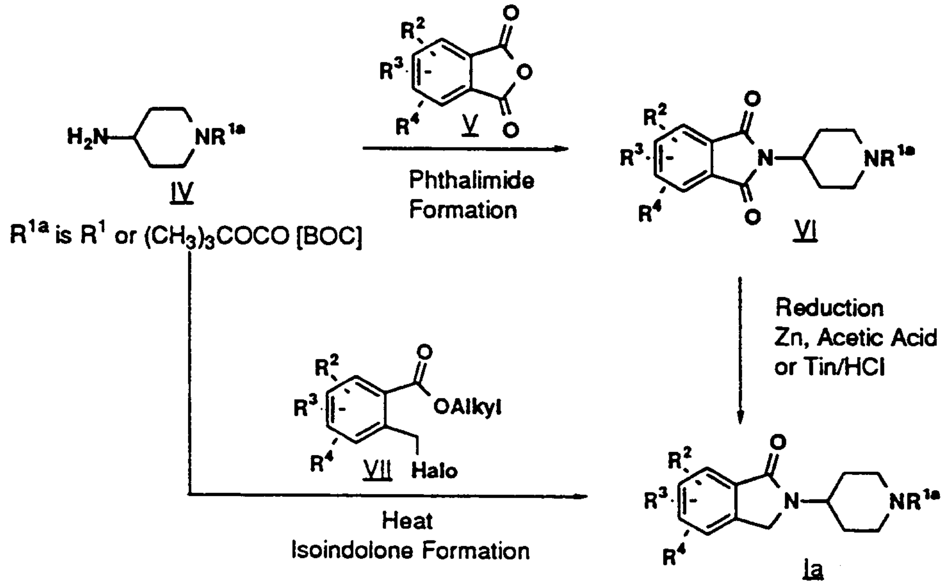


Scheme II. Additional Routes to Isoindolinone Piperidines I

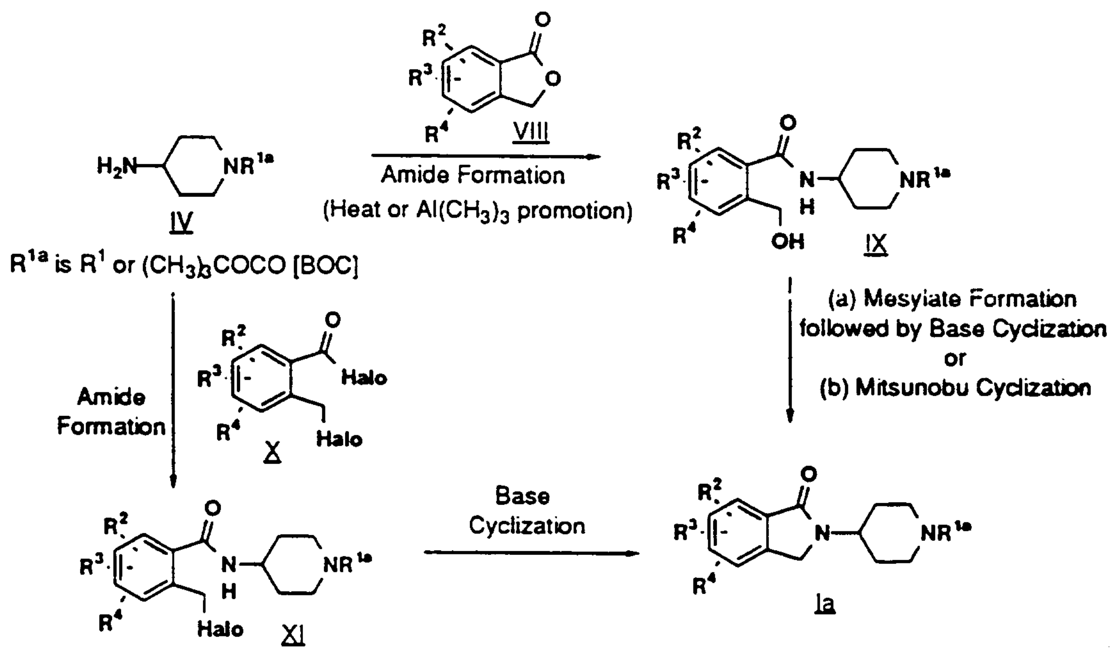

Scheme III. Introduction of $R^1$ by Alkylation or Arylation

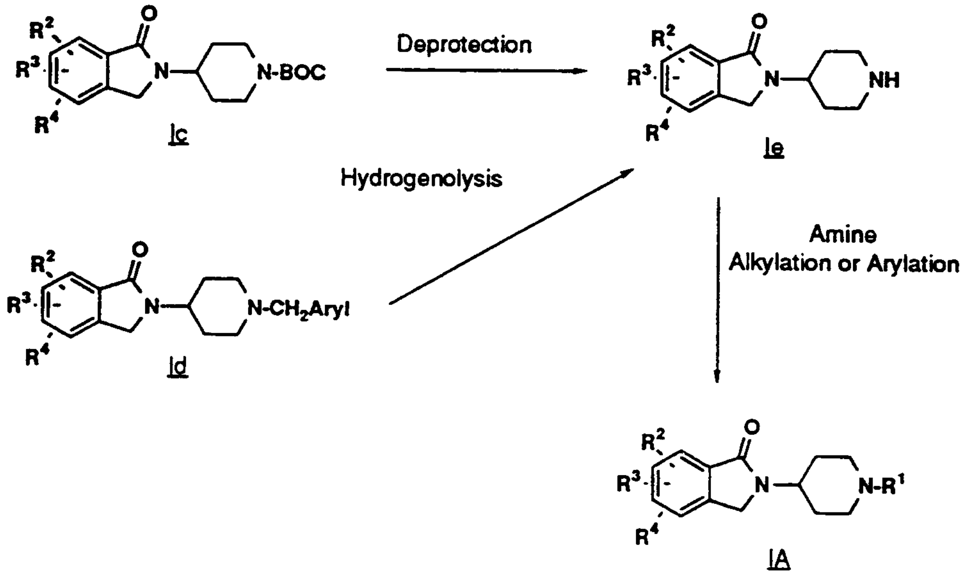


Ic Ie Id IA

Scheme IV. Routes to Starting Materials IVb and IVc

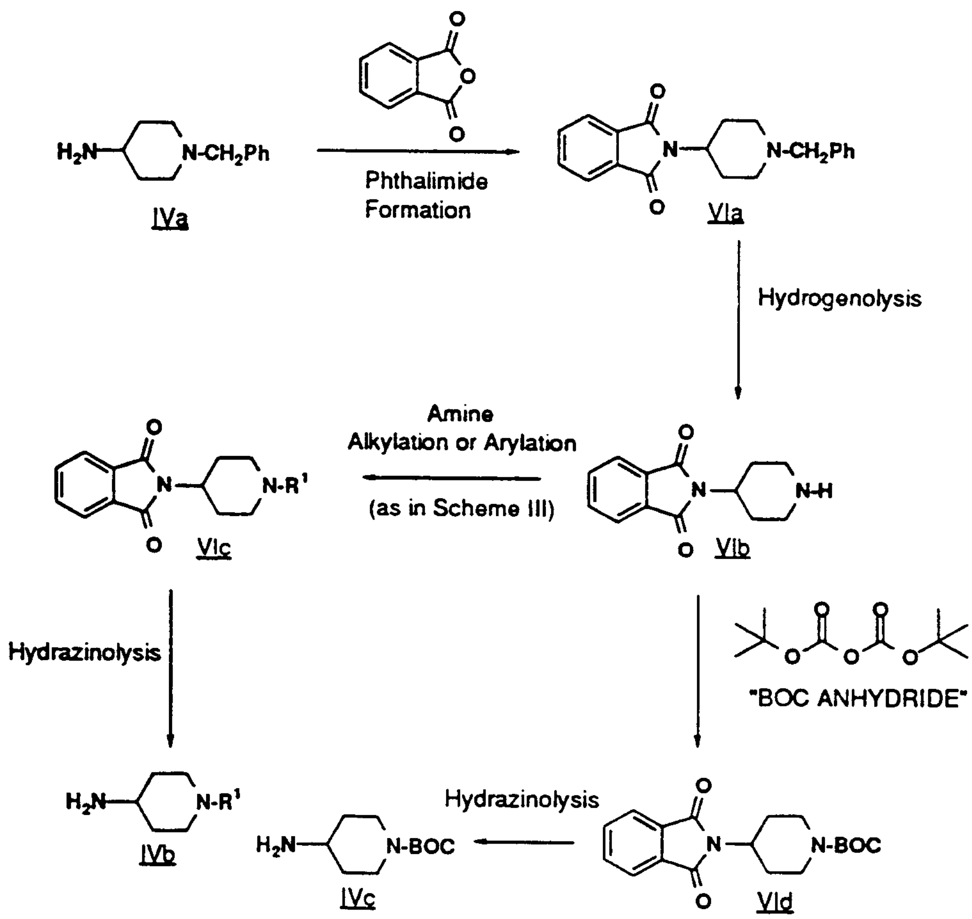


IVa VIa VIc VIb IVb IVc VId

Scheme V. General Routes to Starting Materials IVb

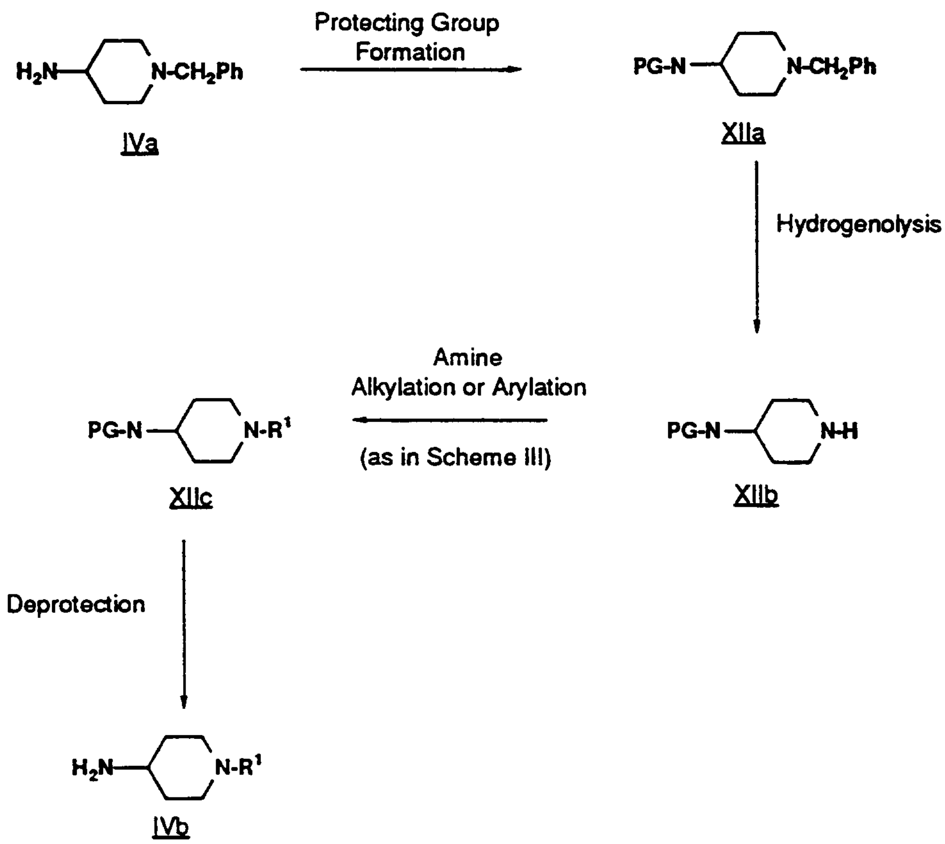


IVa

XIIa

XIIb

XIIc

IVb

Scheme VI. General Routes to II

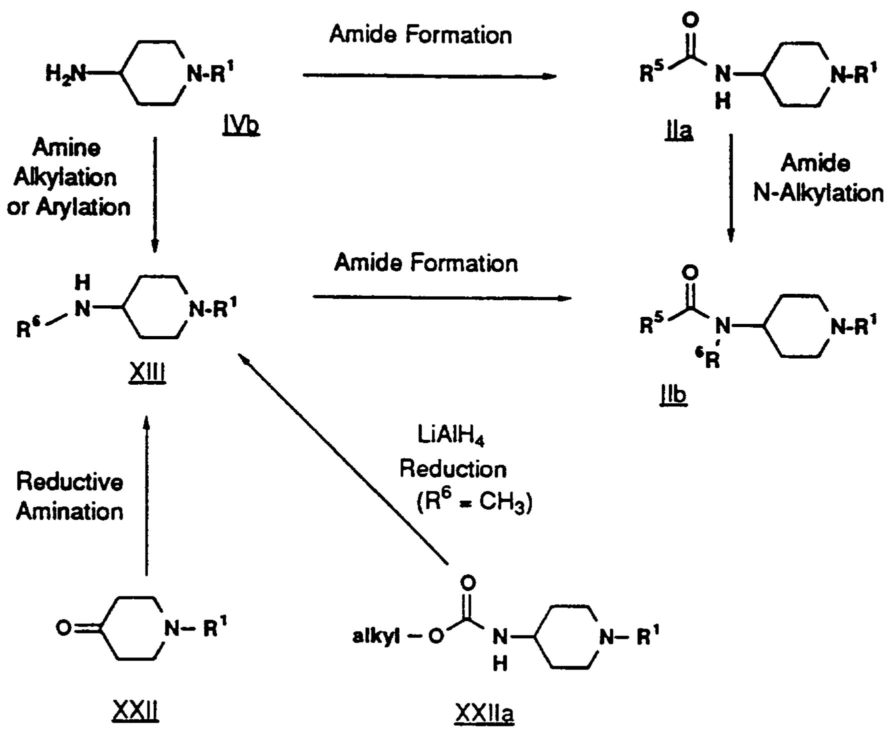


IVb

IIa

XIII

IIb

XXII

XXIIa

Scheme VII. General Route to III

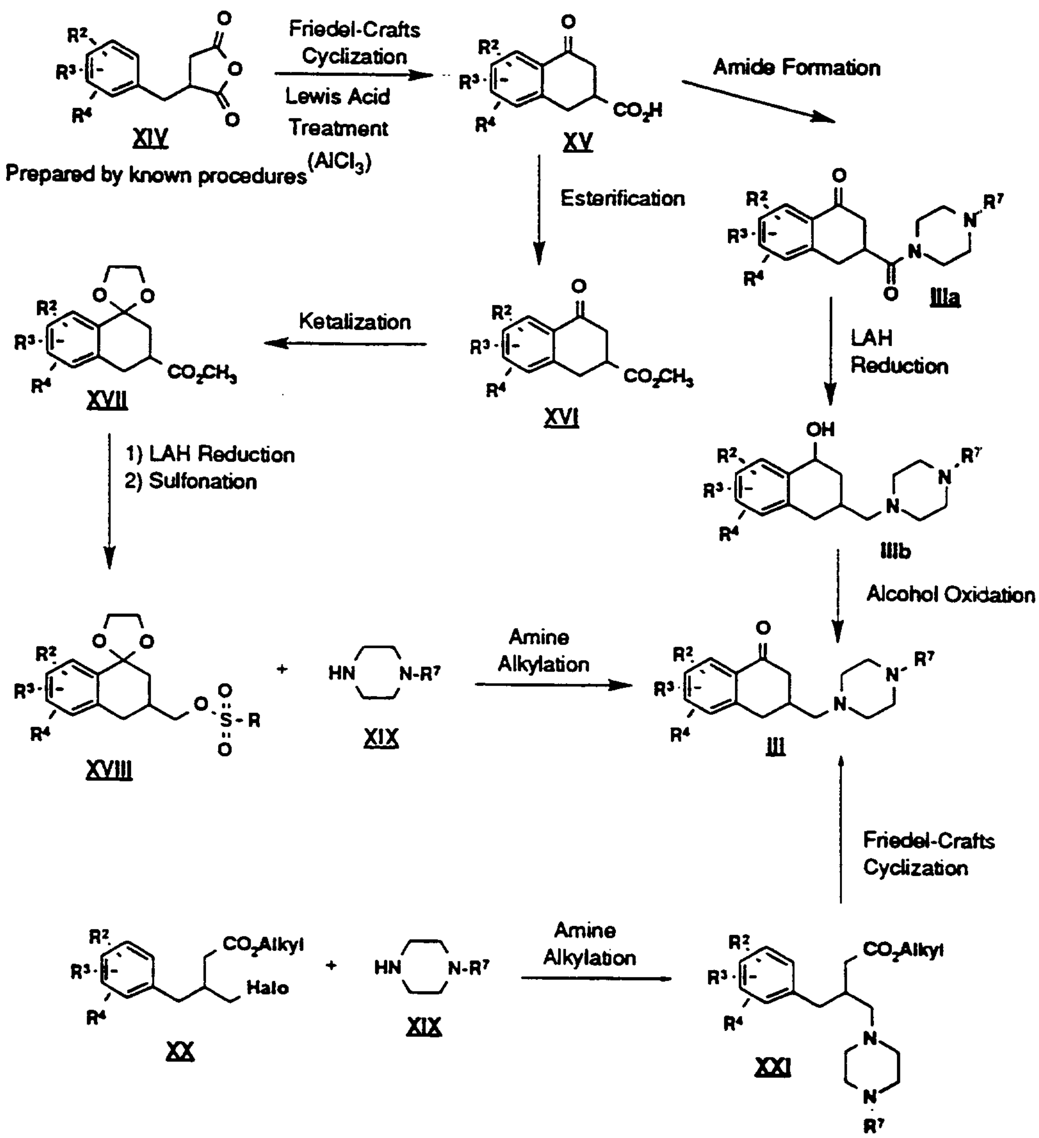

Scheme VIII Preparation of Compounds $IA^1$, $IA^2$

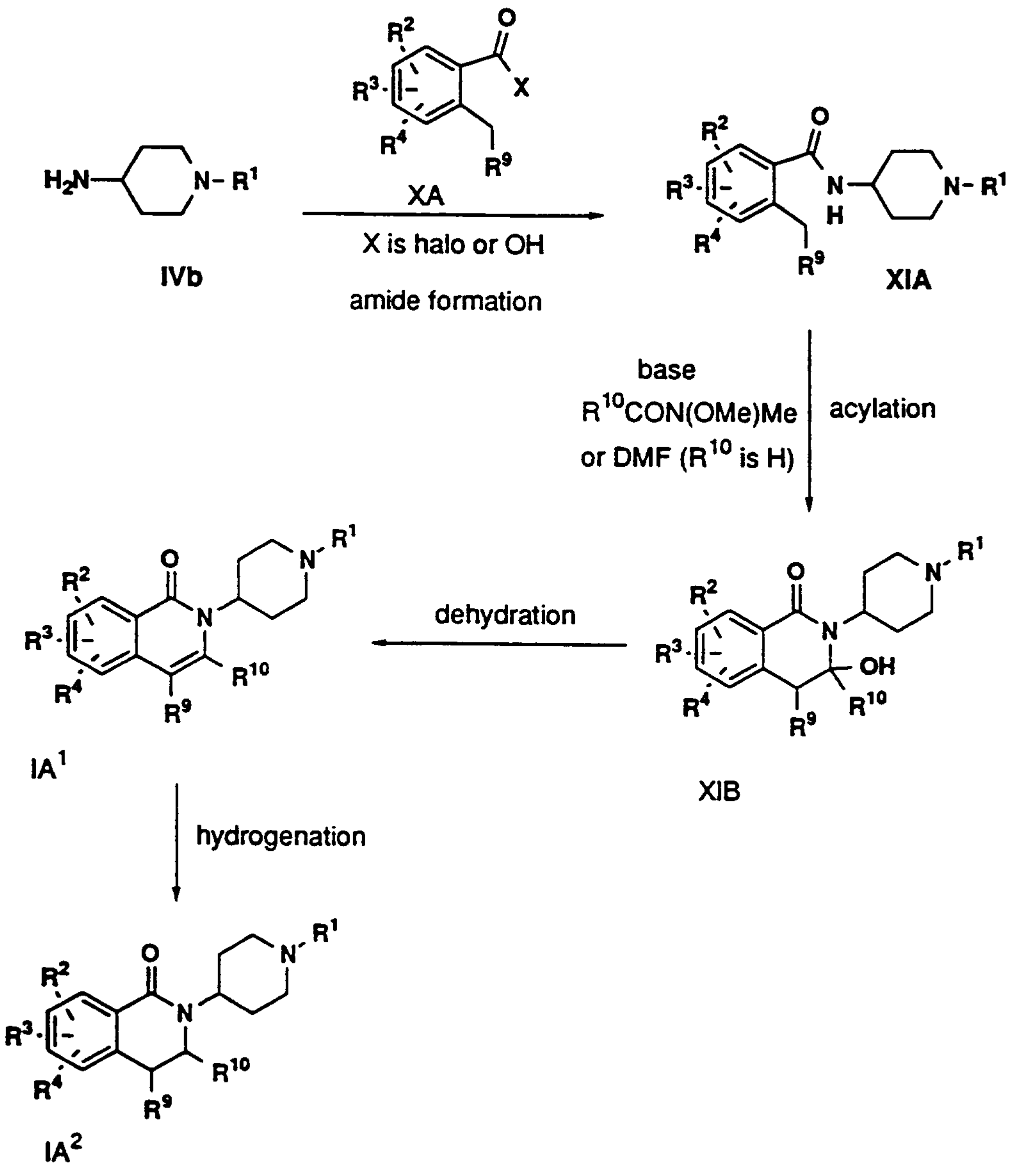

Scheme IX Preparation of Compounds $IA^3$-$IA^6$

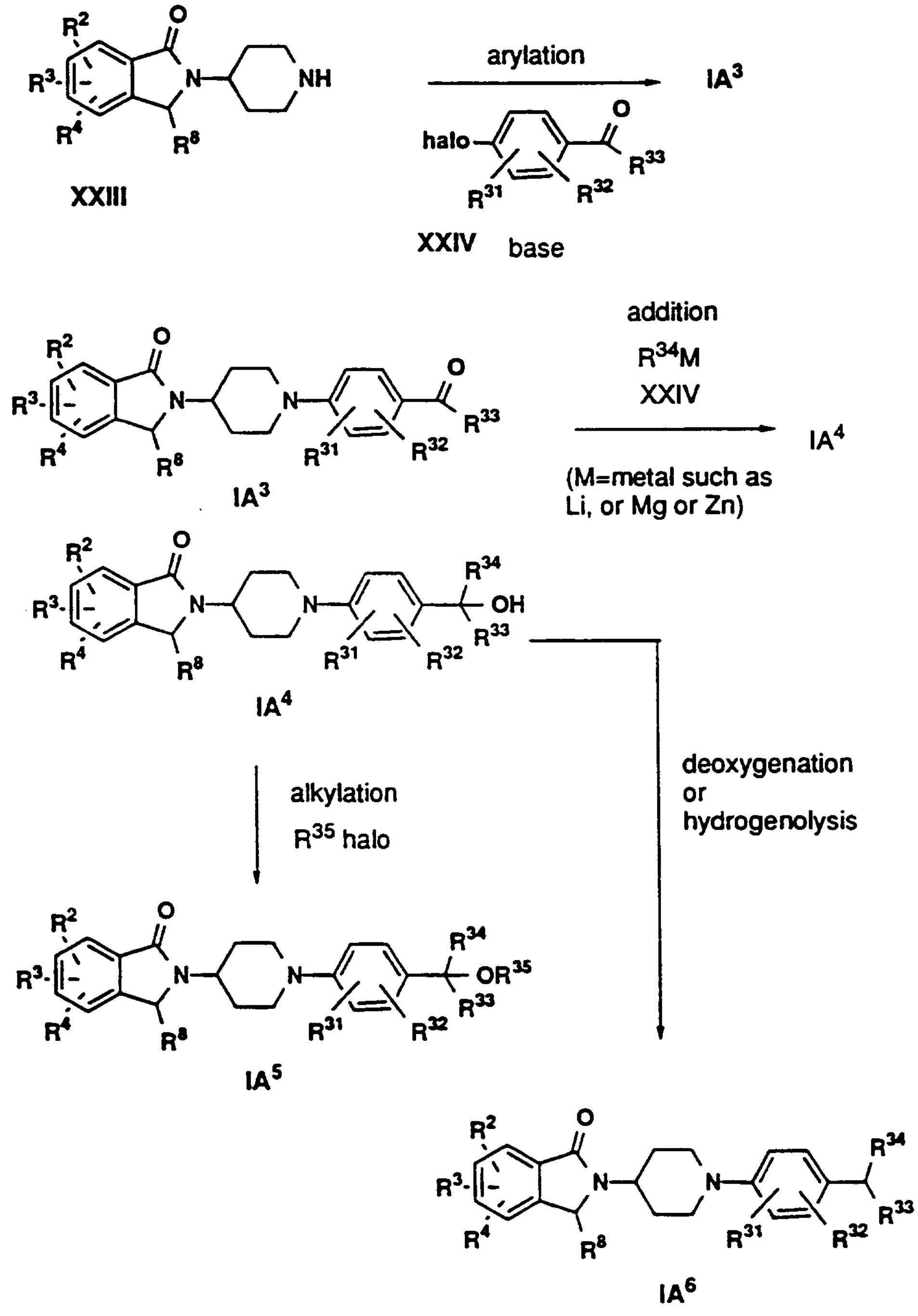


XXIII

XXIV

$IA^3$

$IA^4$

$IA^5$

$IA^6$

$R^{31}$ and $R^{32}$ are independently selected from any of the $R^2$, $R^3$, or $R^4$ radicals;

$R^{33}$ and $R^{34}$ are independently selected from any of the $R^1$ radicals as well as aryloxy, alkoxy, arylalkoxy, heteroarylalkoxy and heteroaryloxy;

$R^{35}$ can be any of the $R^1$ radicals.

Scheme X Preparation of Compound $IA^7$

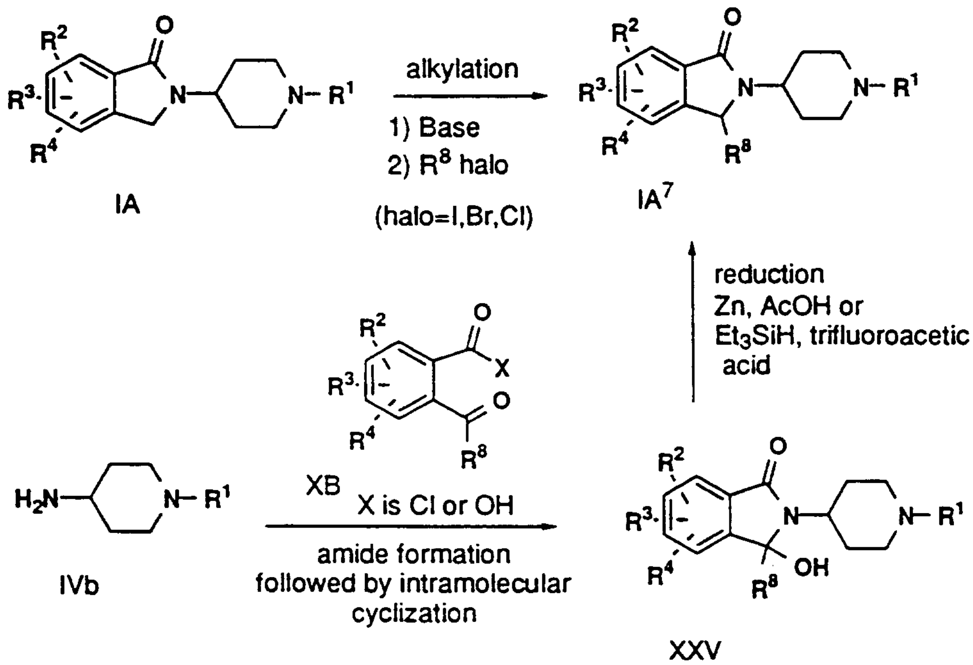


IA

$IA^7$

IVb

XXV

Scheme XI Preparation of Compound II
(Robotic Amide Coupling)

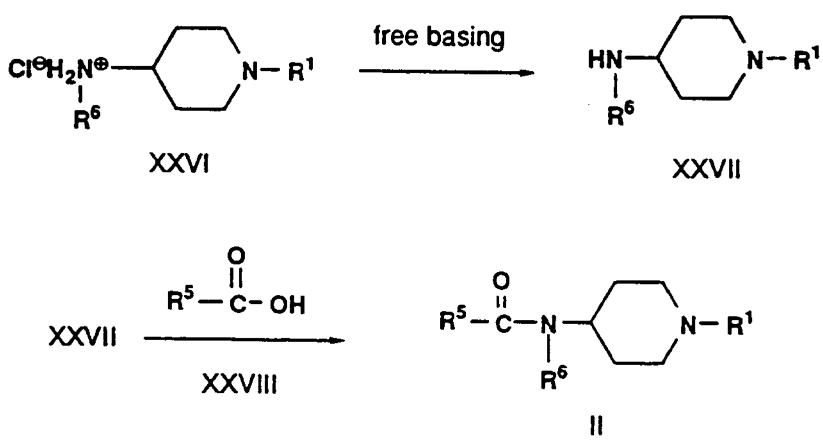


XXVI

XXVII

II

Scheme XII

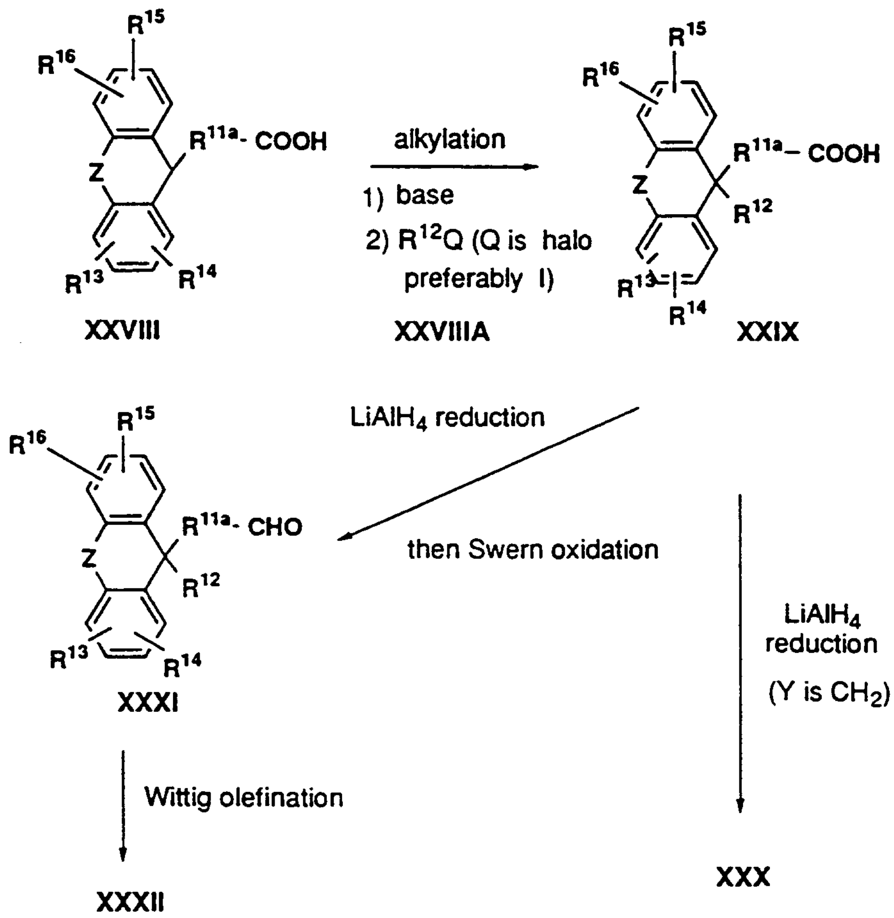


XXVIII XXVIIIA XXIX XXXI XXX XXXII

$R^{11a}$ can be any of the $R^{11}$ radicals.

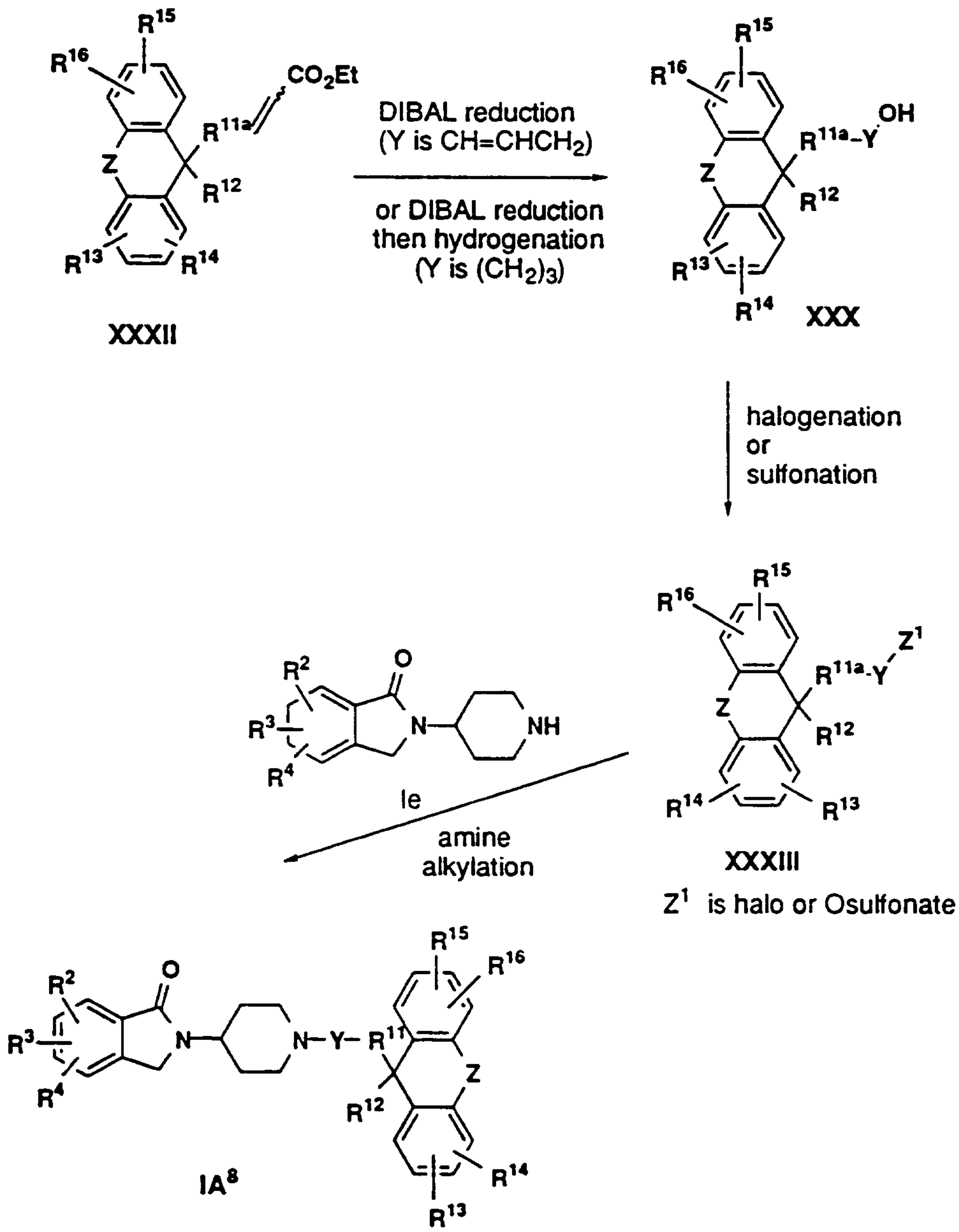


XXXII

XXX

XXXIII

$Z^1$ is halo or Osulfonate

$IA^8$

Phthalimide formation (Reaction Schemes I, IV) may be carried out by heating to about 80 to 150 °C in an oil bath optionally in an inert solvent or by various other procedures known in the art. See, e.g., Example 13 hereinafter.

Reduction (Reaction Scheme I) may be carried out by treatment with such reducing agents as zinc in the presence of acetic acid or tin in the presence of hydrochloric acid under an inert atmoshphere (e.g., argon).

Isoindolone formation (Reaction Scheme I) may be carried out by heating in the range of about 50 to 150 °C in an organic solvent (e.g., toluene, ethanol, dimethylformamide) optionally in the presence of a salt (e.g., potassium carbonate) or a tertiary amine base (e.g., 2,6-di-t-butylpyridine or triethylamine).

Amide formation (Reaction Schemes II, VI, VII, VIII, X, XI) may be carried out by a number of methods known in the art. For example, an amine substrate may be treated with (1) an acid halide $R^5C(O)$halo or compound X or XA in an aprotic solvent, optionally in the presence of a tertiary amine base (e.g., triethylamine); (2) the acid halide in the presence of an aqueous base under Schotten-Baumann conditions; (3) a free carboxylic acid ($R^5CO_2H$) in the presence of a coupling agent such as dicyclohexylcarbodiimide (DCC) or 1-(3-dimethylaminopropyl)-3-ethylcarbodiimide hydrochloride (WSC), optionally in the presence of 1-hydroxybenzotriazole (HOBT); (4) the free acid in the presence of N, N-carbonyldiimidazole in an aprotic organic solvent followed by the amine substrate; (5) trialkylaluminum (e.g., $Al(CH_3)_3$) in an aprotic solvent ,

followed by an ester (e.g., $R^5CO_2$alkyl or compound VIII) or (6) mixed anhydride formation, by reacting the acid with an acid chloride (e.g., isobutyl chloroformate or bis-(2-oxo-3-oxazolidinyl)phosphinic chloride (Bop-Cl)) in the presence of a tertiary amine base (e.g., triethylamine) followed by treatment with the amine substrate.

Mesylate formation (Reaction Scheme II) may be carried out by treatment of the amine-alcohol substrate with methanesulfonyl chloride and triethylamine or pyridine or in an aprotic solvent, such as dichloromethane.

Base cyclization (Reaction Schemes II, VIII) may be carried out by treatment with a base (e.g., potassium t-butoxide or sodium hydride) in an inert solvent (e.g., dimethylformamide, tetrahydrofuran, dimethoxymethane, or toluene). Mitsunobu cyclization (Reaction Scheme II) may be carried out by procedures generally known in the art. See, e.g., R. K. Olsen, J. Org. Chem., 49, 3527 (1984); Genin, M. J., et al., J. Org. Chem., 58, 2334-7 (1993).

Alternatively, a mixture of compounds IV and VIII can be converted to compound Ia in a single pot by heating the mixture in a protic solvent (e.g., water, methanol, ethenyl or isopropanol or mixtures thereof) at 100 to 200 °C. See, e.g., European patent application 81 / 26,749, FR 2, 548,666 (1983).

Protection and deprotection (Reaction Schemes III, IV, V) may be carried out by procedures generally known in the art. See, for example, T. W. Greene, Protecting Groups in Organic Synthesis, Second edition, 1991. PG in Scheme V denotes a nitrogen-protecting group. One particularly useful group is tert-butoxycarbonyl (BOC) which can be derived from the associated anhydride as shown in Scheme IV. BOC-protected amines may typically be deprotected by treatment with acid (e.g., trifluoroacetic acid or hydrochloric acid) in procedures well understood by those having ordinary skill in the art.

Hydrogenolysis (Reaction Schemes III, IV, V) may be carried out with $H_2$ using a balloon apparatus or a Parr Shaker in the presence of a catalyst (e.g., pallladium on activated carbon).

Amine alkylation and arylation (Reaction Schemes III, IV, VII, IX, XII) may be carried out by methods known in the art. Suitable procedures are described in Cortizo, L., J. Med. Chem. 34, 2242-2247 (1991). For example, the alkylation or arylation may be carried out by treating the amine substrate with a halide (e.g., $R^1$-halo) or an oxytosylate (e.g., $R^1$-O-tosylate) in an aprotic solvent (e.g., dimethylformamide), optionally in the presence of a tertiary amine (e.g., triethylamine) or an inorganic base (e.g., potassium carbonate).

Alkylation of the isoindolone (Reaction Schemes III, IV, VII, IX, X) may be carried out by treatment of the isoindolone with a strong base (i.e. sodium bis(trimethylsilyl)amide or lithium diisopropylamide) followed by an alkyl halide (e.g. $R^8$-halo) or alkyl sulfonate (e.g. $R^8$-tosylate) in an inert solvent (e.g. tetrahydrofuran or dimethoxyethane). Alternatively, as seen in Scheme X, amine IVb can be treated under amide formation conditions with a ketone with the structure XB to provide a hydroxylactam XXV, which could be subjected to reduction conditions with such reducing agents as zinc in acetic acid or triethylsilane in trifluoroacetic acid to give $IA^7$.

Reductive amination may be employed as an alternative to the foregoing amine alkylation and arylation procedures when $R^1$, $R^6$ or $R^7$ is $R^9R^{10}CH$- and $R^9$ and $R^{10}$ are each independently hydrogen, alkyl, alkenyl, aryl, heteroaryl, arylalkyl, heteroarylalkyl, cycloalkyl, or cycloalkylalkyl, or $R^9$ and $R^{10}$ together are alkylene (i.e., $R^9R^{10}CH$- forms a cycloalkyl group). Such reductive amination may be carried out by treating the amine with (a) a ketone or aldehyde ($R^9$-C(O)-$R^{10}$), (b) $NaBH_4$, $NaBH_3CN$ or NaB(acetoxy)$_3$H, (c) a protic solvent (e.g., methanol) or a dipolar aprotic solvent (e.g., acetonitrile), and, optionally, (d) an acid (e.g., acetic acid, trifluoroacetic acid, hydrochloric acid, or titanium isopropoxide).

When $R^1$ is aryl or heteroaryl, transition metals (e.g., palladium or copper salts or complexes) may be used to promote the arylation reaction.

Hydrazinolysis of phthalimides may be carried out by standard means known in the art. See, e.g., T. W. Greene, Protecting Groups in Organic Synthesis, Second edition, 1991.

Amide N-alkylation (Reaction Scheme VI) may be carried out by base treatment (e.g., NaH, KH, KN[Si-$(CH_3)_3]_2$, $K_2CO_3$, P4-phosphazene base, or butyl lithium) in an aprotic organic solvent, followed by treatment with $R^6$-halo or $R^6$-O-tosylate. Use of P-phosphazene base is described in T. Pietzonka, D. Seebach, Angew. Chem. Int. Ed. Engl. 31, 1481, 1992.

In Scheme VII, the Friedel-Crafts cyclization may be carried out with, for example, aluminum chloride, boron trifluoride or polyphosphoric acid and aprotic solvents such as nitrobenzene, nitromethane or carbon disulfide at about -20 °C to 80 °C. The esterification may be carried out with a common esterifying agent (e.g., sulfuric acid in methanol) with heating to reflux. Ketalization may be carried out by treatment with such reagents as ethylene glycol in an organic solvent (e.g., benzene) in the presence of an acid catalyst (e.g., p-toluenesulfonic acid). Reduction with lithium aluminum hydride (LAH) may be carried out in an organic solvent (e.g., tetrahydrofuran) from 0 °C to 70 °C. Oxidation of alcohols may be carried out by Oppenauer oxidation, such as treatment with potassium t-butoxide and benzophenone, or by other procedures known in

the art. The sulfonation may be carried out with $RSO_2Cl$ wherein R is alkyl, haloalkyl or aryl in an organic solvent (e.g., pyridine, dichloromethine) in an inert atmosphere (e.g., nitrogen) optionally in the presence of a tertiary amine base (e.g., triethylamine).

Compound III can also be prepared from compound XX as described by Cortizo, L., J. Med. Chem. 34, 2242-2247 (1991).

Dehydration (Scheme VIII) may be carried out employing a strong acid such as hydrochloric acid, sulfuric acid or trifluoroacetic acid.

Hydrogenation (Scheme VIII) may be carried out in the presence of a conventional catalyst such as Pd/C or Pt or Rh under a $H_2$ atmosphere.

The addition reaction shown in Scheme IX may be carried out by treating $IA^3$ with an organometallic reagent XXIV, such as an organolithium or organic magnesium compound where organo is alkyl or aryl.

The deoxygenation or hydrogenation reaction (Scheme IX) is carried out in the presence of a strong acid such as trifluoroacetic acid or boron trifluoride etherate, in the presence of a hydride source such as triethyl silane or tris(trimethylsilyl)silane.

The alkylation in Scheme XII is carries out in the presence of base such as butyllithium or sodium bis-(trimethylsilyl)amide. It will be appreciated that $R^{12}$ in $R^{12}Q$ may be any of the $R^{12}$ groups as defined hereinbefore.

With respect to Scheme XII, the $LiAlH_4$ reduction, Swern oxidation, Wittig olefination and halogenation/sulfonation reactions are conventional reactions well known to those skilled in the art.

The compounds of the invention may be employed in preventing, stabilizing or causing regression of atherosclerosis in a mammalian species by administering a therapeutically effective amount of a compound to decrease the amount or activity of MTP.

The compounds of the invention can be tested for MTP inhibitory activity employing the procedures set out in U.S. application Serial No. 117,362 filed September 3, 1993.

The compounds of the invention may also be employed in lowering serum lipid levels, such as cholesterol or triglyceride (TG) levels, in a mammalian species, by administering a therapeutically effective amount of a compound to decrease the amount or activity of MTP.

The compounds of the invention may be employed in the treatment of various other conditions or diseases using agents which decrease the amount of activity of MTP. For example, compounds of the invention decrease the amount or activity of MTP and therefore decrease serum cholesterol and TG levels, and TG, fatty acid and cholesterol absorption and thus are useful in treating hypercholesterolemia, hypertriglyceridemia, hyperlipidemia, pancreatitis, hyperglycemia and obesity.

The compounds of the present invention are agents that decrease the activity or amount of MTP and can be administered to various mammalian species, such as monkeys, dogs, cats, rats, humans, etc., in need of such treatment. These agents can be administered systemically, such as orally or parenterally.

The agents that decrease the activity or amount of MTP can be incorporated in a conventional systemic dosage form, such as a tablet, capsule, elixir or injectable formulation. The above dosage forms will also include the necessary physiologically acceptable carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to the age, weight, and condition of the patient, as well as the route of administration, dosage form and regimen, and the desired result. In general, the dosage forms described above may be administered in amounts of from about 5 to about 500 mg per day in single or divided doses of one to four times daily.

The following Examples represent preferred embodiments of the invention. All temperatures are in °C unless indicated otherwise.

**Example 1**

**N-[1-(3,3-Diphenylpropyl)-4-piperidinyl]benzamide monohydrochloride**

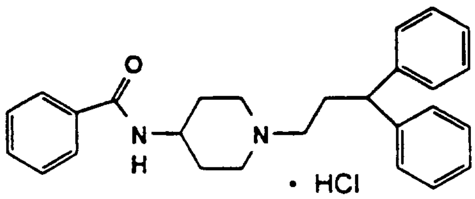

**A. [1-(Phenylmethyl)-4-piperidinyl]carbamic acid, 1,1-dimethylethyl ester**

To a solution of 4-amino-1-benzylpiperidine (20.0 g, 105 mmol) in dichloromethane (150 mL) was added dropwise a solution of di-tert-butyldicarbonate (25.2 g, 116 mmol) in dichloromethane (50 mL) at 0 °C. After addition, the reaction was warmed to room temperature. The reaction was maintained at this temperature for 2 hours. The reaction was evaporated to dryness. The resulting residue was recrystallized from ethyl ether to give compound A (23.5 g, 76 %) as a white solid (melting point 119-121 °C).

**B. 4-Piperidinylcarbamic acid, 1,1-dimethylethyl ester**

A suspension of 64.94 g (0.224 mol) of compound A and 25.6 mL (0.447 mol) of acetic acid in 500 mL of absolute ethanol was warmed to dissolve all solids. After cooling, 6.5 g (1 wt %) of 10% palladium on charcoal was added and the mixture was shaken on a Parr apparatus under initial hydrogen pressure of 40 psi for 23 hours. The catalyst was removed by filtration and the solution was concentrated to a clear oil which was dissolved in 1.5 L of chloroform. The organics were washed with a 3 N KOH solution saturated with NaCl (2 x 75 mL). The aqueous layer was back extracted with chloroform (5 x 200 mL). The combined organics were dried (sodium sulfate) and concentrated to provide 65 g of a white solid which was redissolved in 1.5 L of chloroform and washed with brine (2 x 200) mL to remove residual acetate. The combined aqueous layers were back extracted and the combined organics were dried (sodium sulfate) and concentrated to provide 40.15 g (90%) of compound B as a white solid (melting point 156-159 °C).

**C. γ-Phenylbenzenepropanol, 4-methylbenzenesulfonate ester**

To a solution of tosyl chloride (4.94 g, 25.9 mmol) in dichloromethane (10 mL) was added 3,3-diphenyl-1-propanol (5.00 g, 23.6 mmol) and pyridine (2.86 mL, 35.4 mmol) at room temperature. The reaction was stirred overnight at room temperature. Ethyl ether (200 mL) was added to dilute the reaction, and the organic layer was washed with 1 N HCl (50 mL x 2), saturated sodium carbonate (50 mL x 2), brine (50 mL x 2) and dried over $MgSO_4$. Purification was performed by flash chromatography, loaded and eluted with 25% ethyl acetate in hexane. Pure fractions were combined and evaporated to give compound C (5.2 g, 60%) as a colorless oil.

**D. [1-(3,3-Diphenylpropyl)-4-piperidinyl]carbamic acid, 1,1-dimethylethyl ester**

To a solution of compound C (1.83 g, 5.00 mmol) and compound B (1.00 g, 5.00 mmol) in isopropanol (25 mL) was added potassium carbonate (1.1 g, 8.00 mmol). The reaction was refluxed overnight. The reaction was cooled to room temperature and filtered, and the filtrate was evaporated to dryness. Purification was performed by flash chromatography, loaded and eluted with 2.5% methanol in dichloromethane. Pure fractions were combined and evaporated to give compound D (1.5 g, 76 %) as a colorless oil.

**E. 1-(3,3-Diphenylpropyl)-4-piperidinamine, hydrochloride**

To a stirred solution of 9.21 g (23.34 mmol) of compound D in 60 mL of dioxane was added 58 mL (0.223 mol) of a 4.0 M HCl in dioxane solution. The mixture was stirred for 15 hours then concentrated to provide 8.45 g (100%) of compound E as a white solid containing 10 wt % of dioxane by $^1H$ NMR, melting

point 123-126 °C. A dioxane-free sample of the hydrochloride salt has a melting point of 192-194 °C.

**F. N-[1-(3,3-Diphenylpropyl)-4-piperidinyl]benzamide**

To solution of compound E (100 mg, 0.30 mmol) and triethylamine (152 mg, 0.33 mmol) in dichloromethane (2 mL) was added a solution of benzoyl chloride (46.8 mg, 0.33 mmol) in dichloromethane (0.5 mL) at 0 °C. After addition, the reaction was stirred at 0 °C for 10 minutes. The reaction was diluted with dichloromethane (50 mL), the organic layer was washed with saturated sodium bicarbonate solution (10 mL), water (10 mL) and dried over sodium sulfate. The solution was evaporated to dryness. The resulting residue was recrystallized from isopropanol to give compound F (100 mg, 84%) as a white solid (melting point 151-155 °C).

**G. N-[1-(3,3-Diphenylpropyl)-4-piperidinyl]benzamide, monohydrochloride**

Compound F (100 mg, 0.25 mmol) was dissolved in ethanol (2 mL) and 1 N HCl in diethyl ether (0.5 mL) was added. The mixture was evaporated to give Example 1 (100 mg, 100%) as a white solid, melting point 246-249 °C.

| Analysis for $C_{27}H_{31}ClN_2O \cdot 0.2H_2O$: | | | | |
|---|---|---|---|---|
| Calc'd | C, 73.94; | H, 7.22; | N, 6.39; | Cl, 8.08 |
| Found: | C, 73.90; | H, 7.18; | N, 6.40; | Cl, 8.11 |

## Example 2

**2-[1-(3,3-Diphenyl-2-propenyl)-4-piperidinyl]-2,3-dihydro-1H-isoindol-1-one, monohydrochloride**

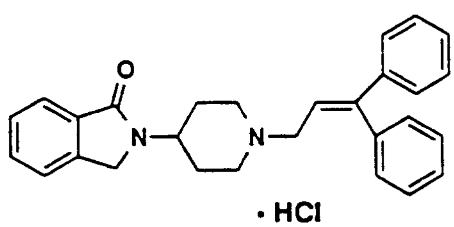

**A. 2-(4-piperidinyl)-2,3-dihydro-1H-isoindol-1-one**

To a solution of compound B from Example 3 (8.5 g, 26.4 mmol) in ethanol (65 mL) was added acetic acid (3.5 mL, 52.8 mmol), followed by 10% palladium on activated carbon (0.7 g) under argon. The slurry was purged with nitrogen and agitated under a pressure of 45 psi of hydrogen gas for 48 hours. The reaction mixture was filtered through Celite® and washed with ethanol. The filtrate was evaporated to dryness. The resulting residue was dissolved in chloroform (100mL) and washed with 1 N KOH saturated with sodium chloride (2 x 30 mL) and dried over $MgSO_4$. The resulting clear solution was evaporated to dryness and azeotroped with toluene (2 x 30 mL) to give compound A (5.0 g, 77%) as a white solid, melting point 137-140 °C.

**B. 3,3-Diphenyl-2-propen-1-ol**

To a solution of β-phenylcinnamaldehyde (5.0 g, 24.0 mmol) in toluene (100 mL) was added 1 M diisobutylaluminum hydride (26.4 mL, 26.4 mmol) at 0 °C. The reaction was stirred at 0 °C for 15 minutes, and methanol (5 mL) was added slowly to quench the reaction. 1 M potassium sodium tartrate solution (150 mL) was added and the mixture was stirred at room temperature overnight. The reaction was diluted with ethyl ether (100 mL), and the organic layer was washed with brine (30 mL) and dried over $Na_2SO_4$. Evaporation gave compound B (3.95 g, 80%) as a pale yellow oil.

**C. 1-Chloro-3,3-diphenyl-2-propene**

To a solution of N-chlorosuccinimide (1.52 g, 11.4 mmol) in dichloromethane (40 mL) was added dimethyl sulfide (1.1 mL, 14.5 mmol) at -40 °C under argon. The reaction was stirred at -40 °C for 10 minutes then warmed to room temperature for 30 minutes. The white cloudy solution was recooled to -40 °C , and a solution of compound B (2.17 g, 10.3 mmol) in dichloromethane (3 mL) was added dropwise. The reaction was stirred at -40 °C for 2 hours and then diluted with hexane (100 mL). The organic layer was washed with water (50 mL), brine (50 mL x 2) and dried over $Na_2SO_4$.Evaporation gave compound C (1.9 g, 81%) as a colorless oil.

**D. 2-[1-(3,3-Diphenyl-2-propenyl)-4-piperidinyl]-2,3-dihydro-1H-isoindol-1-one**

To a solution of compound A (1.63 g, 7.56 mmol) and compound C (1.90 g, 8.32 mmol) in dimethylformamide (35 mL), potassium carbonate (1.10 g, 7.94 mmol) was added at room temperature. The reaction was stirred at 50 °C overnight. The reaction was evaporated to dryness. The resulting residue was dissolved in dichloromethane (150 mL) and washed with water (50 mL x 2), brine (50 mL x 2) and dried over $MgSO_4$. Evaporation gave a crude solid. Purification was performed by flash chromatography, loaded and eluted with 3% methanol in dichloromethane. Pure fractions were combined and evaporated to give compound D (1.95 g, 63 %) as a white solid, melting point 164-167 °C.

| Analysis for $C_{28}H_{28}N_2O$ • 0.3 $H_2O$: | | | |
|---|---|---|---|
| Calc'd: | C, 81.24; | H, 6.96; | N, 6.77; |
| Found: | C, 81.29; | H, 6.88; | N, 6.79. |

**E. 2-[1-(3,3-Diphenyl-2-propenyl)-4-piperidinyl]-2,3-dihydro-1H-isoindol-1-one, monohydrochloride**

To a solution of compound D (200 mg, 0.49 mmol) in methanol (2 mL) was added 1 N HCl in ethyl ether (0.5 mL) at room temperature. The resulting salt was filtered and washed with cold methanol (2 x 0.5 mL). After drying under high vacuum, Example 2 was obtained (160 mg, 80 %) as a white solid, melting point 231 - 235 °C.

| Analysis for $C_{28}H_{29}ClN_2O$ • 0.9 $H_2O$: | | | | |
|---|---|---|---|---|
| Calc'd: | C, 72.92; | H, 6.73; | Cl, 7.69; | N, 6.07; |
| Found: | C, 72.99; | H, 6.91; | Cl, 7.36; | N, 6.06. |

**Example 3**

**2,3-Dihydro-2-[1-(phenylmethyl)-4-piperidinyl]-1H-isoindol-1-one, monohydrochloride**

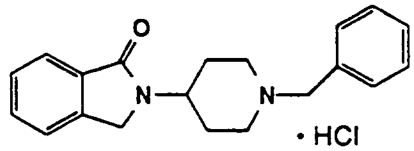

**A. 2-[1-(Phenylmethyl)-4-piperidinyl]-1H-isoindol-1,3(2H)-dione**

A mixture of phthalic anhydride (15.0 g, 101 mmol) and 4-amino-1-benzylpiperidine (19.3 g, 101 mmol) was heated with stirring in an oil bath until the mixture melted (about 125 °C). The reaction was kept at this temperature until the mixture solidified again (about 30 minutes). The reaction was cooled to room temperature. Purification was performed by flash chromatography on 1 kg silica gel, loaded and eluted with

30% ethyl acetate in hexane. Pure fractions were combined and evaporated to give compound A (25 g, 77%) as a white solid, melting point 151-154 °C.

**B. 2,3-Dihydro-2-[1-(phenylmethyl)-4-piperidinyl]-1H-isoindol-1-one**

To a solution of compound A (20.0 g, 62.5 mmol) in acetic acid (248 mL) was added zinc dust (28.6 g, 438 mmol) under argon. With mechanical stirring, the reaction was refluxed overnight. The reaction was filtered through Celite, then evaporated to dryness. Dichloromethane (500 mL) was added, and the organic layer was washed with saturated sodium bicarbonate (2 x 100 mL), brine (100 mL) and dried over $MgSO_4$. Evaporation gave a crude oil. The resulting residue was azeotroped with toluene (2 x 30 mL) to afford a white solid. The product was recrystallized from isopropanol to give compound B (16 g, 80 %) as a white solid (melting point 130-133 °C).

**C. 2,3-Dihydro-2-[1-(phenylmethyl)-4-piperidinyl]-1H-isoindol-1-one, monohydrochloride**

Compound B (200 mg, 0.62 mmol) was dissolved in ethanol (3 mL) and 4 N HCl in dioxane (1 mL) was added. After 2 minutes at room temperature, a white solid precipitated. The solid was filtered and pumped under high vaccum to give Example 3 (120 mg, 60 %) as a white solid, melting point 271-274 °C.

| Analysis for $C_{20}H_{23}N_2OCl \cdot 0.8\ H_2O$: | | | |
|---|---|---|---|
| Calc'd. | C, 67.22; | H, 6.94; | N, 7.84; |
| Found: | C, 66.99; | H, 7.05; | N, 8.07. |

**Example 4**

**2,3-Dihydro-2-[1-(3-phenylpropyl)-4-piperidinyl]-1H-isoindol-1-one, monohydrochloride**

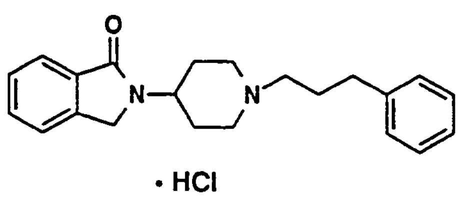

**A. 2,3-Dihydro-2-[1-(3-phenylpropyl)-4-piperidinyl]-1H-isoindol-1-one**

To a solution of compound A from Example 2 (300 mg, 1.39 mmol) in dimethylformamide (8 mL) was added 1-bromo-3-phenylpropane (276 mg, 1.39 mmol, Aldrich) and potassium carbonate (201 mg, 1.46 mmol) at room temperature. The reaction was stirred at room temperature for 30 minutes, then the reaction was heated to 50 °C for 4 hours. The reaction was cooled to room temperature. Dichloromethane (100 mL) was added to dilute the reaction, and the organic layer was washed with water (50 mL x 2), brine (50 mL x 2) and dried over magnesium sulfate. Evaporation under reduced pressure gave a crude oil. Purification was performed by flash chromatography on silica gel (50 g), loaded and eluted with 0.5% methanol in dichloromethane (1.5 L) then 1.2 % methanol in dichloromethane (1.0 L). Pure fractions were combined and evaporated to give compound A (400 mg, 84%) as a colorless oil.

**B. 2,3-Dihydro-2-[1-(3-phenylpropyl)-4-piperidinyl]-1H-isoindol-1-one, monohydrochloride**

Compound A (400 mg, 1.20 mmol) was dissolved in 20% methanol in ethyl ether (2 mL). A solution of 1 M HCl in ethyl ether (4 mL, 4.0 mmol) was added. The HCl salt precipitated and was filtered and washed with ethyl ether. The resulting solid was dried under high vacuum at 60 °C overnight to give Example 4 (320 mg, 80%) as a white solid, melting point 229-231 °C.

| Analysis for $C_{22}H_{27}ClN_2O$: | | | | |
|---|---|---|---|---|
| Calc'd: | C, 71.24; | H, 7.34; | N, 7.55; | Cl, 9.56; |
| Found: | C, 70.96; | H, 7.42; | N, 7.60; | Cl, 9.63. |

**Example 5**

**2-[1-(5,5-Diphenylpentyl)-4-piperidinyl]-2,3-dihydro-1H-isoindol-1-one, monohydrochloride**

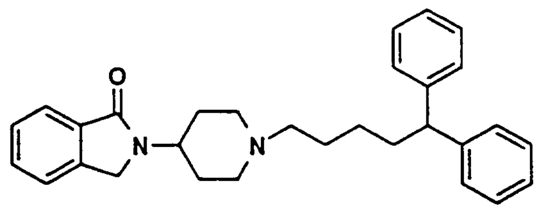

**• HCl**

**A. β-Phenylbenzenepropanal**

To a solution of oxalyl chloride (2.0 M in dichloromethane, 1.53 mL, 30.7 mmol) in dichloromethane (100 mL) was added dropwise a solution of dimethyl sulfoxide (4.35 mL, 61.4 mmol) in dichloromethane (9 mL) at -70 °C. After addition, the reaction was stirred at -70 °C for 30 minutes, then a solution of 3,3-diphenyl-1-propanol (5.0 g, 23.6 mmol) in dichloromethane (10 mL) was added dropwise. The reaction was stirred at -70 °C for 1 hour. Triethylamine (27 mL, 141 mmol) was added and the reaction mixture was warmed to room temperature. Ethyl ether (300 mL) was added to dilute the reaction, the organic layer was washed with water (2 x 100 mL), 1 N HCl (2 x 100 mL), saturated sodium bicarbonate solution (2 x 100 mL), brine (2 x 100 mL) and dried over $MgSO_4$. Evaporation gave compound A (5.0 g, 100%) as a yellowish oil.

**B. (E)-5,5-Diphenyl-2-pentenoic acid, ethyl ester**

To a suspension of sodium hydride (1.14 g, 28.6 mmol) in tetrahydrofuran (50 mL) was added dropwise a solution of triethyl phosphonoacetate (6.13 mL, 30.9 mmol) in tetrahydrofuran (5 mL) at 0 °C. The reaction was stirred at room temperature for 20 minutes (the solution is clear) then recooled to -78 °C. A solution of compound A (5.0 g, 23.8 mmol) in tetrahydrofuran (5 mL) was added dropwise. The reaction was warmed to room temperature and quenched with saturated ammonium chloride solution (5 mL). Ethyl ether (200 mL) was added to dilute the reaction, and the organic layer was washed with water (2 x 50 mL), brine (2 x 50 mL) and dried over $MgSO_4$. Evaporation gave a crude oil. Purification was performed by flash chromatography on 250 g silica gel, loaded and eluted with 6% ethyl acetate in hexane. Pure fractions were combined and evaporated to give compound B (5.0 g, 75%) as a colorless oil.

**C. (E)-5,5-Diphenyl-2-penten-1-ol**

To a solution of compound B (4.97 g, 17.8 mmol) in toluene (30 mL) at 0 °C was added dropwise diisobutyl aluminum hydride (1.0 M in toluene) (39.1 mL, 39.1 mmol). The reaction was stirred at 0 °C for 1 hour. The reaction was quenched with methanol (5 mL). Potassium sodium tartrate solution (1 M, 200 mL) was added, and the reaction mixture was stirred for 3.5 hours. Ethyl ether (200 mL) was added, and the organic layer was washed with water (2 x 50 mL), brine (2 x50 mL) and dried over $MgSO_4$. Evaporation gave a crude oil. Purification was performed by flash chromatography on 300 g silica gel, loaded and eluted with 20% ethyl acetate in hexane. Pure fractions were combined and evaporated to give compound C as a colorless oil (3.6 g, 85%).

**D. (E)-1-Chloro-5,5-diphenyl-2-pentene**

To a solution of N-chlorosuccinimide (2.22 g, 16.6 mmol) in dichloromethane (50 mL) at -40 °C was added dropwise methyl sulfide (1.55 mL, 21.1 mmol). The reaction was stirred at -40 °C for 10 minutes then warmed to room temperature for 30 minutes. The reaction was recooled to -40 °C, and a solution of compound C (3.6 g, 15.1 mmol) in dichloromethane (5 mL) was added dropwise. The reaction was stirred at -40 °C for 2 hours then warmed to room temperature for 30 minutes. Hexane (300 mL) was added to dilute the reaction and the organic layer was washed with water (2 x 50 mL), brine (2 x 50 mL) and dried over $MgSO_4$. Evaporation gave compound D (3.4 g, 87%) as a colorless oil.

**E. (E)-2-[1-(5,5-Diphenyl-2-pentenyl)-4-piperidinyl]-2,3-dihydro-1H-isoindol-1-one**

To a solution of compound A from Example 2 (800 mg, 3.70 mmol) in dimethylformamide (20 mL) was added compound D (952 mg, 3.70 mmol) followed by anhydrous potassium carbonate (536 mg, 3.89 mmol). The reaction was stirred at 50 °C for 3 hours. The reaction was cooled to room temperature. Ethyl acetate (100 mL) was added to dilute the reaction, and the organic layer was washed with water (2 x 50 mL), brine (2 x 50 mL) and dried over $Na_2SO_4$. Evaporation gave a crude oil. Purification was performed by flash chromatography on 100 g of silica gel, loaded and eluted with 2% methanol in dichloromethane. Pure fractions were combined and evaporated to give compound E (1.0 g, 62%) as a white solid (melting point 136-141 °C).

**F. 2-[1-(5,5-Diphenylpentyl)-4-piperidinyl]-2,3-dihydro-1H-isoindol-1-one**

To a solution of compound E (500 mg, 1.36 mmol) in ethanol (10 mL) was added 10% palladium on activated carbon (50 mg) under argon at room temperature. A hydrogen balloon was connected to the solution. Hydrogenation was maintained overnight. The reaction was filtered through Celite, and the filtrate was evaporated to dryness. Purification was performed by flash chromatography on 100 g silica gel, loaded and eluted with 2.5% methanol in dichloromethane. Pure fractions were combined and evaporated to give compound F (400 mg, 80%) as a white solid, melting point 121-124 °C.

**G. 2-[1-(5,5-Diphenylpentyl)-4-piperidinyl]-2,3-dihydro-1H-isoindol-1-one, monohydrochloride**

Compound F (400 mg, 0.91 mmol) was dissolved in 20% methanol in ethyl ether (2 mL). A solution of 1 M HCl in ethyl ether (4 mL, 4.0 mmol) was added. The HCl salt precipitated and was filtered and washed with ethyl ether. The resulting solid was dried under high vacuum at 60 °C overnight to give Example 5 (320 mg, 80%) as a white solid (melting point 208-211 °C).

| Analysis for $C_{30}H_{35}ClN_2O$: | | | | |
|---|---|---|---|---|
| Calc'd: | C, 75.85; | H, 7.43; | N, 7.90; | Cl, 7.46; |
| Found: | C, 75.54; | H, 7.54; | N, 7.82; | Cl, 7.56. |

**Example 6**

**N-[1-(3,3-Diphenylpropyl)-4-piperidinyl]cyclohexanecarboxamide, monohydrochloride**

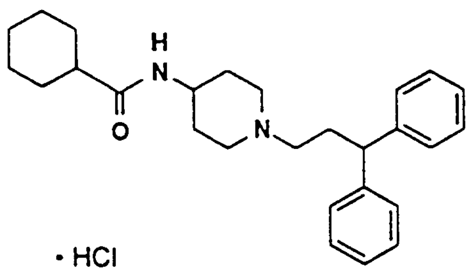

**A. N-[1-(3,3-Diphenylpropyl)-4-piperidinyl]cyclohexanecarboxamide**

To a stirred solution of 405 mg (1.22 mmol) of compound E from Example 1 and 7 mg (5 mol %) of 4-dimethylaminopyridine in 8 mL of methylene chloride at 0 °C under argon were added 296 μL (3.67 mmol) of pyridine and 171 μL (1.28 mmol) of cyclohexylcarbonyl chloride. After warming to room temperature, the mixture was stirred for one hour and diluted with methylene chloride and water. The organics were separated, and the aqueous, layer was basified with 1 M KOH and extracted with methylene chloride. The combined organics were dried (sodium sulfate) and concentrated to provide a yellow solid which was dried under high vacuum. The crude product was purified by flash chromatograghy on silica gel (80 g) eluted with 9:1 methylene chloride/methanol. Pure fractions were combined and concentrated to yield 438 mg (88%) of compound A as a clear, glassy solid.

**B. N-[1-(3,3-Diphenylpropyl)-4-piperidinyl]cyclohexanecarboxamide, monohydrochloride**

To a solution of 430 mg (1.06 mmol) of compound A in 4 mL of methylene chloride was added 2.12 mL (2.12 mmol) of a 1.0 M solution of hydrogen chloride in diethyl ether. The opaque white solution was concentrated and dried under vacuum to provide 375 mg (76%) of Example 6 as a white solid, melting point greater than 250 °C.

| Analysis for $C_{27}H_{37}N_2OCl$: | | | | |
|---|---|---|---|---|
| Calcd.: | C, 73.53; | H, 8.46; | N, 6.35; | Cl, 8.04; |
| Found: | C, 73.38; | H, 8.52; | N, 6.16; | Cl, 7.97. |

Example 7

**2-[1-(3-Butylheptyl)-4-piperidinyl]-2,3-dihydro-1H-isoindol-1- one, monohydrochloride**

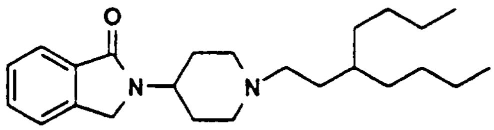

• HCl

**A. 3-Butyl-2-heptenoic acid, ethyl ester**

To a suspension of sodium hydride (60% in mineral oil) (1.01 g, 25.3 mmol) in tetrahydrofuran (40 mL) was added dropwise a solution of triethyl phosphonoacetate (5.44 mL, 27.4 mmol) in tetrahydrofuran (5 mL) at 0 °C. The reaction was warmed to room temperature and stirring was continued until the solution was clear. The reaction was recooled to -78 °C, a solution of 5-nonanone (3.0 g, 21.1 mmol) in tetrahydrofuran (5 mL) was added dropwise. The reaction was stirred at -78 °C for 1 hour. The reaction was warmed to room temperature and quenched with saturated ammonium chloride (5 mL). Ethyl ether (200 mL) was added to dilute the reaction, and the organic layer was washed with water (2 x 50 mL), brine (2 x 50 mL) and dried over magnesium sulfate. Purification was performed by flash chromatography on 400 g silica gel, loaded and eluted with 15% ethyl acetate in hexane. Pure fractions were combined and evaporated to give compound A (1.63 g, 37%) as a colorless oil.

**B. 3-Butyl-2-hepten-1-ol**

To a solution of compound A (1.63 g, 7.69 mmol) in toluene (20 mL) at 0 °C was added a solution of diisobutylaluminum hydride (1 M solution in toluene, 16.9 mL, 16.9 mmol). The reaction was stirred at room temperature for 10 minutes and quenched with methanol (5 mL). Potassium sodium tartrate solution (1 M, 100 mL) was added, the mixture was stirred overnight. Ethyl ether (100 mL) was added, and the organic layer was washed with water (2 x 50 mL), brine (2 x 50 mL) and dried over magnesium sulfate. Evaporation gave compound B (1.30 g, 99%) as a colorless oil.

**C. 3-Butyl-2-hepten-1-yl chloride**

To a suspension of N-chlorosuccinimide (1.12 g, 8.42 mmol) in dichloromethane (20 mL) at -40 °C was added dropwise a solution of methyl sulfide (0.79 mL, 10.7 mmol) in dichloromethane (1 mL). After addition, the reaction was warmed to room temperature for 30 minutes. The reaction was recooled to -40 °C, and a solution of 3 (1.3 g, 7.65 mmol) in dichloromethane (2 mL) was added. The reaction was stirred at -40 °C for 2 hours and warmed to room temperature. Hexane (150 mL) was added to dilute the reaction, and the organic layer was washed with water (2 x 50 mL), brine (2 x 50 mL) and dried over magnesium sulfate. Evaporation gave compound C (860 mg, 60%) as a cololess oil.

**D. 2-[1-(3-Butyl-2-heptenyl)-4-piperidinyl]-2,3-dihydro-1H-isoindol-1-one**

To a solution of compound A from Example 2 (974 mg, 4.51 mmol) in dimethylformamide (14 mL) was added a solution of compound C (850 mg, 4.51 mmol) in dimethylformamide (2 mL) followed by anhydrous potassium carbonate (653 mg, 4.74 mmol). The reaction was stirred at 50 °C for 3 hours. The reaction was cooled to room temperature. Ethyl acetate (100 mL) was added to dilute the reaction, and the organic layer was washed with water (2 x 50 mL), brine (2 x 50 mL) and dried over magnesium sulfate. Evaporation gave a crude oil. Purification was performed by flash chromatography on 100 g of silica gel, loaded and eluted with 2% methanol in dichloromethane. Pure fractions were combined and evaporated to give compound D (1.13 g, 68%) as a cololess oil.

**E. 2-[1-(3-Butylheptyl)-4-piperidinyl]-2,3-dihydro-1H-isoindol-1-one**

To a solution of compound D (500 mg, 1.36 mmol) in ethanol (10 mL) was added 10% palladium on activated carbon (50 mg) under argon at room temperature. Argon on the reaction was replaced by hydrogen. A hydrogen balloon was connected to the solution. Hydrogenation was maintained overnight. The reaction was filtered through Celite, and the filtrate was evaporated to dryness. Purification was performed by flash chromatography on 100 g silica gel, loaded and eluted with 2.5% methanol in dichloromethane. Pure fractions were combined and evaporated to give compound F (480 mg, 95%) as a waxy solid.

**F. 2-[1-(3-Butylheptyl)-4-piperidinyl]-2,3-dihydro-1H-isoindol-1-one, monohydrochloride**

Compound E (480 mg, 1.30 mmol) was dissolved in 20% methanol in ethyl ether (2 mL). A solution of 1 M HCl in ethyl ether (4 mL, 4.0 mmol) was added. The HCl salt precipitated and was filtered and washed with ethyl ether. The resulting solid was dried under high vacuum at 60 °C overnight to give Example 7 (300 mg, 62%) as a white solid (melting point 185-187 °C).

| Analysis for $C_{24}H_{39}ClN_2O \cdot 0.5\ H_2O$: | | | | |
|---|---|---|---|---|
| Calc'd: | C, 69.29; | H, 9.69; | N, 6.73; | Cl, 8.52; |
| Found: | C, 69.17; | H, 9.75; | N, 6.88; | Cl, 8.91 . |

**Example 8**

**N-[1-(3,3-Diphenylpropyl)-4-piperidinyl]pentamide, monohydrochloride**

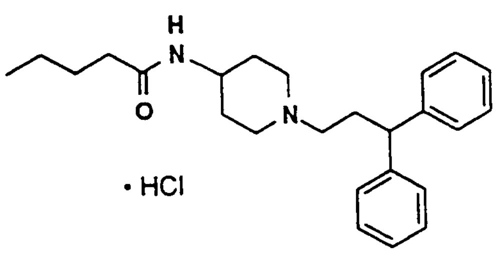

**A. N-[1-(3,3-Diphenylpropyl)-4-piperidinyl]pentamide**

To a stirred solution of 385 mg (1.16 mmol) of compound E from Example 1 and 7 mg (5 mol %) of 4-dimethylaminopyridine in 8 mL of methylene chloride at 0 °C under argon were added 282 µL (3.49 mmol) of pyridine and 147 µL (1.22 mmol) of valeryl chloride. After warming to room temperature, the mixture was stirred for one hour and diluted with methylene chloride and water. The organic layers were separated, and the aqueous layer was basified with 1 M KOH and extracted with methylene chloride. The combined organic layers were dried (sodium sulfate) and concentrated to provide a yellow solid which was dried under high vacuum. The crude product was purified by flash chromatography on silica gel (75 g) eluted with 95:5 methylene chloride/methanol. Pure fractions were combined and concentrated to yield 334 mg (76%) of compound A as a clear, glassy solid, melting point 126-128 °C.

**B. N-[1-(3,3-Diphenylpropyl)-4-piperidinyl]pentamide, monohydrochloride**

To a solution of 319 mg (0.84 mmol) of compound A in 4 mL of methylene chloride was added 1.68 mL (1.68 mmol) of a 1.0 M solution of hydrogen chloride in diethyl ether and the heterogeneous mixture was stirred for thirty minutes. The resulting precipitate was filtered, washed with ether, and dried under vacuum to provide 327 mg (72%) of Example 8 as a yellow solid, melting point 189 - 191 °C.

| Analysis for $C_{25}H_{35}N_2OCl + 0.3\ H_2O$: | | | | |
|---|---|---|---|---|
| Calc'd: | C, 71.41; | H, 8.54; | N, 6.66; | Cl, 8.43; |
| Found: | C, 71.56; | H, 8.46; | N, 6.51; | Cl, 8.66. |

**Example 9**

**(E)-2,3-Dihydro-2-[1-[3-(2-phenoxyphenyl)-2-propenyl]-4-piperidinyl]-1H-isoindol-1-one, monohydrochloride**

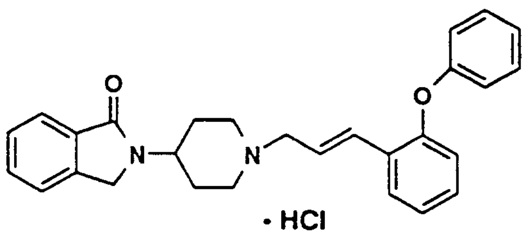

**A. 2-Phenoxybenzenemethanol**

To a solution of 2-phenoxybenzoic acid (5.0 g, 23.3 mmol) in tetrahydrofuran (50 mL) was added dropwise at 0 °C lithium aluminum hydride solution (1 M in tetrahydrofuran, 23.3 mL, 23.3 mmol). The reaction was warmed to room temperature and stirring was continued for 8 hours. The reaction was quenched with methanol (5 mL), and 1 M potassium sodium tartrate solution (100 mL) was added. The mixture was stirred at room temperature overnight. Ethyl ether (200 mL) was added, and the organic layer was washed with water (2 x 50 mL), brine (2 x 50 mL) and dried over magnesium sulfate. Evaporation gave compound A (4.65 g, 99%) as a colorless oil.

**B. 2-Phenoxybenzaldehyde**

To a solution of oxalyl chloride (2.0 M in dichloromethane, 15.1 mL, 30.3 mmol) in dichloromethane (100 mL) at -70 °C was added dropwise a solution of dimethyl sulfoxide (4.25 mL, 60.6 mmol) in dichloromethane (5 mL). After addition, the reaction was stirred at -70 °C for 30 minutes, then a solution of compound A (4.65 g, 23.3 mmol) in dichloromethane (10 mL) was added dropwise. The reaction was stirred at -70 °C for 1 hour. Triethylamine (27 mL) was added and the reaction mixture was warmed to room temperature. Ethyl ether (300 mL) was added to dilute the reaction, and the organic layer was washed with water (2 x 100 mL), 1 N HCl (2 x 100 mL), saturated sodium bicarbonate solution (2 x 100 mL) and brine (2 x 100 mL) and dried over $MgSO_4$. Evaporation gave compound B as a yellowish oil (4.63 g, 100%).

**C. (E)-3-(2-Phenoxyphenyl)-2-propenoic acid, ethyl ester**

To suspension of sodium hydride (1.12 g, 28.1 mmol) in tetrahydrofuran (50 mL) was added dropwise a solution of triethyl phosphonoacetate (6.04 mL, 30.4 mmol) in tetrahydrofuran (5 mL) at 0 °C. Then the reaction was stirred at room temperature for 20 minutes (the solution was clear). The reaction was recooled to -78 °C, and a soluiton of compound A (4.63 g, 23.4 mmol) in tetrahydrofuran (5 mL) was added dropwise. The reaction was warmed to room temperature and quenched with saturated ammonium chloride solution (5 mL). Ethyl ether (200 mL) was added to dilute the reaction, and the organic layer was washed with water (2 x 50 mL), brine (2 x 50 mL) and dried over $MgSO_4$. Evaporation gave a crude oil. Purification was performed by flash chromatography on 500 g silica gel, loaded and eluted with 10% ethyl acetate in hexane. Pure fractions were combined and evaporated to give compound C (6.0 g, 96%) as a colorless oil.

**D. (E)-3-(2-Phenoxyphenyl)-2-propenol**

To a solution of compound C (2.5 g, 9.33 mmol) in toluene at 0 °C was added dropwise a diisobutyl aluminum hydride (1.0 M in toluene) (20.5 mL, 20.5 mmol) solution. The reaction was stirred at 0 °C for 1

hour. The reaction was quenched with methanol (5 mL). 1 M potassium sodium tartrate solution (100 mL) was added, and the reaction mixture was stirred for 3.5 hours. Ethyl ether (200 mL) was added, and the organic layer was washed with water (2 x 50 mL), brine (2 x 50 mL) and dried over $MgSO_4$. Evaporation gave a crude oil. Purification was performed by flash chromatography on 300 g silica gel, loaded and eluted with 20% ethyl acetate in hexane. Pure fractions were combined and evaporated to give compound D (1.85 g, 88%) as a colorless oil.

**E. (E)-1-(3-Chloro-1-propenyl)-2-phenoxybenzene**

To a solution of N-chlorosuccinimide (1.11 g, 8.33 mmol) in dichloromethane (20 mL) was added dropwise methyl sulfide (0.78 mL, 10.6 mmol) at -40 °C. The reaction was stirred at -40 °C for 10 minutes then warmed to room temperature for 30 minutes. The reaction was recooled to -40 °C, and a solution of compound D (1.71 g, 7.57 mmol) in dichloromethane was added dropwise. The reaction was stirred at -40 °C for 3 hours, then warmed to room temperature for 30 minutes. Hexane (100 mL) was added to dilute the reaction, and the organic layer was washed with water (2 x 50 mL), brine (2 x 50 mL) and dried over $MgSO_4$. Evaporation gave compound E (1.72 g, 93%) as a colorless oil.

**F. (E)-2,3-Dihydro-2-[1-[3-(2-phenoxyphenyl)-2-propenyl]-4-piperidinyl]-1H-isoindol-1-one**

To a solution of compound A from Example 2 (0.88 g, 4.09 mmol) in dimethylformamide (10 mL) was added a solution of compound E (1.0 g, 4.09 mmol)in dimethylformamide (2 mL) followed by potassium carbonate (592 mg, 4.29 mmol). The reaction was stirred at 50 °C for 14 hours. The reaction was cooled to room temperature. Ethyl ether (100 mL) was added to dilute the reaction, and the organic layer was washed with water (2 x 50 mL), brine (2 x 50 mL) and dried over $MgSO_4$. Evaporation gave a crude oil. Purification was performed by flash chromatography on 150 g silica gel, loaded and eluted with 2% methanol in dichloromethane. Pure fractions were combined and evaporated to give compound F (1.1 g, 63%) as a colorless oil.

**G (E)-2,3-Dihydro-2-[1-[3-(2-phenoxyphenyl)-2-propenyl]-4-piperidinyl]-1H-isoindol-1-one, monohydrochloride**

To a solution of compound F (500 mg, 1.15 mmol) in ethyl ether: methanol (2 mL, 5 : 1) was added 1 M HCl in ethyl ether (1.5 mL, 1.5 mmol). The HCl salt precipitated from the solution. The salt was filtered and dried at 60 °C under vacuum to give Example 9 (300 mg, 55%) as a white solid, melting point 215-218 °C.

| Analysis for $C_{28}H_{29}ClN_2O_2$: | | | | |
|---|---|---|---|---|
| Calc'd: | C, 72.95; | H, 6.34; | N, 6.08; | Cl, 7.69; |
| Found: | C, 72.49; | H, 6.39; | N, 6.04; | Cl, 7.37. |

**Example 10**

**2,3-Dihydro-2-[1-[3-(2-methoxyphenyl)propyl]-4-piperidinyl]-1H-isoindol-1-one, monohydrochloride**

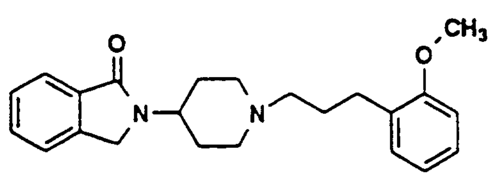

• HCl

**A. 2-Methoxybenzenepropanol**

To a solution of 3-(2-methoxyphenyl)propionic acid (2.0 g, 11.1 mmol) in tetrahydrofuran (25 mL) was added dropwise at 0 °C lithium aluminum hydride solution (1 M in tetrahydrofuran, 11.1 mL, 11.1 mmol).

The reaction was warmed to room temperature and stirring was continued overnight. The reaction was quenched with methanol (5 mL), and 1 M potassium sodium tartrate solution (100 mL) was added. The mixture was stirred at room temperature overnight. Ethyl ether (200 mL) was added, and the organic layer was washed with water (2 x 50 mL), brine (2 x 50 mL) and dried over magnesium sulfate. Evaporation gave compound A (1.5 g, 81%) as a colorless oil.

**B. 1-(3-Bromopropyl)-2-methoxybenzene**

To a solution of compound A (620 mg, 3.73 mmol) and triphenylphosphine (1.08 g, 4.11 mmol) in dichloromethane (10 mL) was added N-bromosuccinimide (731 mg, 4.11 mmol) at 0 °C. The reaction was stirred at 0 °C for 2 hours. Dichloromethane (100 mL) was added to dilute the reaction, and the organic layer was washed with water (2 x 50 mL), brine (2 x 50 mL) and dried over $MgSO_4$. Purification was performed by flash chromatography on 100 g silica gel, loaded and eluted with 10% dichloromethane in hexane. Pure fractions were combined and evaporation to give compound B (582 mg, 68%) as a colorless oil.

**C. 2,3-Dihydro-2-[1-[3-(methoxyphenyl)propyl]-4-piperidinyl]-1H-isoindol-1-one**

To a solution of compound A from Example 2 (549 mg, 2.54 mmol) in dimethylformamide (10 mL) was added a solution of compound B (582 mg, 2.54 mmol) in dimethylformamide (1 mL) followed by potassium carbonate (386 mg, 2.80 mmol). The reaction was stirred at 50 °C for 14 hours. The reaction was cooled to room temperature. Ethyl ether (100 mL) was added to dilute the reaction, and the organic layer was washed with water (2 x 50 mL), brine (2 x 50 mL) and dried over $MgSO_4$. Evaporation gave a crude oil. Purification was performed by flash chromatography on 150 g silica gel, loaded and eluted with 2% methanol in dichloromethane. Pure fractions were combined and evaporated to give compound C (560 mg, 61%) as a colorless oil.

**D. 2,3-Dihydro-2-[1-[3-(2-methoxyphenyl)propyl]-4-piperidinyl]-1H-isoindol-1-one, monohydrochloride**

To a solution of compound C (500 mg, 1.37 mmol) in methanol (2 mL) was added 1 M HCl in ethyl ether (1.5 mL, 1.5 mmol). The mixture was evaporated and dried at 70 °C under vacuum to give Example 10 (300 mg, 60%) as a yellowish solid, melting point 191-195 °C.

| Analysis for $C_{23}H_{29}ClN_2O_2$ + 0.3 mol $H_2O$: | | | | |
|---|---|---|---|---|
| Calc'd: | C, 67.98; | H, 7.34; | N, 6.89; | Cl, 8.72; |
| Found: | C, 67.92; | H, 7.63; | N, 6.75; | Cl, 8.54 |

## Example 11

**6-Fluoro-3,4-dihydro-3-[[4-(2-methoxyphenyl)-1-piperazinyl]-methyl]-1(2H)-naphthalenone**

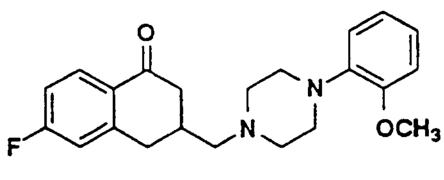

**A. α-Acetyl-3-fluorobenzenepropanoic acid, ethyl ester**

To a solution of 500 mL of 10% dimethylformamide in benzene was added 58.6% NaH (41 g, 1.0 mol) cooled in an ice bath was added ethyl acetoacetate (130 g, 1.0 mol) was added. The reaction was stirred at room temperature for 30 minutes, and m-fluorobenzyl chloride (145 g, 1.0 mol) was added. The reaction was heated to reflux for 3 hours and gave an NaCl precipitate which was then removed by filtration. The

filtrated was poured into $H_2O$, acidified with concentrated HCl and was extracted with a mixture of ether and benzene. The organic layer was washed with $H_2O$, brine. dried over $Na_2SO_4$ and concentrated in vacuo. The crude product was purified by distillation (112 - 119 °C / 25 mmHg) to give compound A (133 g, 56 %).

| Analysis for $C_{13}H_{15}FO_3$: | | |
|---|---|---|
| Calc'd: | C, 65.53; | H, 6.35; |
| Found: | C, 65.56; | H 6.12. |

**B. 2-Acetyl-2-[(3-fluorophenyl)methyl]butanedioic acid, diethyl ester**

This reaction procedure was followed as described above for the preparation of compound A. The reaction scale is as follows: Compound A (130 g, 0.546 mol), ethyl chloroacetate (67 g, 0.546 mol), 58.6 % NaH (22.36 g, 0.546 mol) and 400 mL of 20 % dimethylformamide in benzene. The reflux time in this reaction was 21 hours and the crude product was purified by distillation at 135-158 °C / 0.2 mmHg to give compound B (119 g, 67 %).

**C. 2-[(3-Fluorophenyl)methyl]butanedioic acid, diethyl ester**

To a solution of compound B (119.3 g, 0.368 mol) in 550 mL $H_2O$ was added NaOH (45 g, 1.10 mol) and the reaction was reflux for 23 hours. The reaction was cooled to room temperature, and the reaction mixture was washed with ether. The aqueous layer was placed in the ice bath, acidified with concentrated HCl and gave a precipitate. The crude product was removed by filtration and recrystallized in hot benzene to give compound C (57.8 g, 69 %), melting point 120.5 - 121.5 °C.

| Analysis for $C_{11}H_{11}FO_4$: | | |
|---|---|---|
| Calc'd: | C, 58.41; | H, 4.90; |
| Found: | C, 58.91; | H, 5.10. |

**D. 3-[(3-Fluorophenyl)methyl]-3,4-dihydro-2,5-furandione**

To a solution of compound C (43.0 g, 0.19 mol) in 100 mL acetic anhydride was added 8 mL acetic acid. The reaction was heated to reflux for 20 minutes and concentrated in vacuo with dry benzene. The crude product was dissolved in 10 mL benzene, 70 mL skelly B was added and upon cooling in an ice bath, a crystalline solid formed. The crystals were collected by filtration and recrystallized in isopropanol/skelly B to give compound D (24.0 g, 61 %), melting point 55 - 57 °C.

| Analysis for $C_{11}H_9FO_3$: | | |
|---|---|---|
| Calc'd: | C, 63.46; | H, 4.36; |
| Found: | C, 63.92; | H, 5.25. |

**E. 7-Fluoro-1,2,3,4-tetrahydro-4-oxo-2-naphthalenecarboxylic acid**

To 500 mL of nitrobenzene was slowly added $AlCl_3$ (30.66 g, 0.23 mol) and compound D (23.85 g, 0.115 mol) keeping the temperature between 20-25 °C. The reaction was stirred at room temperature for 67 hours and was poured into a mixture of 360 g ice and 170 mL concentrated HCl. The nitrobenzene was then removed by distillation. The crude product was crystallized in the ice bath and was recrystallized from benzene/skelly B to give compound E (20.0 g, 84 %), melting point 146 - 147 °C.

| Analysis for $C_{11}H_9FO_3$: | | |
|---|---|---|
| Calc'd: | C, 63.46; | H, 4.36 |
| Found: | C, 63.54; | H, 4.48. |

**F. 7-Fluoro-1,2,3,4-tetrahydro-4-oxo-2-naphthalenecarboxylic acid, methyl ester**

To a solution of compound E (5.0 g, 0.024 mol) in 25 mL methanol was added 1 mL concentrated $H_2SO_4$. The reaction mixture heated to reflux for 40 hours. The reaction mixture was concentrated in vacuo and was partitioned between ethyl acetate and 5 % $NaHCO_3$. The organic layer was washed further with $H_2O$, brine, dried over $Na_2SO_4$ and was concentrated in vacuo. The crude product was crystallized in a mixture of ethyl acetate and skelly B and was recrystallized in hot skelly B to give compound F (4.9 g, 92 %), melting point 90 -92 °C.

| Analysis for $C_{12}H_{11}FO_3$: | | |
|---|---|---|
| Calc'd: | C, 64.86; | H, 4.99; |
| Found: | C, 65.21; | H, 5.21. |

**G. 6-Fluoro-3',4'-dihydrospiro[1,3-dioxolane-2,1'(2'H)-naphthalene]-3'-carboxylic acid, methyl ester**

To a solution of compound F (103.4 g, 0.465 mol) in 700 mL of dry benzene was added ethylene glycol (78.5 mL, 1.395 mol), followed by a catalytic amount of p-toluenesulfonic acid. The reaction was heated to reflux for 66 hours. The reaction mixture was concentrated in vacuo, and the crude product was crystallized in methanol to give compound G (82 g, 66 %), melting point 79-81 °C.

| Analysis for $C_{14}H_{15}FO_4$: | | |
|---|---|---|
| Calc'd: | C, 63.15; | H, 5.67; |
| Found: | C, 63.13; | H, 5.82. |

**H. 6-Fluoro-3',4'-dihydrospiro[1,3-dioxolane-2,1'(2'H)-naphthalene]-3'-methanol**

To a suspension of lithium aluminum hydride (11.25 g, 0.296 mol) in 700 mL of dry tetrahydrofuran was added a solution of compound G (78.8 g, 0.296 mol) in 300 mL tetrahydrofuran. The reaction was heated to reflux for 17 hours and 22.5 mL $H_2O$ and 18 mL 10% NaOH was added with cooling. The reaction was stirred at room temperature for 2 hours. The reaction mixture was filtered and the filtrate was concentrated in vacuo to give compound H (69.4 g, 78 %).

| Analysis for $C_{13}H_{15}FO_3$ | | |
|---|---|---|
| Calc'd: | C, 65.53; | H, 6.35 |
| Found: | C, 65.82; | H, 6.72. |

**I. 6-Fluoro-3',4'-dihydrospiro[1,3-dioxolane-2,1'(2'H)-naphthalene]-3'-methanol, methanesulfonate ester**

To a solution of compound H (61.1 g, 0.256 mol) in 175 mL dry pyridine under nitrogen was added methanesulfonyl chloride (27.15 mL, 0.358 mol) maintaining the temperature between 10 and 15 °C. The reaction was stirred between 5 - 10 °C for 30 minutes and room temperature for 2.5 hours. The reaction mixture was poured into ice-water and extracted with $CH_2Cl_2$. The organic layer was further washed with $H_2O$, brine, dried over $Na_2SO_4$ and was concentrated in vacuo. The crude product was further evaporated

with toluene at 35 °C under water pressure to give compound I (83.7 g, quant.).

**J. 6-Fluoro-3,4-dihydro-3-[[4-(2-methoxyphenyl)-1-piperazinyl]-methyl]-1(2H)-naphthalenone**

To a solution of compound I (10.0 g, 0.0316 mmol) in 150 mL of 25 % methyl isobutyl ketone in absolute ethanol was added $Na_2CO_3$ (2.7 g, 0.0316 mol) and 1-(2-methoxyphenyl)piperazine followed by a catalytic amount of KI. The reaction was heated to reflux for 25 hours and the mixture was filtered. The filtrate was concentrated in vacuo and dissolved in $CH_2Cl_2$. The organic layer was washed with $H_2O$, $NaHCO_3$, brine, dried over $Na_2SO_4$ and was concentrated in vacuo. 15 % HCl (100 mL) was added to the crude and stirred at room temperature for 4 hours. The solution was filtered and was extracted with ethyl ether. The aqueous solution was then basified and extracted with ethyl ether. The final ethyl ether layer was washed with $H_2O$, brine, dried over $Na_2SO_4$ and was concentrated in vacuo. The crude product was recrystallized from methanol twice to give Example 11 (6.57 g, 56 %), melting point 111-113 °C.

| Analysis for $C_{22}H_{25}N_2O_2F$: | | | |
|---|---|---|---|
| Calc'd: | C, 71.72; | H, 6.84; | N, 7.60; |
| Found: | C, 70.11; | H, 7.06, | N, 7.83. |

**Example 12**

**3,4-Dihydro-3-[(4-phenyl-1-piperazinyl)methyl]-1-(2H)-naphthalenone, monohydrochloride**

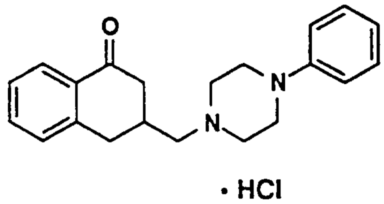

**A. 2-Acetyl-2-(phenylmethyl)butanedioic acid, diethyl ester**

This reaction procedure followed the procedure described in the preparation of compound B of Example 11. The reaction scale is as follows: Benzyl acetoacetate (180 g, 0.86 mol), ethyl chloroacetate (105 g, 0.86 mol), 58.6 % NaH (35.2 g, 0.86 mol) and 300 mL of 10 % dimethylformamide in dry benzene. The reflux time in this reaction was 3 hours and the crude product was purified by distillation at 148-159 °C / 0.3 mmHg to give compound A (164.7 g, 63 %).

**B. 2-(Phenylmethyl)butanedioic acid**

This reaction procedure followed the procedure described in the preparation of compound C of Example 11. The reaction scale is as follows: compound A (164.7 g, 0.54 mol) and 1.5 L of 2 N NaOH. The reaction was reflux for 20 hours and gave compound B (95.8 g, 85 %), melting point 152-156 °C.

**C. 3,4-Dihydro-3-(phenylmethyl)-2,5-furandione**

This reaction procedure was followed as described in the preparation of compound D of Example 11. 95.8 g of compound B gave 72 g (82 %) of compound C, boiling point 156 °C (0.4 mm), and the resulting solid was recrystallzed from hot benzene, melting point 94-96 °C.

**D. 1,2,3,4-Tetrahydro-4-oxo-2-naphthalenecarboxylic acid**

This reaction procedure was followed as described in the preparation of compound E of Example 11. The reaction scale is as follows: compound C (55.7 g, 0.29 mol), $AlCl_3$ (80 g, 0.6 mol) and 280 mL nitrobenzene. The nitrobenzene was removed by distillation and the aqueous was crystallized to give

compound D (50.8 g, 91 %), melting point 145 - 148 °C.

**E. 1,2,3,4-Tetrahydro-4-oxo-2-naphthalenecarboxylic acid, methyl ester**

To a solution of N-nitro-N-methyl urea in 500 mL ether was added 135 mL of 40 % KOH, followed by compound D (50.8 g, 0.27 mol), while cooling in an ice bath. The reaction was stirred at room temperature for 1 hour and acetic acid was added to react with excess diazomethane. The ethyl ether layer was washed with 200 mL of 5 % NaOH, 200 mL of dilute acetic acid, 200 mL of dilute $NaHCO_3$, brine, dried over $Na_2SO_4$ and was concentrated in vacuo. The crude product was isolated by distillation at 124 °C / 0.15 mmHg to give compound E (50.3 g, 91 %).

**F. 3',4'-Dihydrospiro[1,3-dioxolane-2,1'(2'H)-naphthalene]-3'-carboxylic acid, methyl ester**

This reaction procedure was followed as described in the preparation of compound G of Example 11. The reaction scale is as follows: compound E (5.0 g, 0.025 mol), ethylene glycol (4.8 Ml, 0.075 mol), 40 mL dry benzene and a catatalytic amount p-toluenesulfonic acid. The reaction was reflux for 64 hours and was concentrated in vacuo to give compound F (6.0 g, 95 %).

**G. 3',4'-Dihydrospiro[1,3-dioxolane-2,1'(2'H)-naphthalene]-3'-methanol**

This reaction procedure was followed as described in the preparation of compound H of Example 11. The reaction scale is as follows: compound F (7.3 g, 0.028 mol), lithium aluminum hydride (1.06 g, 0.028 mol) and 50 mL dry tetrahydrofuran. The crude product was isolated by distillation at 152-153 °C / 0.15 mmHg to give compound G (4.0 g, 62 %).

| Analysis for $C_{13}H_{16}O_3$: | | |
|---|---|---|
| Calc'd: | C, 70.89; | H, 7.32; |
| Found: | C, 70.73; | H 7.33. |

**H. 3',4'-Dihydrospiro[1,3-dioxolane-2,1'(2'H)-naphthalene]-3'-methanol, methanesulfonate ester**

This reaction procedure was followed as described in the preparation of compound I of Example 11. The reaction scale is as follows: compound G (3.16 g, 0.144 mol), methanesulfonyl chloride (1.6 mL, 0.202 mol) and 30 mL pyridine. The reaction was stirred at room temperature for 2 hours and the crude product was precipitated by pouring onto ice to give compound H (3.35 g, 78 %), melting point 75-79 °C.

**I. 3,4-Dihydro-3-[(4-phenyl-1-piperazinyl)methyl]-1-(2H)-naphthalenone, monohydrochloride**

To a solution of compound H (1.43 g, 0.048 mmol) in 50 mL of a mixture of methyl isobutyl ketone and absolute ethanol was added $Na_2CO_3$ (0.71 g, 0.048 mol) and 1-phenylpiperazine (1.77 g, 0.011 mol). The reaction was heated to reflux for 20 hours and the particles was removed by filtration. The filtrate was concentrated in vacuo and dissolved in ethyl acetate. The organic layer was washed with $H_2O$, 5 % $NaHCO_3$ and was concentrated in vacuo to dryness. 100 mL of 10 % HCl was added to the crude and stirred at room temperature for 4 hours. The mixture was extracted with ethyl ether and the aqueous solution was then basified with concentrated $NH_4OH$ to pH 9 and extracted with ethyl ether. The ethyl ether layer was combined, washed with $H_2O$, brine, dried over $Na_2SO_4$ and concentrated in vacuo. The crude product was redissolved in 200 mL ethyl ether, saturated with HCl, and the solid precipitate was recrystallized from hot ethanol to give Example 12 (0.43 g, 23 %), melting point 243-246 °C.

| Analysis for $C_{21}H_{24}N_2O$ • HCl: | | | | |
|---|---|---|---|---|
| Calc'd: | C, 64.09; | H, 6.67; | N, 7.13; | Cl, 9.95; |
| Found: | C, 70.77; | H, 7.10; | N, 7.69; | Cl, 10.69. |

**Example 13**

**3,4-Dihydro-3-[[4-(2-methoxyphenyl)-1-piperazinyl]carbonyl]-1(2H)-naphthalenone, monohydrochloride**

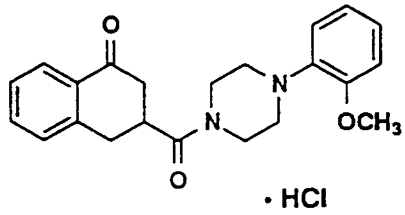

**A. 1,2,3,4-Tetrahydro-4-oxo-2-naphthalenecarboxylic acid**

To a solution of KOH (6.7 g, 0.12 mol) in 60 mL $H_2O$ was added compound E from Example 12 (10.0 g, 0.049 mol). The reaction was warmed gently for 30 minutes and was then cooled to room temperature and was acidified with 1 N HCl. The crude product was filtered, washed with cold $H_2O$ and dried over $P_2O_5$ to give compound A (8.68 g, 93 %), melting point 148-150 °C.

**B. 3,4-Dihydro-3-[[4-(2-methoxyphenyl)-1-piperazinyl]carbonyl]-1(2H)-naphthalenone, monohydrochloride**

To a solution of compound A (9.5 g, 0.05 mmol) and triethylamine (8.38 mL, 0.05 mol) in 125 mL $CH_2Cl_2$ was added isobutyl chloroformate (6.58 mL, 0.05 mol) at -10 °C. The reaction was stirred at -5 to -10 °C for 10 minutes and was followed by 1-(2-methoxyphenyl)piperazine (9.61 g, 0.05 mol) in 25 mL $CH_2Cl_2$. The ice bath was removed and the reaction was stirred at room temperature for 17 hours. The reaction mixture was washed with 5% $NaHCO_3$, $H_2O$, brine, dried over $Na_2SO_4$ and concentrated in vacuo. The crude product was dissolved in ethyl ether, bubbled with HCl and was filtered to give Example 13 (15.45 g, 85 %), melting point 197-199 °C.

| Analysis for $C_{22}H_{24}N_2O_3$ • HCl: | | | | |
|---|---|---|---|---|
| Calc'd: | C, 65.89; | H, 6.28; | N, 6.99; | Cl, 8.85; |
| Found: | C, 66.27; | H, 6.41; | N, 7.35; | Cl, 9.58. |

**Example 14**

**3,4-Dihydro-3-[[4-(2-methoxyphenyl)-1-piperazinyl]methyl]-1(2H)-naphthalenone, dihydrochloride**

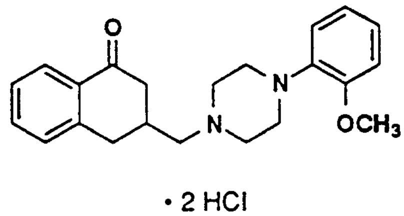

**A. 1,2,3,4-Tetrahydro-3-[[4-(2-methoxyphenyl)-1-piperazinyl]methyl]-1-naphthalenol**

To a solution of the free base of compound B of Example 13 (11.74 g, 0.0322 mol) in 50 mL of dry tetrahydrofuran was added lithium aluminum hydride (2.45 g, 0.0644 mol) in 50 mL of dry tetrahydrofuran. The reaction was heated to reflux for 22 hours. The reaction was mixed with 5 mL $H_2O$, 4 mL of 10 % NaOH and was stirred at room temperature for 2 hours. The solids were removed by filtration, washed with

tetrahydrofuran and concentrated in vacuo to give compound A (10.1 g, 89 %).

| Analysis for $C_{22}H_{28}N_2O_2$ • 2 HCl • $H_2O$: | | | | | |
|---|---|---|---|---|---|
| Calc'd:<br>Found: | C, 59.59;<br>C, 59.45; | H, 7.27;<br>H, 7.10; | N, 6.32;<br>N, 6.50; | Cl, 15.99;<br>Cl, 16.49; | KF, 4.06;<br>KF, 4.36. |

**B. 3,4-Dihydro-3-[[4-(2-methoxyphenyl)-1-piperazinyl]methyl]-1(2H)-naphthalenone, dihydrochloride**

To a solution of compound A (4.91 g, 0.014 mmol) in 120 mL benzene was added potassium tert-butoxide (3.93 g, 0.035 mol) and benzophenone (11.8 g, 0.065 mol). The reaction was refluxed for 16 hours and washed with $H_2O$. The organic layer was washed further with brine, dried over $Na_2SO_4$ and concentrated in vacuo to dryness. The crude product was dissolved in ethyl ether, bubbled with HCl salt, recrystallized from methanol / ethyl ether to give Example 14 (5.2 g, 87 %), melting point 218-219 °C.

| Analysis for $C_{22}H_{26}N_2O_2$ • 2 HCl | | | | |
|---|---|---|---|---|
| Calc'd:<br>Found: | C, 62.41;<br>C, 62.61; | H, 6.67;<br>H, 6.87; | N, 6.62;<br>N, 6.37. | Cl, 16.75; |

**Example 15**

**5-Chloro-2,3-dihydro-2-[1-(phenylmethyl)-4-piperidinyl]-1H-isoindol-1-one, monohydrochloride**

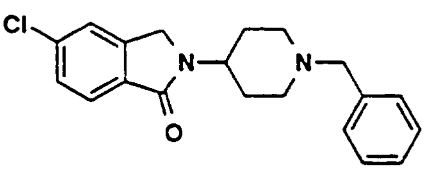

**• HCl**

**A. 5-Chloro-1,3-isobenzofurandione**

4-Chlorophthalic acid (446.5 g, 2.23 mol) was heated neat until $H_2O$ was no longer released to give compound A (415.9 g, quantitative), melting point 138-140 °C.

**B. 5-Chloro-1H-isoindole-1,3(2H)-dione**

A solution of compound A (415.9 g, 2.28 mol) and 1000 mL of 28 % ammonium hydroxide was heated at 300 °C until $H_2O$ was no longer released to give compound B (361.0 g, 89 %).

**C. 5-Chloro-2-[1-(phenylmethyl)-4-piperidinyl]-1H-isoindole-1,3(2H)-dione**

To a solution of compound B (10.0 g, 55.2 mmol) in 100 mL amyl alcohol was added 4-amino-1-benzylpiperidine (10.5 g, 55.2 mmol). The reaction was heated to reflux for 16 hours. The reaction mixture was concentrated in vacuo and dissolved in 250 mL $CHCl_3$. The $CHCl_3$ layer was washed with $H_2O$, dried over $Mg_2SO_4$ and was concentrated in vacuo. The crude product was dissolved in 400 mL isopropyl ether, treated with charcoal and filtered. The filtrate was acidifed with 4 N HCl in dioxane to give compound C (19.0 g, 97 %) as a white solid, melting point 233 - 234.5 °C.

| Analysis for $C_{20}H_{19}ClN_2O_2 \cdot HCl$: | | | |
|---|---|---|---|
| Calc'd: | C, 61.40; | H, 5.16; | N, 7.16; |
| Found: | C, 62.04; | H, 5.64; | N, 7.31. |

**D. 5-Chloro-2,3-dihydro-2-[1-(phenylmethyl)-4-piperidinyl]-1H-isoindol-1-one, monohydrochloride**

To a solution of compound C (5.5 g, 14.1 mmol) in 40 mL acetic acid and 8.25 mL concentrated HCl was added tin (4.2 g, 35.3 mmol). The reaction was heated at 95-100 °C for 16 hours, treated with 5 % NaOH to pH greater than 9, and extracted with $CHCl_3$. The organic layer was dried over $Mg_2SO_4$ and was concentrated in vacuo to the dryness. The crude product was dissolved in 200 mL $H_2O$ and treated with HCl in dioxane to give Example 15 (4.64 g, 87 %), melting point 269-271 °C.

| Analysis for $C_{20}H_{21}ClN_2O$ + 0.8 HCl + 0.2 $H_2O$: | | | | | |
|---|---|---|---|---|---|
| Calc'd: | C, 64.29; | H, 5.99; | N, 7.50; | Cl, 17.08; | $H_2O$, 0.96; |
| Found: | C, 64.19; | H, 6.05; | N, 7.54; | Cl, 16.96; | $H_2O$, 0.95. |

**Example 16**

**2,3-Dihydro-2-[1-[3-(2-phenoxyphenyl)propyl]-4-piperidinyl]-1H-isoindol-1-one, monohydrochloride**

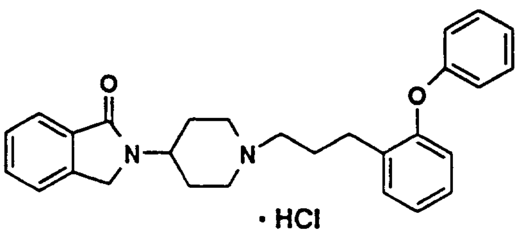

**A. 2,3-Dihydro-2-[1-[3-(2-phenoxyphenyl)propyl]-4-piperidinyl]-1H-isoindol-1-one**

To a solution of compound F from Example 9 (450 mg, 1.06 mmol) in ethanol (10 mL) was added 10% palladium on activated carbon (45 mg) under argon at room temperature. Argon on the reaction was replaced by hydrogen. A hydrogen balloon was connected to the solution. Hydrogenation was maintained overnight. The reaction was filtered through Celite, and the filtrate was evaporated to dryness. Purification was performed by flash chromatography on 100 g silica gel, loaded and eluted with 1.5% methanol in dichloromethane. Pure fractions were combined and evaporated to give compound A (450 mg, 100%) as a colorless oil.

**B. 2,3-Dihydro-2-[1-[3-(2-phenoxyphenyl)propyl]-4-piperidinyl]-1H-isoindol-1-one, monohydrochloride**

Compound A (450 mg, 1.06 mmol) was dissolved in 20% methanol in ethyl ether (2 mL). A solution of 1 M HCl in ethyl ether (2 mL, 2.0 mmol) was added. The HCl salt precipitated and was filtered and washed with ethyl ether. Dichloromethane (80 mL) was added to dissolve the solid, and the organic layer was washed with saturated sodium bicarbonate solution (2 x 30 mL). Evaporation gave a colorless oil. Purification was performed by flash chromatography, loaded and eluted with 1.5% methanol in dichloromethane. Pure fractions were combined and evaporated to give a colorless oil. The resulting oil was dissolved in 20% methanol in hexane. A solution of 1 M HCl in ethyl ether (1 mL, 1.0 mmol) was added. The HCl salt precipitated and was filtered and washed ethyl ether. The resulting solid was dried under high vacuum at 60 °C overnight to give Example 16 (160 mg, 35%) as a white solid, melting point 199-202 °C.

| Analysis. for $C_{28}H_{31}ClN_2O_2$ • 0.5 $H_2O$: | | | |
|---|---|---|---|
| Calc'd: | C, 71.25; | H, 6.83; | N, 5.93; |
| Found: | C, 71.13; | H, 6.78; | N, 5.93. |

**Example 17**

**(Z)-2,3-Dihydro-2-[1-(5,5-diphenyl-2-pentenyl)-4-piperidinyl]-1H-isoindol-1-one, monohydrochloride**

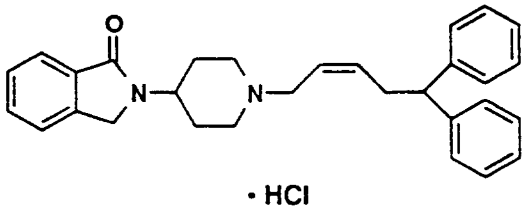

**• HCl**

**A. (Z)-5,5-Diphenyl-2-pentenoic acid, methyl ester**

To a suspension of bis(2,2,2-trifluoroethyl)-(methoxycarbonylmethyl)phosphonate (4.16 g, 13.1 mmol) and 18-crown-6 (3.46 g, 13.1 mmol) in tetrahydrofuran (65 mL) at 0 °C was added dropwise 0.5 M potassium bis(trimethylsilyl)amide in toluene (26.2 mL, 13.1 mmol). The reaction was stirred at 0 °C for 15 minutes, then cooled to -78 °C. A solution of compound A from Example 5 (5.0 g, 23.8 mmol) in tetrahydrofuran (5 mL) was added dropwise. The reaction was stirred at -78 °C for 1 hour, then warmed to room temperature and quenched with saturated ammonium chloride solution (5 mL). Ethyl ether (200 mL) was added to dilute the reaction, and the organic layer was washed with water (2 x 50 mL), brine (2 x 50 mL) and dried over $MgSO_4$. Evaporation gave a crude oil. Purification was performed by flash chromatography on 250 g silica gel, loaded and eluted with 6% ethyl acetate in hexane. Pure fractions were combined and evaporated to give compound A (2.2 g, 70%) as a colorless oil.

**B. (Z)-5,5-Diphenyl-2-penten-1-ol**

To a solution of compound A (2.2 g, 8.27 mmol) in toluene (20 mL) at 0 °C was added dropwise diisobutylaluminum hydride (1.0 M in toluene, 18.2 mL, 18.2 mmol). The reaction was stirred at 0 °C for 1 hour. The reaction was quenched with methanol (5 mL). Potassium sodium tartrate solution (1 M, 200 mL) was added, and the reaction mixture was stirred for 3.5 hours. Ethyl ether (200 mL) was added, and the organic layer was washed with water (2 x 50 mL), brine (2 x50 mL) and dried over $MgSO_4$. Evaporation gave a crude oil. Purification was performed by flash chromatography on 300 g silica gel, loaded and eluted with 20% ethyl acetate in hexane. Pure fractions were combined and evaporated to give compound B as a colorless oil (1.9 g, 97%).

**C. (Z)-1-Chloro-5,5-diphenyl-2-pentene**

To a solution of N-chlorosuccinimide (0.56 g, 4.16 mmol) in dichloromethane (12 mL) at -40 °C was added dropwise methyl sulfide (0.4 mL, 5.29 mmol). The reaction was stirred at -40 °C for 10 minutes then warmed to room temperature for 30 minutes. The reaction was recooled to -40 °C, and a solution of compound B (0.9 g, 3.78 mmol) in dichloromethane (5 mL) was added dropwise. The reaction was stirred at -40 °C for 2 hours then warmed to room temperature for 30 minutes. Hexane (300 mL) was added to dilute the reaction and the organic layer was washed with water (2 x 50 mL), brine (2 x 50 mL) and dried over $MgSO_4$. Evaporation gave compound C (0.9 g, 93%) as a colorless oil.

**D. (Z)-2,3-Dihydro-2-[1-(5,5-diphenyl-2-pentenyl)-4-piperidinyl]-1H-isoindol-1-one**

To a solution of compound A from Example 2 (756 mg, 3.50 mmol) in dimethylformamide (12 mL) was added compound C (900 mg, 3.50 mmol) followed by anhydrous potassium carbonate (531 mg, 3.85 mmol). The reaction was stirred at 50 °C overnight. The reaction was cooled to room temperature. Ethyl

ether (100 mL) was added to dilute the reaction, and the organic layer was washed with water (2 x 50 mL), brine (2 x 50 mL) and dried over $Na_2SO_4$. Evaporation gave a crude oil. Purification was performed by flash chromatography on 100 g of silica gel, loaded and eluted with 2% methanol in dichloromethane. Pure fractions were combined and evaporated to give compound D (950 mg, 62%) as a white solid, melting point 138-140 °C.

**E. (Z)-2,3-Dihydro-2-[1-(5,5-diphenyl-2-pentenyl)-4-piperidinyl]-1H-isoindol-1-one, monohydrochloride**

To a solution of compound D (500 mg, 1.15 mmol) in methanol (2 mL) was added 1 M HCl in ethyl ether (1.5 mL, 1.5 mmol). The mixture was evaporated to dryness. The resulting white solid was dried at 60 °C under vacuum to give Example 17 (300 mg, 80%) as a white solid, melting point 174-177 °C.

| Analysis for $C_{30}H_{33}ClN_2O$ + 1.2 $H_2O$: | | | | |
|---|---|---|---|---|
| Calc'd: | C, 72.84; | H, 7.21; | N, 5.66; | Cl, 7.17; |
| Found: | C, 72.74; | H, 6.88; | N, 5.70; | Cl, 7.42. |

## Example 18

**N-[1-(3,3-Diphenylpropyl)-4-piperidinyl]-2-methoxybenzamide, monohydrochloride**

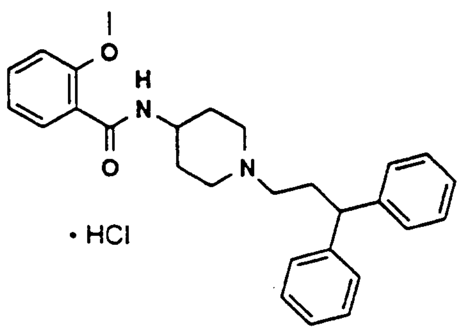

**A. N-[1-(3,3-Diphenylpropyl)-4-piperidinyl]-2-methoxybenzamide**

To a stirred solution of 503 mg (1.52 mmol) of compound E from Example 1 in 8 mL of methylene chloride at 0 °C were added 370 µL (4.52 mmol) of pyridine and 238 µL (1.59 mmol) of o-anisoyl chloride. After warming to room temperature, the mixture was stirred for 1 h then diluted with methylene chloride and water. The organics were separated and the aqueous layer basified with 1 N KOH and extracted with methylene chloride. The combined organics were dried (sodium sulfate) and concentrated to provide a yellow oil which was dried under high vacuum. Flash chromatography on silica gel (180 g) eluted with 2% methanol in ethyl acetate afforded 336 mg (56%) of title compound A as a yellow solid, melting point 96-98 °C.

**B. N-[1-(3,3-Diphenylpropyl)-4-piperidinyl]-2-methoxybenzamide, monohydrochloride**

To a solution of 364 mg (0.85 mmol) of compound A in 4 mL of methylene chloride was added a freshly prepared saturated solution of hydrogen chloride in diethyl ether. The opaque white mixture was concentrated and dried to provide 329 mg (83%) of Example 18 as an off-white solid, melting point 170-172 °C.

| Analysis for $C_{28}H_{33}N_2O_2Cl + 1.11\ H_2O$: | | | |
|---|---|---|---|
| Calcd.<br>Found | C, 69.34;<br>C, 69.41; | H, 7.32;<br>H, 7.31; | N, 5.78;<br>N, 5.71. |

**Example 19**

**N-[1-(3,3-Diphenylpropyl)-4-piperidinyl]-2-methylbenzamide, monohydrochloride**

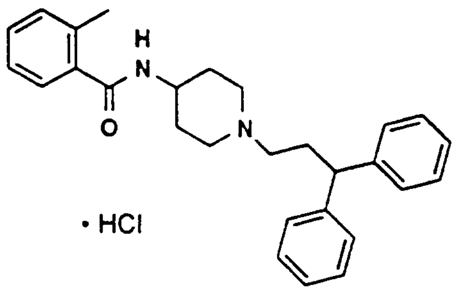

**A. N-[1-(3,3-Diphenylpropyl)-4-piperidinyl]-2-methylbenzamide**

Compound A was prepared as described for compound A in Example 18, using 485 mg (1.46 mmol) of compound E from Example 18, 336 µL (4.38 mmol) of pyridine, and 200 µL (1.54 mmol) of o-toluoyl chloride. The crude product was purified by flash chromatography on silica gel eluted with 98:2 ethyl acetate/methanol to provide 345 mg (67%) of compound A as a yellow solid.

**B. N-[1-(3,3-Diphenylpropyl)-4-piperidinyl]-2-methylbenzamide, monohydrochloride**

To a solution of 342 mg (0.83 mmol) of compound A in 2 mL of methylene chloride was added a freshly prepared saturated solution of hydrogen chloride in diethyl ether. The opaque white mixture was concentrated, evaporated from methylene chloride to remove residual ether, and dried under vacuum to provide 348 mg (94%) of Example 19 as a white solid, melting point 237-239 °C.

| Analysis for $C_{28}H_{33}N_2OCl \bullet 1.15\ H_2O$: | | | | |
|---|---|---|---|---|
| Calc'd:<br>Found: | C, 71.60;<br>C, 71.59; | H, 7.57;<br>H, 7.31; | N, 5.96,<br>N, 5.97, | Cl, 7.55;<br>Cl, 7.86. |

## Example 20

**N-[1-(3,3-Diphenylpropyl)-4-piperidinyl]-2-pyridineamide, monohydrochloride**

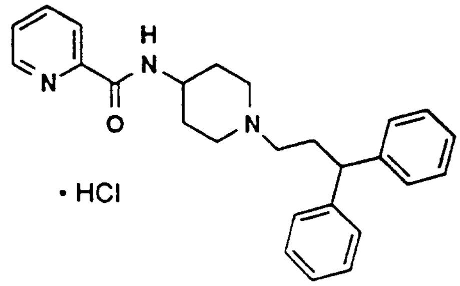

**A. N-[1-(3,3-Diphenylpropyl)-4-piperidinyl]-2-pyridineamide**

To a stirred suspension of 199 mg (1.62 mmol) of picolinic acid in 2.5 mL of methylene chloride at 0 °C was added 225 μL (1.62 mmol) of triethylamine. After all the solids had dissolved, the solution was treated with 412 mg (1.62 mmol) of bis(2-oxo-3-oxazolidinyl)phosphinic chloride, and stirred for 30 minutes. A methylene chloride solution of 535 mg (1.62 mmol) of compound E from Example 1 was converted to the free amine by washing with sodium bicarbonate and concentrating the organic layer to a brown oil which was redissolved in 1 mL of dry methylene chloride and added to the reaction mixture. After stirring at room temperature for 16 hours, the reaction was quenched with water and 4 M HCl and diluted with methylene chloride. The aqueous layer was basified with 1 N KOH and extracted two times. The combined organics were dried (sodium sulfate) and concentrated to provide 554 mg of a brown oil. The crude product was purified by flash chromatography on silica gel eluted with 98:2 ethyl acetate / methanol to provide 316 mg (58%) of compound A as a brown glass.

**B. N-[1-(3,3-Diphenylpropyl)-4-piperidinyl]-2-pyridinamide, monohydrochloride**

The hydrochloride salt of compound A was prepared by the procedure used for compound B in Example 18, using 316 mg (0.83 mmol) of compound A, to afford 336 mg (83%) of Example 20 as a yellow solid, melting point 109-116 °C.

| Analysis for $C_{26}H_{30}N_3OCl + 1.42\ H_2O$ | | | |
|---|---|---|---|
| Calc'd: | C 67.65, | H 7.17, | N 9.10; |
| Found: | C 67.53, | H 7.10, | N 9.22. |

## Example 21

**N-[1-(3,3-Diphenylpropyl)-4-piperidinyl]-N-methylbenzamide, monohydrochloride**

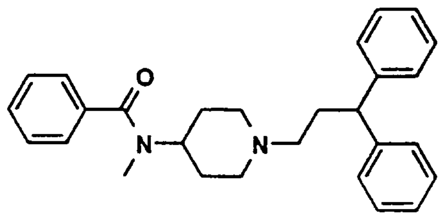

• HCl

**A. N-[1-(3,3-Diphenylpropyl)-4-piperidinyl]-N-methylamine**

To a solution of 550 mg (1.4 mmol, 1 eq) of compound D from Example 1 in 5 mL of tetrahydrofuran at 0 °C was added 8.4 mL (8.4 mmol, 6 eq) of a 1 M solution of lithium aluminum hydride in tetrahydrofuran and the reaction was allowed to warm to room temperature. After 15 hours, the reaction was heated at 60 °C for 4 hours, then quenched by slow addition of a saturated aqueous solution of $Na_2SO_4$. To the resulting heterogeneous mixture was added solid $Na_2SO_4$ and the mixture was stirred for 30 minutes. The solids were removed by filtration and rinsed well with ethyl acetate. Concentration of the organic filtrate afforded 400 mg (93%) of compound A as a viscous pale yellow oil which was used without further purification.

**B. N-[1-(3,3-Diphenylpropyl)-4-piperidinyl]-N-methylbenzamide**

Compound B was prepared from 390 mg (1.3 mmol) of compound A, 158 µL (1.4 mmol) of pyridine and 166 µL (1.4 mmol) of benzoyl chloride as described for compound A in Example 18. Flash chromatography on silica gel (75 g) eluted with 1.5% methanol in t-butylmethylether afforded 472 mg (88%) of compound B as a pale yellow oil.

**C. N-[1-(3,3-Diphenylpropyl)-4-piperidinyl]-N-methylbenzamide, monohydrochloride**

To a solution of 460 mg (1.1 mmol, 1 eq) of compound B in 5 mL of ether and 1 mL of $CH_2Cl_2$ was added an excess of HCl as a saturated solution in ether and the resulting heterogeneous mixture was stirred for 20 min. The solid was isolated by filtration, rinsed well with ether, concentrated and the solvent remnants were removed in a vacuum oven at 52 °C and full vacuum to afford 540 mg (82%) of Example 21 as a white solid; melting point 216-217 °C.

| Analysis for $C_{34}H_{37}N_2OCl$ : | | | | |
|---|---|---|---|---|
| Calc'd:<br>Found: | C 74.90,<br>C 74.64, | H 7.41,<br>H 7.38, | N 6.24,<br>N 6.35, | Cl 7.90<br>Cl 7.75 |

## Examples 22 to 204

Additional compounds falling within the scope of the present invention are described by the following structures. Substituents for each example are identified in the table following each structure.

## Table A

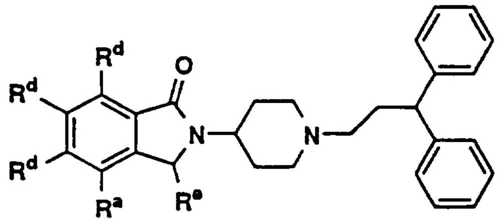

| Ex. No. | $R^a$ | $R^b$ | $R^c$ | $R^d$ | $R^e$ |
|---|---|---|---|---|---|
| 22 | H | H | H | F | H |
| 23 | H | H | H | 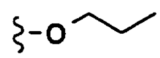 | H |
| 24 | H | H | F | Cl | H |
| 25 | H | H | $CF_3$ | H | H |
| 26 | H | $OCH_3$ | H | H | 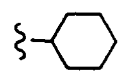 |
| 27 | H | H | H | 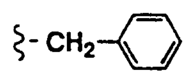 | H |
| 28 | 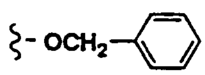 | H | H | H | H |
| 29 | H | H | 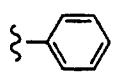 | H | 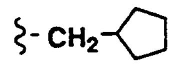 |
| 30 | F | Cl | H | H | H |
| 31 | H | H | H | 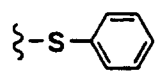 | H |
| 32 | H | H | Cl | H | H |
| 33 | H | H | H | 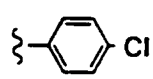 | H |
| 34 | H | H | H | H | 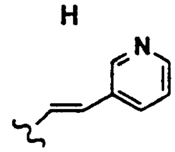 |
| 35 | H | H | H | Cl | H |
| 36 | H | H | $CH_3$ | H | H |
| 37 | H | CH3 | H | 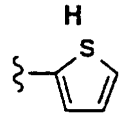 | H |

Table A (continued)

| Ex. No. | $R^a$ | $R^b$ | $R^c$ | $R^d$ | $R^e$ |
|---|---|---|---|---|---|
| 38 | $SCH_3$ | H | H | H | H |
| 39 | H | H | $OCH_3$ | H | butyl |
| 40 | H | H | H | $SCH_3$ | H |
| 41 | H | H | H | H | pentyl |
| 42 | H | H | H | 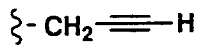 | H |
| 43 | H | 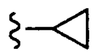 | H | H | H |
| 44 | H | H | H | 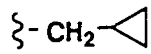 | H |

Examples 45 to 63

**Table B**

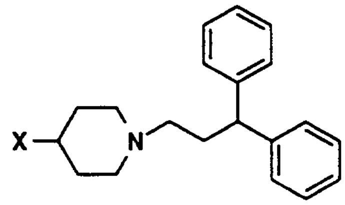

**Examples of X**

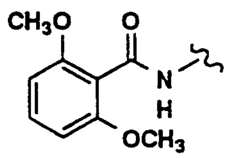
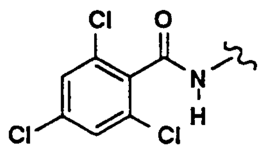
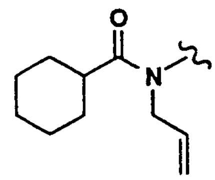

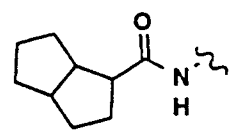
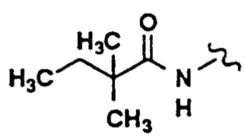
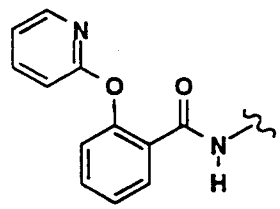

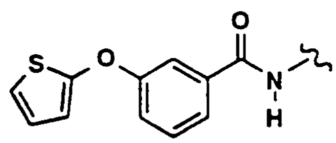
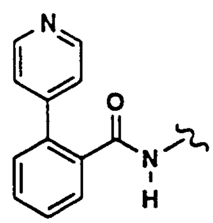
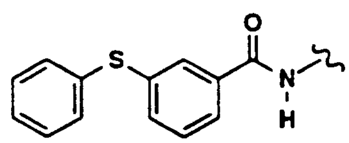

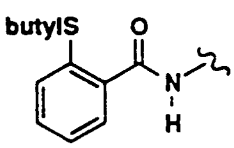
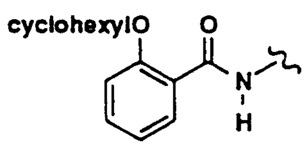
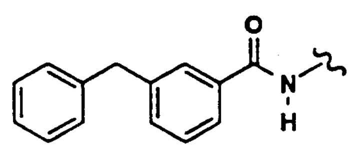

## Table B (continued)

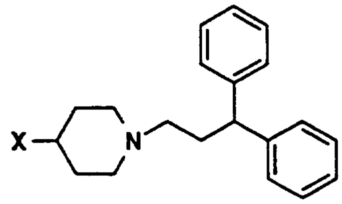

| Examples of X | | |
|---|---|---|
| 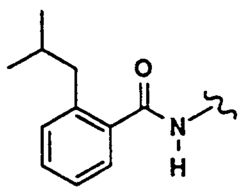 | 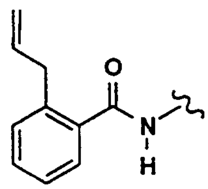 | 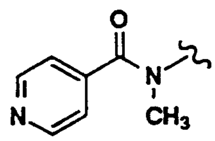 |
| 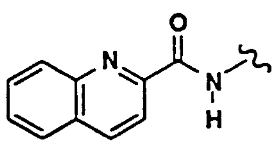 | 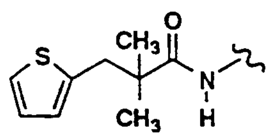 | 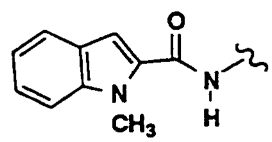 |
| 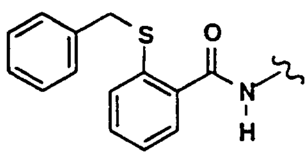 | | |

Examples 64 to 204

## Table C

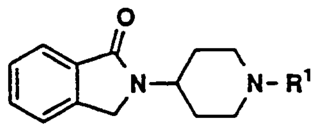 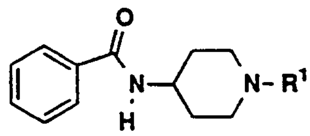 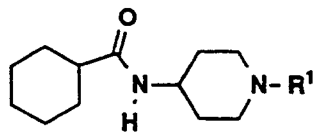

Examples of $R^1$

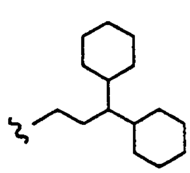 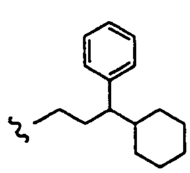 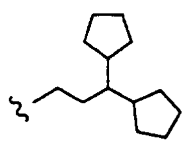 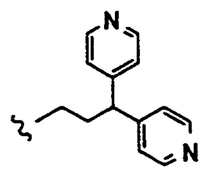

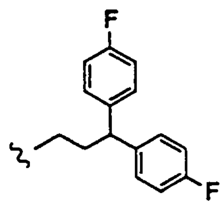 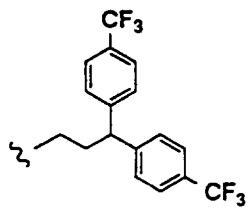 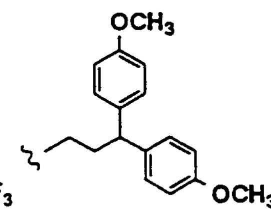 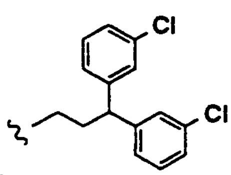

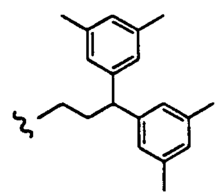 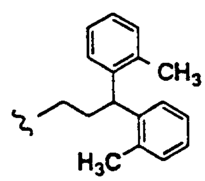 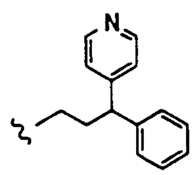 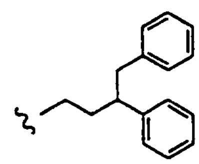

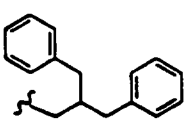 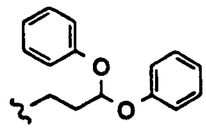 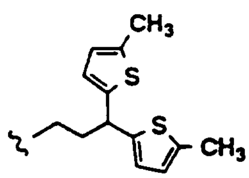 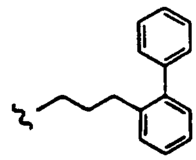

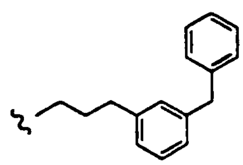 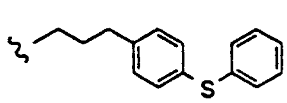 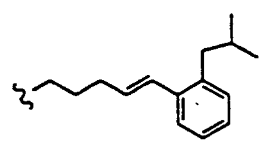

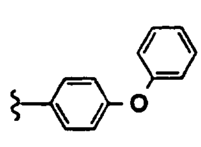 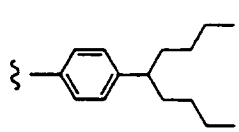 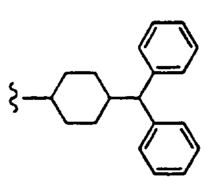 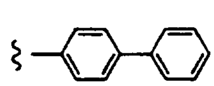

## Table C (continued)

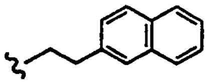
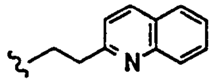
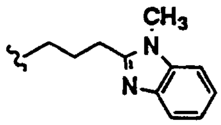
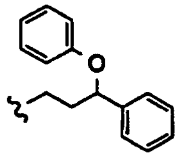
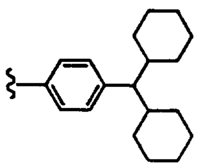
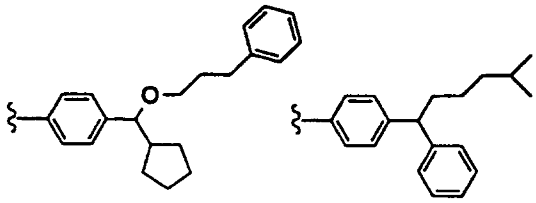
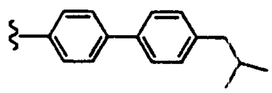
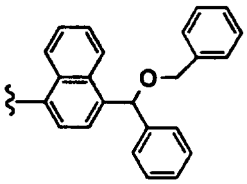
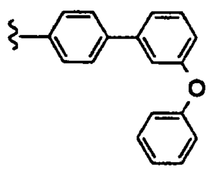
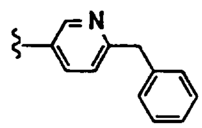
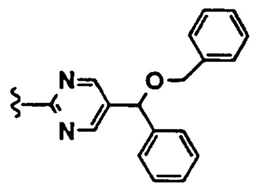
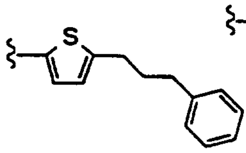
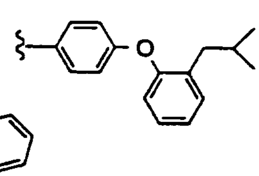
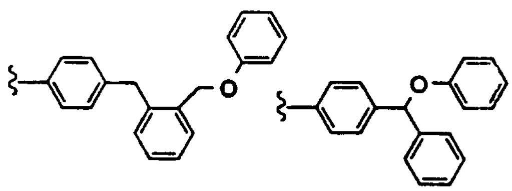
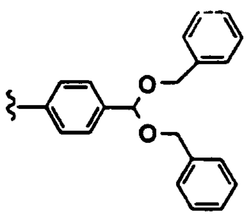
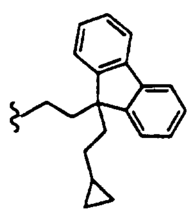
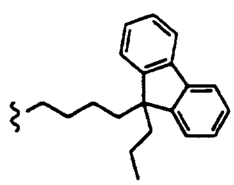
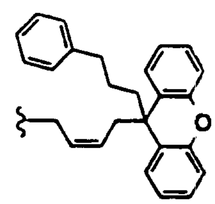
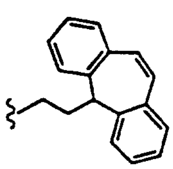
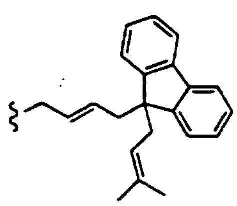
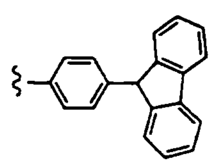
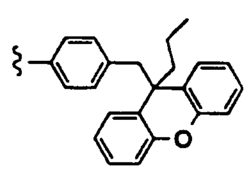

Example 205

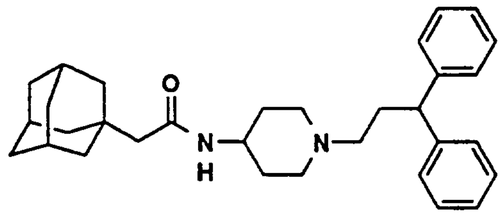

A. 1-(3,3-Diphenylpropyl)-4-piperidinamine hydrochloride

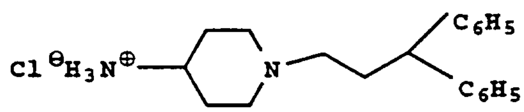

The title compound was prepared as described in Example 1, Part E.

B. 1-(3,3-Diphenylpropyl)-4-piperidinamine

A 2.2 g sample of Part A compound was suspended in $CH_2Cl_2$ (25 mL) and washed with 1 N KOH (15 mL). The organic solution was filtered through cotton and concentrated to afford 1.68 g (95%) of Part B amine as a clear oil which was used without further purification or characterization.

C.

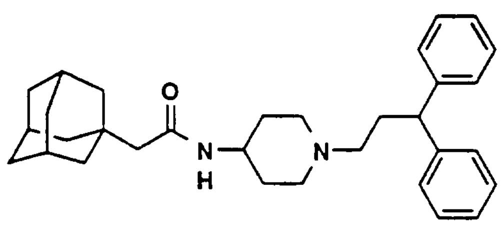

The coupling of carboxylic acid to the Part B amine was carried out using a standard carbodiimide mediated coupling. The process was automated by using a Zymark Benchmate® robotic workstation to carry out the acid-amine coupling and the purification of the resulting amide products. An IBM PC was used to run the Zymark Benchmate® workstation operating program and to write the Benchmate® procedures. The standard protocol for preparation of amides from the Part B free diamine and a carboxylic acid was as follows:

A 16 mm x 100 mm tube was charged with 47 mg (0.24 mmol, 4 eq) of adamantane acetic acid and capped loosely with a plastic cap/column holder. The Benchmate® then carried out the following steps on the tube:

1) Added 500 $\mu$L (1 2.5 mg, 0.092 mmol, 1.5 eq) of a 25 mg/mL solution of 1-hydroxybenzotriazole in DMF

2) Added 500 µL (11.5 mg, 0.092 mmol, 1.5 eq) of a 23 mg/mL solution of diisopropylcarbodiimide in $CH_2Cl_2$

3) Added 500 µL (18 mg, 0.061 mmol, 1 eq) of a 36 mg/mL solution of Part B diamine in $CH_2Cl_2$

4) Washed syringe with 3mL of $CH_2Cl_2$

5) Mixed tube contents by vortexing at speed 3 for 15 sec.

After 19 h, the reaction was complete (no starting Part B diamine remained as determined by TLC; 10% MeOH + 1% $NH_4OH$ in $CH_2Cl_2$, $I_2$; $R_f$[diamine] = 0.13).

The reaction mixture contents were then purified by ion exchange chromatography on a solid phase extraction cartridge mediated by the Benchmate® robotic workstation using the following protocol:

1) Condition a Varian solid phase extraction column (500 mg, SCX cation exchange) with 10 mL of MeOH at 0.25 mL/sec

2) Load reaction contents onto column at 0.05 mL/sec

3) Wash column with 2 x 10 mL of MeOH at 0.1 mL/sec

4) Wash column with 2 mL of 0.1 M ammonia in MeOH at 0.1 mL/sec

5) Elute column with 2 mL of 1 M ammonia in MeOH and collect into a tared receiving tube at 0.1 mL/sec.

All solution/solvent deliveries were followed by 1.8 mL of air and a 10 sec push delay was used after loading reaction contents onto the ion exchange column.

The product solution was concentrated on a Savant Speed Vac (approx. 2 mm Hg for 5 h) and final solvent remnants were removed by further exposure to high vac (0.015 mm Hg, 14 h) to afford 22 mg (77% yield) of title compound. Products were characterized by HPLC and MS.

M.S. (electrospray, pos. ions) 471 (M+H).

Examples 206 to 276

Following the procedure of Example 205, the following compounds of the invention were prepared.

206.

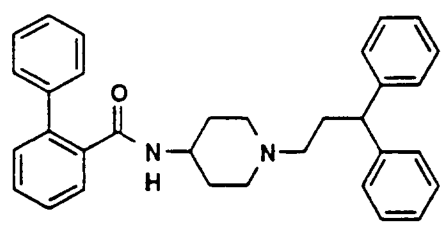

M.S. (electrospray, pos. ions) 475 (M + H).

207.

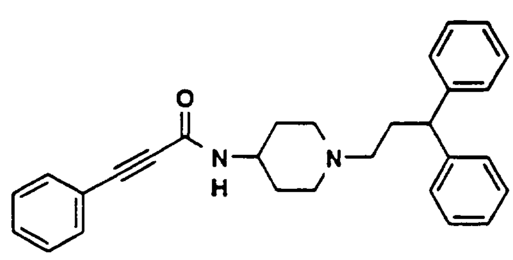

M.S. (electrospray, pos. ions) 423 (M + H).

208.

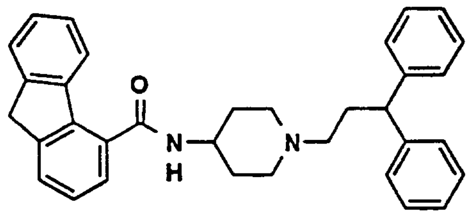

M.S. (electrospray, pos. ions) 487 (M + H).

209.

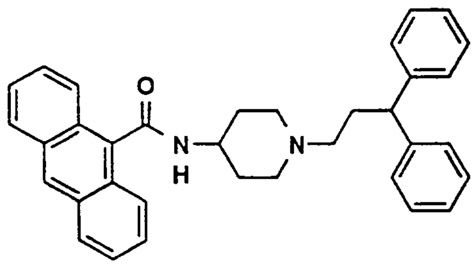

M.S. (electrospray, pos. ions) 499 (M + H).

210.

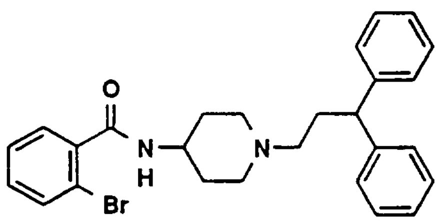

M.S. (electrospray, pos. ions) 477 (M + H), 479 (M + H + 2).

211.

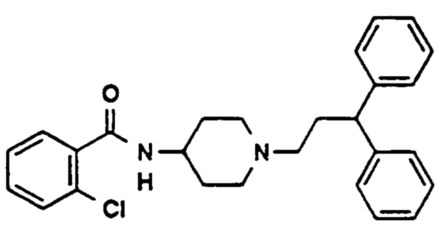

M.S. (electrospray, pos. ions) 433 (M + H).

212.

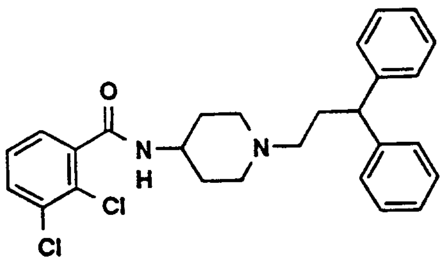

M.S. (electrospray, pos. ions) 467 (M + H), 469 (M + H + 2).

213.

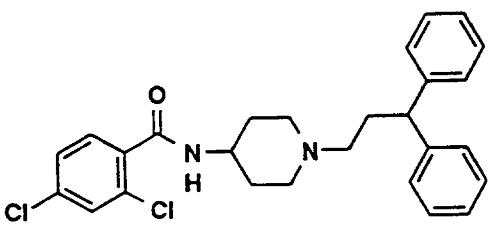

M.S. (electrospray, pos. ions) 467 (M + H), 469 (M + H + 2).

214.

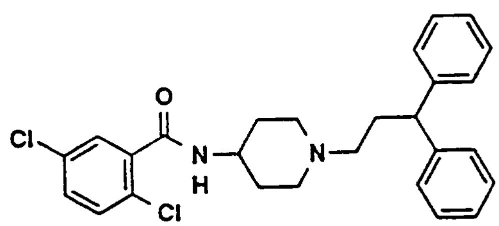

M.S. (electrospray, pos. ions) 467 (M + H), 469 (M + H + 2).

215.

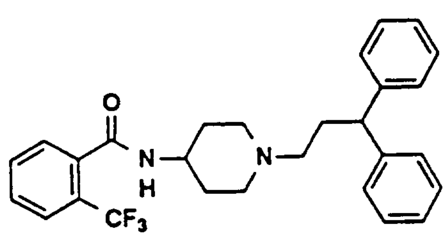

M.S. (electrospray, pos. ions) 467 (M + H).

216.

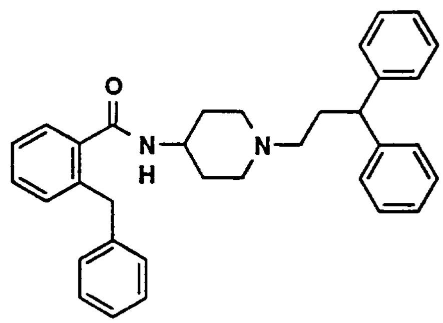

M.S. (electrospray, pos. ions) 489 (M + H).
217.

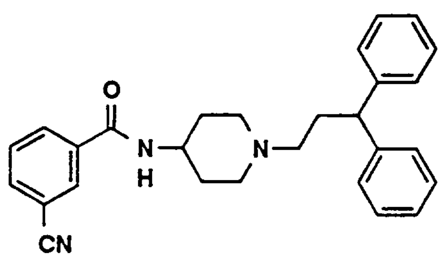

M.S. (electrospray, pos. ions) 424 (M + H).
218.

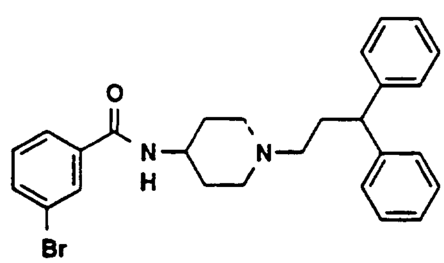

M.S. (electrospray, pos. ions) 477 (M + H), 479 (M + H + 2).
219.

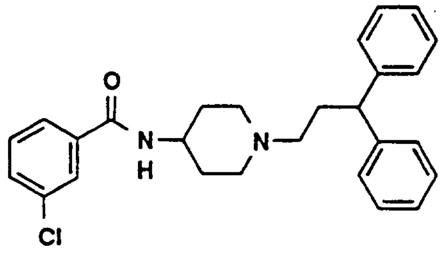

M.S. (electrospray, pos. ions) 433 (M + H).
220.

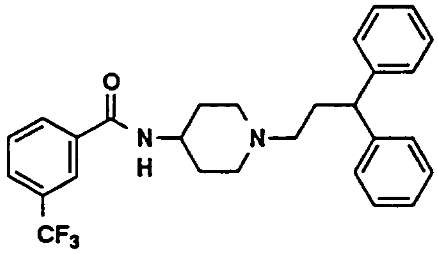

M.S. (electrospray, pos. ions) 467 (M + H).
221.

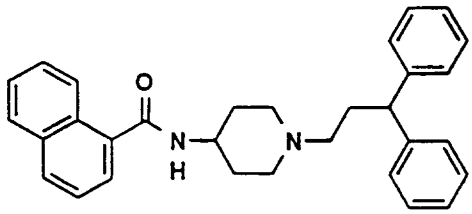

M.S. (electrospray, pos. ions) 449 (M + H).
222.

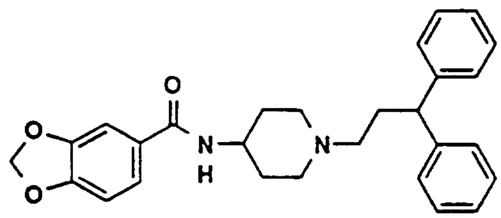

M.S. (electrospray, pos. ions) 443 (M + H).
223.

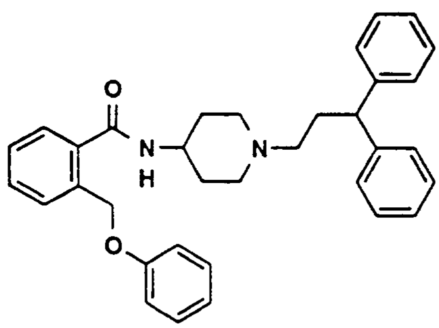

M.S. (electrospray, pos. ions) 505 (M + H).
224.

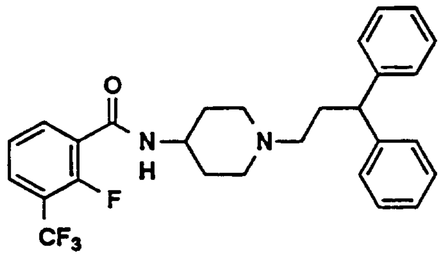

M.S. (electrospray, pos. ions) 485 (M + H).

225.

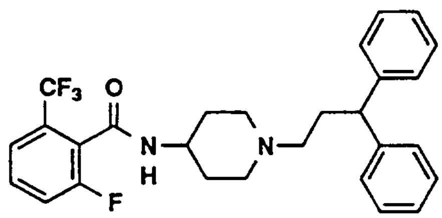

M.S. (electrospray, pos. ions) 485 (M + H).

226.

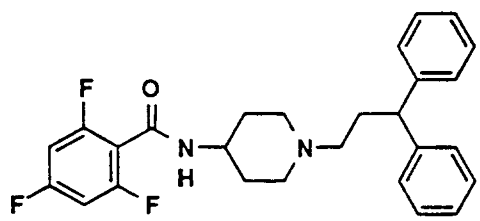

M.S. (electrospray, pos. ions) 453 (M + H).

227.

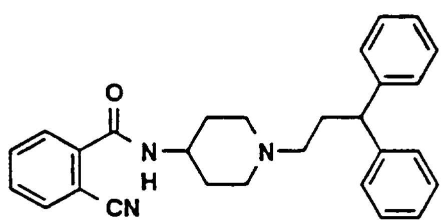

M.S. (electrospray, pos. ions) 424 (M + H).

228.

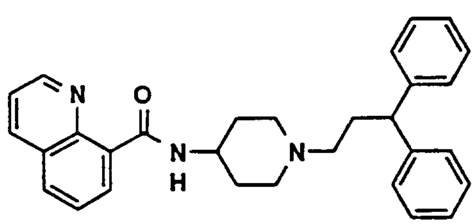

M.S. (electrospray, pos. ions) 450 (M + H).

229.

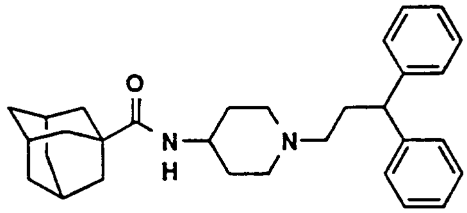

M.S. (electrospray, pos. ions) 457 (M + H).

230.

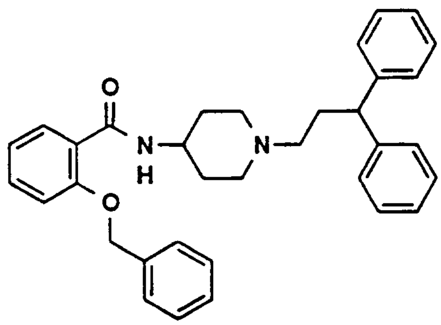

M.S. (electrospray, pos. ions) 505 (M + H).

231.

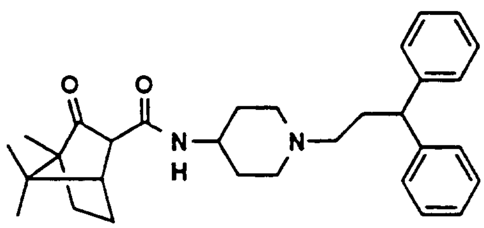

M.S. (electrospray, pos. ions) 473 (M + H).

232.

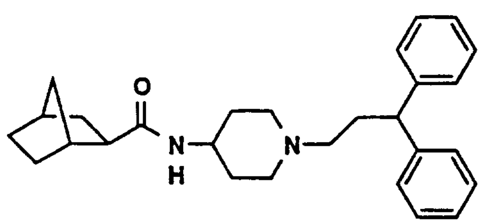

M.S. (electrospray, pos. ions) 417 (M + H).

233.

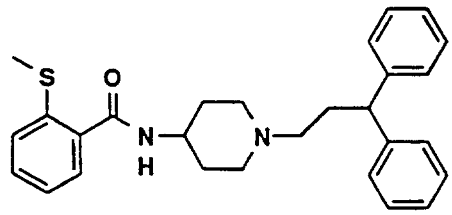

M.S. (electrospray, pos. ions) 445 (M + H).
234.

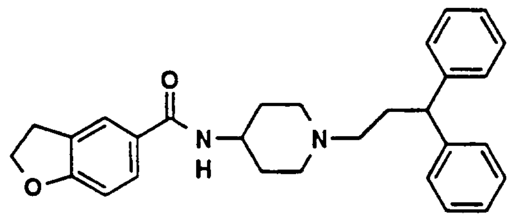

M.S. (electrospray, pos. ions) 441 (M + H).
235.

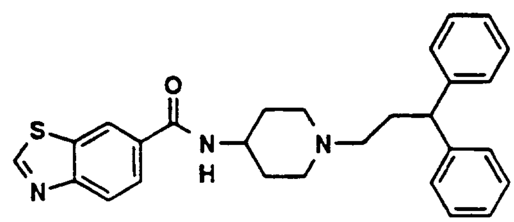

M.S. (electrospray, pos. ions) 456 (M + H).
236.

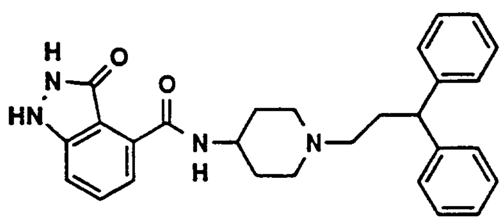

M.S. (electrospray, pos. ions) 455 (M + H).
237.

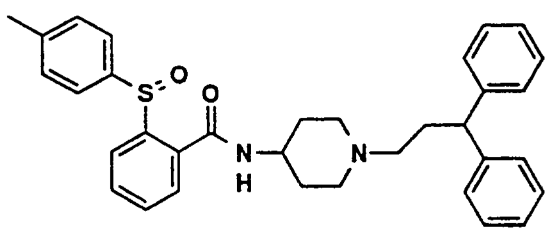

M.S. (electrospray, pos. ions) 537 (M + H).

238.

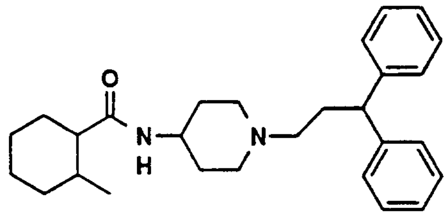

**mixture of cis and trans**

M.S. (electrospray, pos. ions) 419 (M + H).

239.

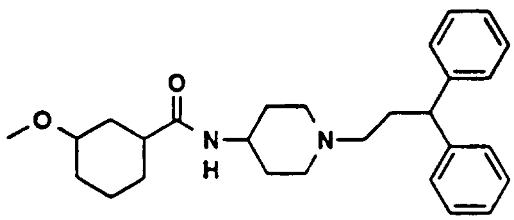

**mixture of cis and trans**

M.S. (electrospray, pos. ions) 435 (M + H).

240.

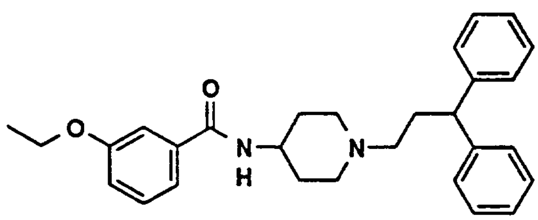

M.S. (electrospray, pos. ions) 443 (M + H).

241.

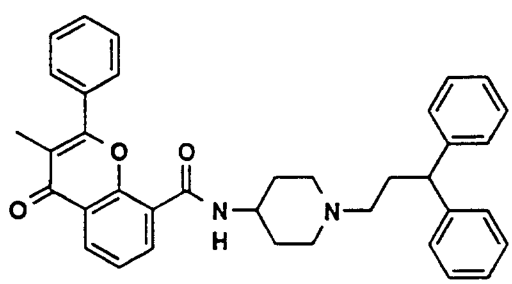

M.S. (electrospray, pos. ions) 557 (M + H).

242.

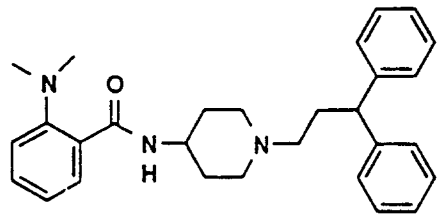

M.S. (electrospray, pos. ions) 442 (M + H).

243.

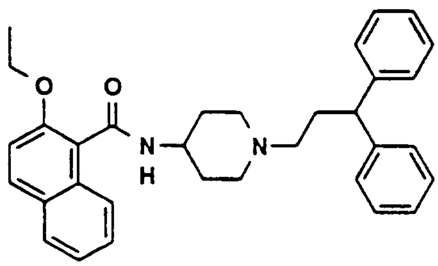

M.S. (electrospray, pos. ions) 493 (M + H).

244.

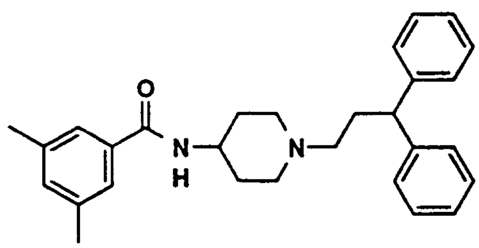

M.S. (electrospray, pos. ions) 427 (M + H).

245.

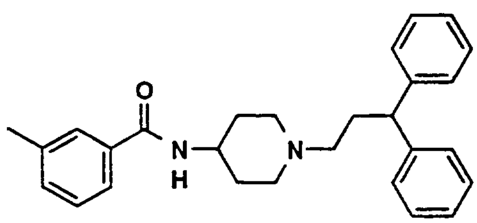

M.S. (electrospray, pos. ions) 413 (M + H).

246.

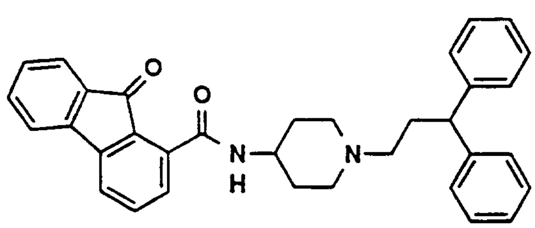

M.S. (electrospray, pos. ions) 501 (M + H).
247.

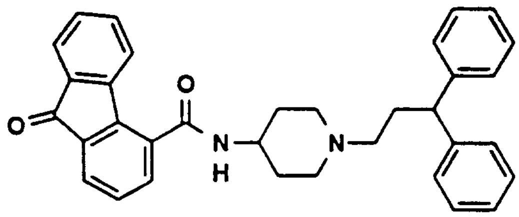

M.S. (electrospray, pos. ions) 501 (M + H).
248.

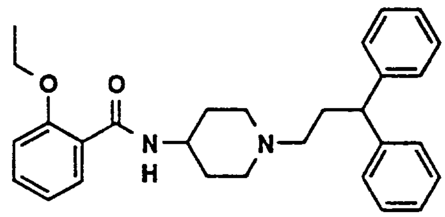

M.S. (electrospray, pos. ions) 443 (M + H).
249.

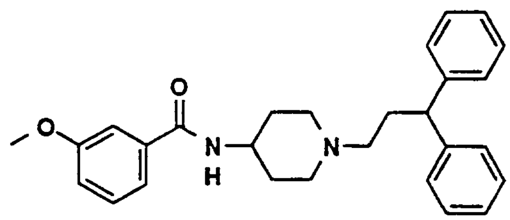

M.S. (electrospray, pos. ions) 429 (M + H).
250.

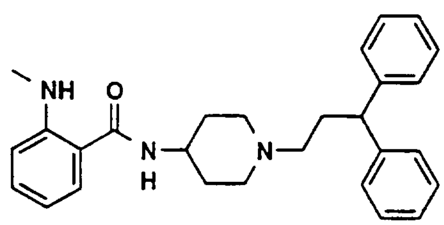

M.S. (electrospray, pos. ions) 428 (M + H).
251.

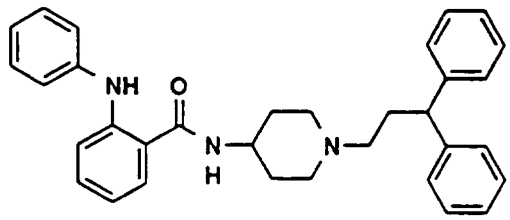

M.S. (electrospray, pos. ions) 490 (M + H).

252.

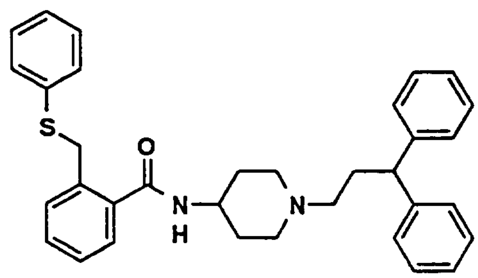

M.S. (electrospray, pos. ions) 521 (M + H).

253.

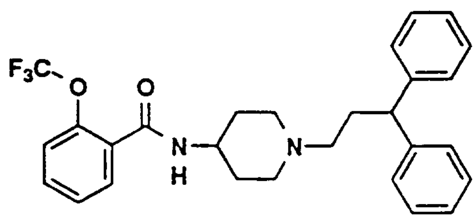

M.S. (electrospray, pos. ions) 483 (M + H).

254.

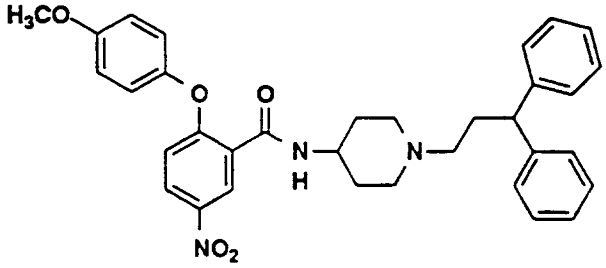

M.S. (electrospray, pos. ions) 566 (M + H).

255.

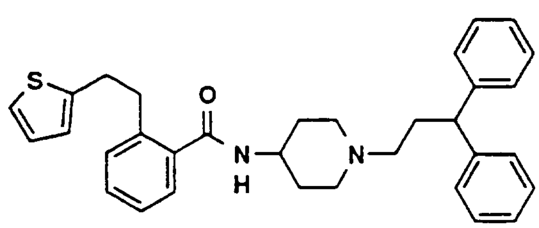

M.S. (electrospray, pos. ions) 509 (M + H).
256.

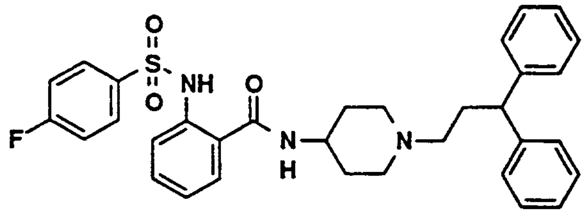

M.S. (electrospray, pos. ions) 572 (M + H).
257.

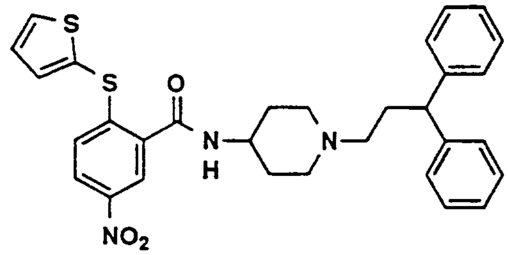

M.S. (electrospray, pos. ions) 558 (M + H).
258.

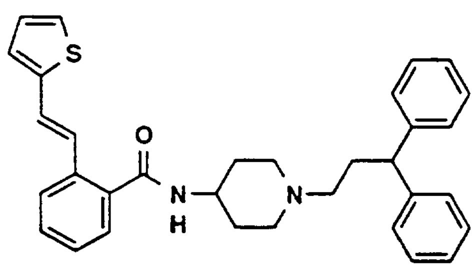

M.S. (electrospray, pos. ions) 507 (M + H).
259.

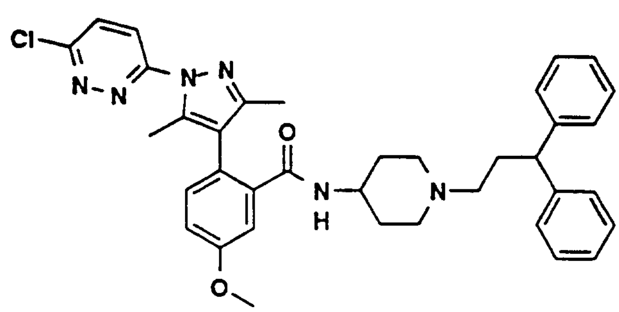

M.S. (electrospray, pos. ions) 635 (M + H).
260.

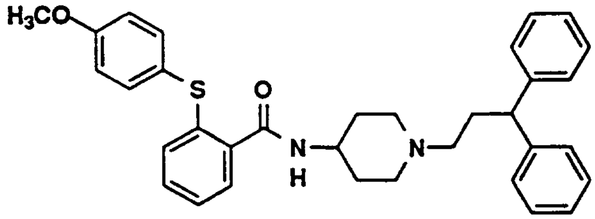

M.S. (electrospray, pos. ions) 537 (M + H).

261.

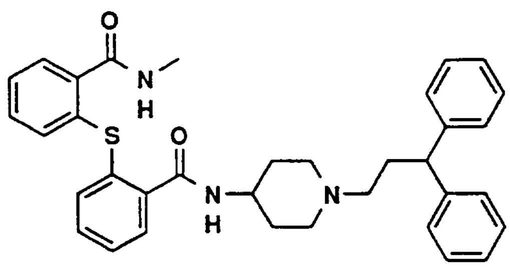

M.S. (electrospray, pos. ions) 564 (M + H).

262.

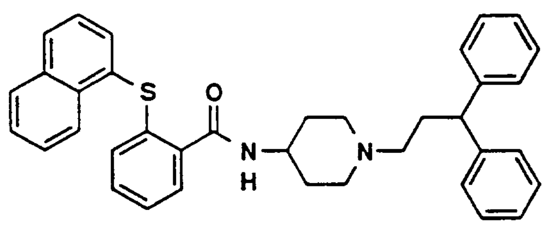

M.S. (electrospray, pos. ions) 557 (M + H).

263.

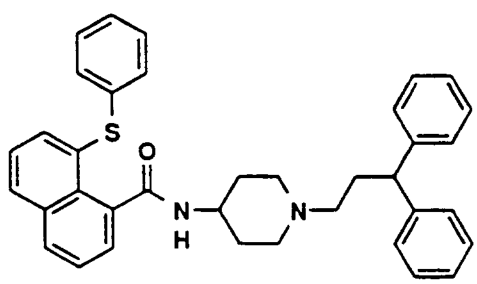

M.S. (electrospray, pos. ions) 557 (M + H).

264.

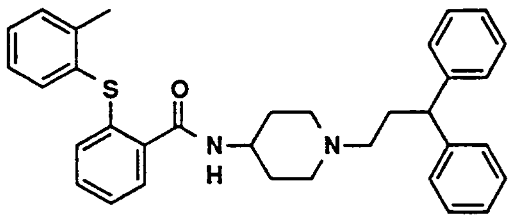

M.S. (electrospray, pos. ions) 521 (M + H).

265.

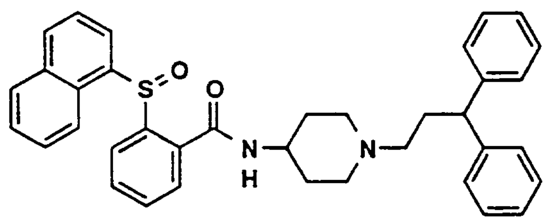

M.S. (electrospray, pos. ions) 573 (M + H).

266.

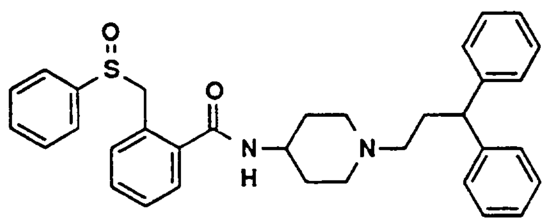

M.S. (electrospray, pos. ions) 537 (M + H).

267.

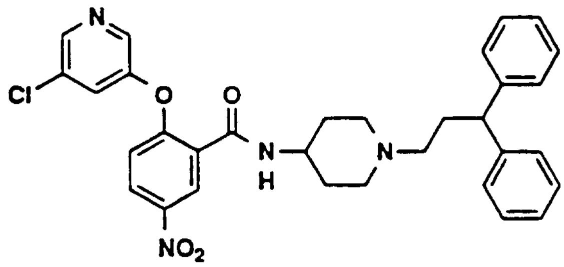

M.S. (electrospray, pos. ions) 571 (M + H).

268.

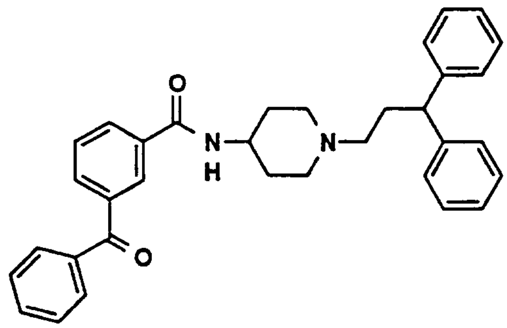

M.S. (electrospray, pos. ions) 503 (M + H).
269.

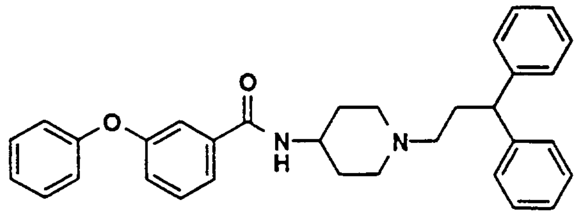

M.S. (electrospray, pos. ions) 491 (M + H).
270.

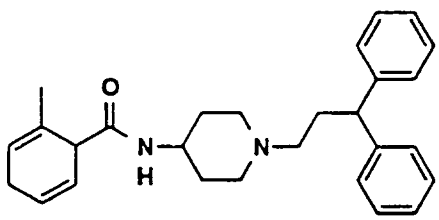

M.S. (electrospray, pos. ions) 415 (M + H).
271.

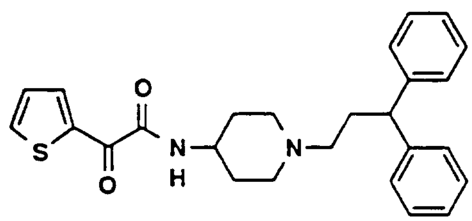

M.S. (electrospray, pos. ions) 433 (M + H).
272.

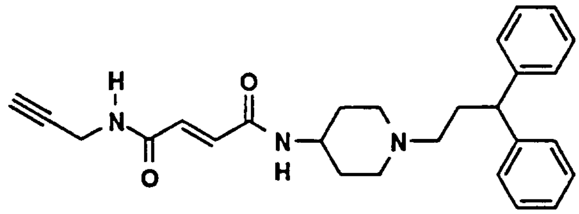

M.S. (electrospray, pos. ions) 430 (M + H).

273.

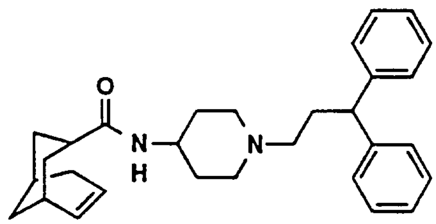

M.S. (electrospray, pos. ions) 443 (M + H).

274.

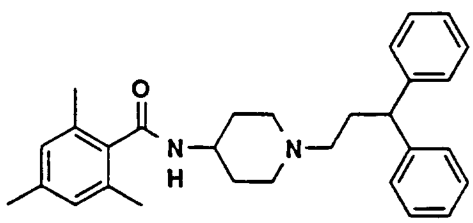

M.S. (Cl, pos. ions) 441 (M + H).

275.

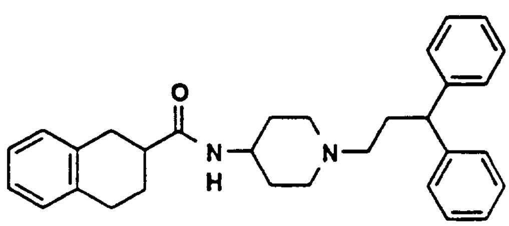

M.S. (electrospray, pos. ions) 453 (M + H).

276.

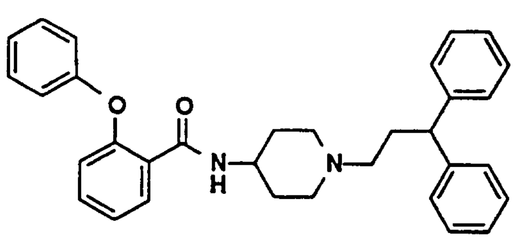

M.S. (electrospray, pos. ions) 491 (M + H).

Example 277

(Z)-2,3-Dihydro-2-[1-[3-(2-phenoxyphenyl)-2-propenyl]-4-piperidinyl]-1H-isoindol-1-one, monohydrochloride

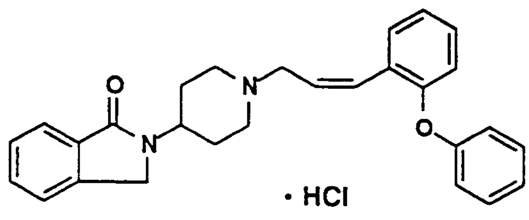

A.

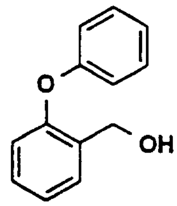

To a solution of 2-phenoxybenzoic acid (Aldrich) (2.5 g, 11.7 mmol) in THF (20 mL) at 0 °C was added dropwise a solution of borane-tetrahydrofuran complex in THF (1.0M, 17.5 mL, 17.5 mmol). The reaction was stirred at RT for 3 h. The reaction was quenched with water/THF (1:1, 2 mL) followed by potassium carbonate until solution was basic. Ethyl ether (200 mL) was added and the organic layer was washed with water (2 x 50 mL), saturated sodium bicarbonate solution (2 x 50 mL), brine (2 x 50 mL) and dried over $MgSO_4$. Evaporation gave title compound (2.3 g, 95%) as a crude oil.

B.

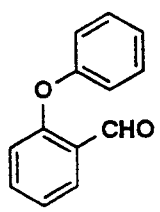

To a solution of oxalyl chloride (2.0M, 4.23 mL, 8.45 mL) in dichloromethane (20 mL) at -74 °C was added dropwise a solution of DMSO (1.2 mL, 16.9 mmol) in dichloromethane (1 mL). The reaction was stirred at -74 °C for 1h. A solution of Part A compound (1.3 g, 6.50 mmol) in dichloromethane (4 mL) was added dropwise. The reaction was stirred at -74 °C for 1.5 h. Triethylamine (5.4 mL, 39 mmol) was added and the reaction was warmed to RT over 1 h. Ethyl ether (100 mL) was added and the organic layer was washed with 1 N HCl solution (2 x 30 mL), water (2 x 30 mL), saturated sodium bicarbonate solution (2 x 30 mL), brine (2 x 30 mL) and dried over $MgSO_4$. Evaporation gave title compound (1.29 g, 100%) as a crude oil.

C.

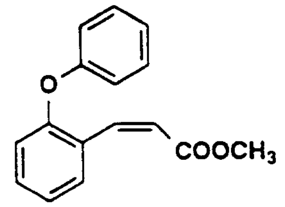

To a solution of bis(2,2,2-trifluoroethyl)(methoxycarbonylmethyl)phosphonate (3.04 g, 9.56 mmol), 18-crown-6 (2.53 g, 9.56 mmol) in THF (60 mL) at 0 °C was added a solution of potassium bis(trimethylsilyl)-amide in toluene (0.5M, 19.1 mL, 9.56 mmol). The reaction was stirred at 0 °C for 30 min then cooled to -78 °C. A solution of Part B compound (1.72 g, 8.79 mmol) in THF (2 mL) was added. The reaction was stirred at -78 °C for 1 h. The reaction was quenched with saturated ammonium chloride solution (5 mL). Ethyl ether (200 mL) was added to the reaction, and the organic layer was washed with water (2 x 50 mL), brine (2 x 50 mL) and dried over $MgSO_4$. Purification was performed by flash chromatography on silica gel (200 g), loaded and eluted with 5% ethyl acetate in hexane. Pure fractions were combined and evaporated to give title compound (1.38 g, 58%) as a colorless oil.

D.

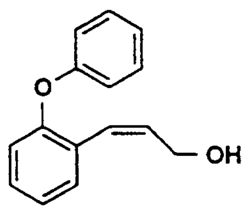

To a solution of Part C compound (1.38 g, 5.15 mmol) in THF (20 mL) at 0 °C was added dropwise a solution of diisobutylaluminum hydride in hexane (1.0M, 11.3 mL, 11.3 mmol). The ice bath was removed and the reaction was stirred at RT for 15 min. The reaction was quenched by methanol (2 mL) followed by potassium sodium tartrate solution (1M, 100 mL). The mixture was stirred at RT overnight. Ethyl ether (200 mL) was added and the organic layer was washed with water (2 x 50 mL), brine (2 x 50 mL) and dried over $MgSO_4$. Purification was performed by flash chromatography on silica gel (150 g), loaded and eluted with 20% ethyl acetate in hexane. Pure fractions were combined and evaporated to give title compound (1.24 g, 100%) as a colorless oil.

E.

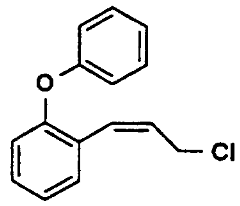

To a solution of N-chlorosuccinimide (802 mg, 6.01 mmol) in dichloromethane (15 mL) at -40 °C was added dropwise methyl sulfide (0.56 mL, 7.64 mmol). The reaction was stirred at -40 °C for 10 min then warmed to RT for 30 min. The reaction was recooled to -40 °C, and a solution of Part D compound(1.24 g, 5.46 mmol) in dichloromethane (2 mL) was added dropwise. The reaction was stirred at -40 °C for 2 h then warmed to RT for 30 min. Hexane (300 mL) was added to dilute the reaction and the organic layer was washed with water (2 x 50 mL), brine (2 x 50 mL) and dried over $MgSO_4$. Evaporation gave title compound (1.12 g, 84%) as a colorless oil.

F. (Z)-2,3-Dihydro-2-[1-[3-(2-phenoxyphenyl)-2-propenyl]-4-piperidinyl]-1H-isoindol-1-one, monohydrochloride

To a solution of Part E compound (600 mg, 2.45 mmol) in DMF (20 mL) was added Example 2 Part A compound (2-(4-piperidinyl)-2,3-dihydro-1H-isoindol-1-one) (530 mg, 2.45 mmol) followed by anhydrous potassium carbonate (372 mg, 2.69 mmol). The reaction was stirred at 55 °C overnight. The reaction was cooled to RT. Ethyl ether (200 mL) was added to dilute the reaction, and the organic layer was washed with water (2 x 50 mL), brine (2 x 50 mL) and dried over $MgSO_4$. Evaporation gave a crude oil. Purification was performed by flash chromatography on 100 g of silica gel, loaded and eluted with 2% methanol in dichloromethane. Pure fractions were combined and evaporated to give a colorless oil. The resulting oil was dissolved in methanol (2 mL) and HCl in ethyl ether solution (1.0 M, 3.0 mL, 3.0 mmol) was added. The HCl salt precipitated from the solution. The salt was filtered and dried at 60 °C under vacuum to give title compound (490 mg, 80%) as a white solid.
m.p. 196-198 °C.

| Anal. Calc. for $C_{28}H_{28}N_2O_2$ • HCl: | | | | |
|---|---|---|---|---|
| | C, 72.95; | H, 6.34; | N, 6.08; | Cl, 7.69 |
| Found: | C, 72.48; | H, 6.42; | N, 5.98; | Cl, 7.70. |

Example 278

2,3-Dihydro-2-[1-[4-(hydroxyphenylmethyl)phenyl]-4-piperidinyl]-1H-isoindol-1-one

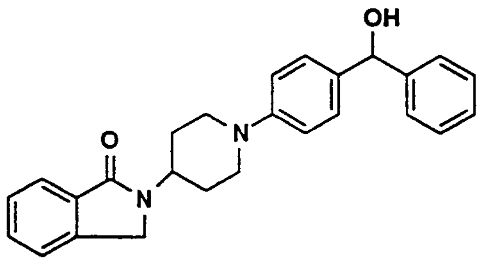

A.

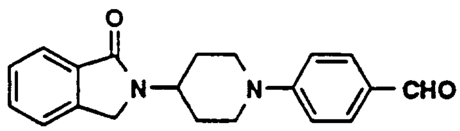

Potassium carbonate (10.6 g, 76.5 mmol) was added to a solution of Example 2 Part A compound (15.0 g, 69.6 mmol) and 4-fluorobenzaldehyde (Aldrich) (8.61 g, 69.6 mmol) in N,N-dimethylacetamide (100 mL) and the reaction was stirred at 125 °C for 24 h, then cooled to RT. The reaction was dissolved in $CH_2Cl_2$ - (500 mL) and washed with water (4 x 180 mL) and brine (2 x 200 mL), then dried over $MgSO_4$. Evaporation gave a solid mass. The crude product was triturated with EtOAc (10 mL), then washed with EtOAc (10 mL) and filtered to give title compound (17.8 g, 80%) as a white solid (mp 211-241 °C).

B. 2,3-Dihydro-2-[1-[4-(hydroxyphenylmethyl)phenyl]-4-piperidinyl]-1H-isoindol-1-one

To a solution of Part A compound (2.30 g, 7.19 mmol) in THF (30 mL) at 0 °C was added dropwise phenyl magnesium bromide solution (1.0M, 7.91 mL, 7.91 mmol). The reaction was stirred at RT for 4 h over which time the reaction became clear. Saturated ammonium chloride solution (5 mL) was added to quench the reaction. Ethyl ether (200 mL) was added and the organic layer was washed with water (2 x 50 mL), brine (2 x 50 mL) and dried over $MgSO_4$. Purification was perfomed by flash chromatography on silica gel (200 g), loaded and eluted with 10% acetone in dichloromethane. Pure fractions were combined and evaporated to give title compound (1.5 g, 52%) as a white solid.
m.p. 164-166 °C

| Anal. Calc. for $C_{26}H_{26}N_2O_2 \cdot 0.2H_2O$: | | | |
|---|---|---|---|
| | C, 77.66; | H, 6.62; | N, 6.97 |
| Found: | C, 77.77; | H, 6.44; | N, 6.94 |

Example 279

2,3-Dihydro-2-[1-[4-(phenylmethyl)phenyl]-4-piperidinyl]-1H-isoindol-1-one

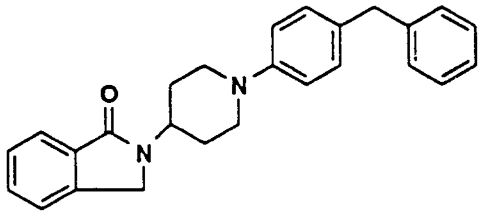

To a solution of Example 278 compound (300 mg, 0.75 mmol) and triethylsilane (0.24 mL, 1.50 mmol) in dichloromethane (5 mL) at 0 °C was added dropwise a solution of boron trifluoride etherate (0.18 mL, 1.50 mmol). The reaction was stirred at RT overnight. Saturated ammonium chloride solution (2 mL) was added to quench the reaction. Ethyl ether (100 mL) was added and the organic layer was washed with water (2 x 30 mL), brine (2 x 30 mL) and dried over $MgSO_4$. Purification was performed by flash chromatography on silica gel (50 g), loaded and eluted with 30% acetone in hexane. Pure fractions were combined and evaporated to give title compound (96 mg, 34%) as a white solid.
m.p. 151-155 °C

| Anal. Calc. for $C_{26}H_{26}N_2O$ • $0.5H_2O$: | | | |
|---|---|---|---|
| | C, 79.76; | H, 6.95; | N, 7.16 |
| Found: | C, 79.88; | H, 6.78; | N, 7.12. |

Example 280

2-[1-(4-Benzoylphenyl)-4-piperidinyl]-2,3-dihydro-1H-isoindol-1-one

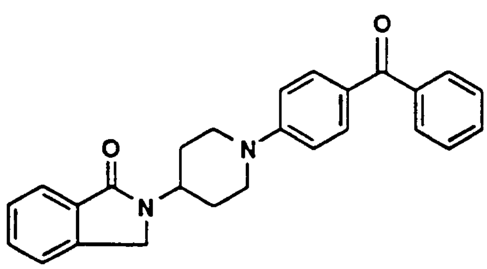

To a solution of 4-fluorobenzophenone (Aldrich) (3.0 g, 15.0 mmol) and Example 2, Part A compound (3.24 g, 15.0 mmol) in N,N-dimethylacetamide (30 mL) was added potassium carbonate (2.28 g, 16.5 mmol). The reaction was refluxed 15 h then cooled to RT. Ethyl ether (300 mL) was added and the solution was washed with water (2 x 70 mL), brine (2 x 70 mL) and dried over $MgSO_4$. Evaporation gave a crude solid. Purification was perfomed by flash chromatography on silica gel (300 g), loaded and eluted with 4% acetone in dichloromethane. Pure fractions were combined and evaporated to give title compound (3.5 g, 59%) as a yellowish solid.
m.p. 173-175 °C.

| Anal. Calc. for $C_{26}H_{24}N_2O_2$: | | | |
|---|---|---|---|
| | C, 78.76; | H, 6.10; | N, 7.07 |
| Found: | C, 78.34; | H, 6.12; | N, 7.00. |

Example 281

2-[1-(4-Diphenylmethyl)phenyl]-4-piperidinyl]-2,3-dihydro-1H-isoindol-1-one

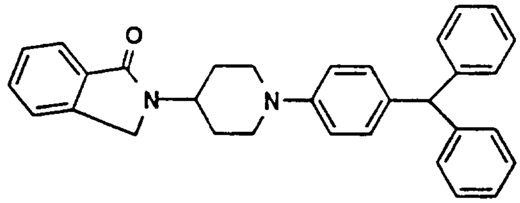

A.

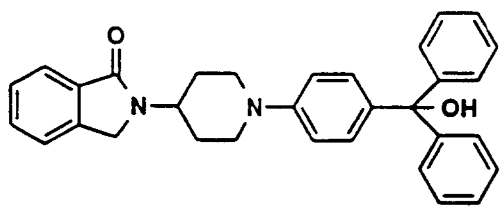

To a solution of Example 280 compound (2.0 g, 5.05 mmol) in THF (40 mL) at 0 °C was added dropwise a solution of phenylmagnesium bromide (1.0M, 5.55 mL, 5.55 mmol) in THF. The reaction was stirred at RT for 2 days. Saturated ammonium chloride solution (2 mL) was added to quench the reaction. Ethyl ether (200 mL) was added and the organic layer was washed with water (2 x 50 mL), brine (2 x 50 mL) and dried over $MgSO_4$. Purification was performed by flash chromatography on silica gel (200 g), loaded and eluted with 2% methanol in dichloromethane. Pure fractions were combined and evaporated to give title compound (400 mg, 17%) as a white solid (m.p. 163-166 °C).

B. 2-[1-(4-Diphenylmethyl)pheny]-4-piperidinyl]-2,3-dihydro-1H-isoindol-1-one

To a solution of Part A compound (150 mg, 0.32 mmol) in TFA (1 mL) at 0 °C was added triethylsilane (0.06 mL, 0.35 mmol). The reaction was stirred at RT for 30 min. Saturated sodium bicarbonate solution (20 mL) was added to quench the reaction. Ethyl ether (50 mL) was added and the organic layer was washed with water (2 x 20 mL), brine (2 x 20 mL) and dried over $MgSO_4$. Purification was perfomed by flash chromatography on silica gel (50 g), loaded and eluted with 10% acetone in dichloromethane. Pure fractions were combined and evaporated to give title compound (120 mg, 77%) as a white solid.
m.p. 162-165 °C.

| Anal. Calc. for $C_{32}H_{30}N_2O$: | | | |
|---|---|---|---|
| | C, 83.81; | H, 6.59; | N, 6.11 |
| Found: | C, 83.65; | H, 6.69; | N, 5.97. |

Example 282

(Z)-N-[1-(5,5-Diphenyl-2-pentenyl)-4-piperidinyl]-2-phenoxybenzamide

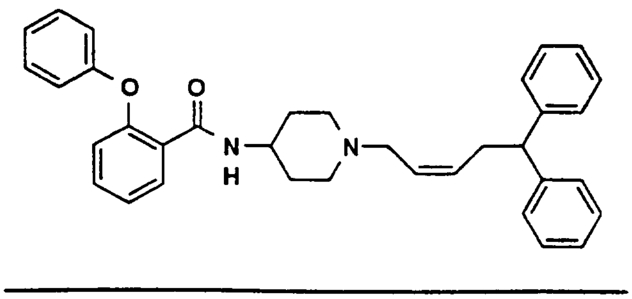

A.

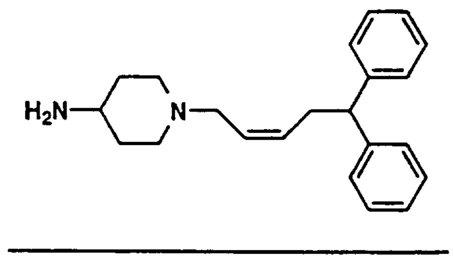

A(1). (Z)-[1-(5,5-Diphenyl-2-pentenyl)-4-piperidinyl]carbamic acid, 1,1-dimethylethyl ester

A stirred suspension of 1.16 g (5.78 mmol) of 4-piperidinylcarbamic acid, 1,1-dimethylethyl ester (Example I Part B), 1.35 g (5.26 mmol) of (Z)-1-chloro-5,5-diphenyl-2-pentene, and 799 mg (5.78 mmol) of potassium carbonate in 15 mL of N,N-dimethylformamide was heated to 60 °C for eighteen hours, cooled. filtered and concentrated to a dark oil which was chromatographed on silica gel (150 g) eluted with 95:5 methylene chloride/methanol to provide 1.82 g (82%) of title compound, as an off-white solid.
m.p. 105-108 °C.

| Analysis Calcd. for $C_{27}H_{36}N_2O_2$ • 0.35 $H_2O$: | | | |
|---|---|---|---|
| | C, 75.97; | H, 8.66; | N, 6.56 |
| Found | C, 75.81; | H, 8.79; | N, 6.72. |

A(2).

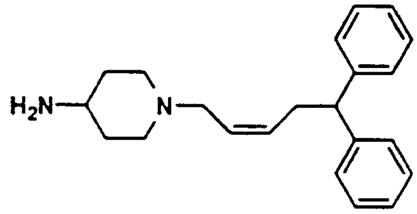

Part A(1) compound 1.44 g (34.31 mmol) was treated with 8.5 mL (34.31 mmol) of 4 M HCl in dioxane. The crude product was twice evaporated from methylene chloride and dried under high vacuum to provide 1.42 mg (100%) of di-HCl salt of the title A compound as an off-white solid.

The free amine was obtained by suspending/dissolving the di-HCl salt in $CH_2Cl_2$ and washing with 1 N KOH. The $CH_2Cl_2$ solution was filtered through cotton and concentrated and the diamine was used without characterization.

B. (Z)-N-[1-(5,5-Diphenyl-2-pentenyl)-4-piperidinyl]-2-phenoxybenzamide

To a solution of 197 mg (0.9 mmol, 1.5 eq) of orthophenoxybenzoic acid (Aldrich) and 124 mg (0.9 mmol, 1.5 eq) of 1-hydroxybenzotriazole (HOBt) in 10 mL of $CH_2Cl_2$ and 0.5 mL of DMF was added 144 μL (0.9 mmol, 1.5 eq) of diisopropylcarbodiimide (DIC) followed by a solution of 196 mg (0.6 mmol, 1 eq) of Part A compound in 2.5 mL of $CH_2Cl_2$. After 17 h, the reaction was diluted with $CH_2Cl_2$ and washed with 1 N KOH. The organic solution was filtered through cotton and concentrated to afford a viscous heterogeneous mixture which was chromatographed on silica gel (75 g) eluted with 4% methanol in $CH_2Cl_2$. The material obtained from this column was contaminated with a significant amount of urea by-product. The mixture was dissolved in EtOAc from which most of the urea precipitated. After filtration of the precipitated urea and concentration of the EtOAc solution, the resulting oil was chromatographed on silica gel (75 g) eluted with EtOAc to afford 192 mg of title compound as a pale yellow waxy solid which was recrystallized from ether/hexanes to provide 138 mg (44%) of title compound as a white crystalline solid; m.p. 91-94 °C.

| Anal. Calcd. for $C_{35}H_{36}N_2 \bullet 0.4\ H_2O$: | | | |
|---|---|---|---|
| | C, 80.24; | H, 7.08; | N, 5.35 |
| Found: | C, 79.85; | H, 6.87; | N, 5.13. |

Example 283

(Z)-N-[1-(5,5-Diphenyl-2-pentenyl)-4-piperidinyl]-3-phenoxybenzamide

To a solution of 197 mg (0.9 mmol, 1.5 eq) of *meta*phenoxybenzoic acid (Aldrich) and 124 mg (0.9 mmol, 1.5 eq) of 1-hydroxybenzotriazole (HOBt) in 10 mL of $CH_2Cl_2$ and 0.5 mL of DMF was added 144 μL (0.9 mmol, 1.5 eq) of diisopropylcarbodiimide (DIC) followed by a solution of 196 mg (0.6 mmol, 1 eq) of Example 282 Part A compound in 2.5 mL of $CH_2Cl_2$. After 17 h, the reaction was diluted with $CH_2Cl_2$ and washed with 1 N KOH. The organic solution was filtered through cotton and concentrated to afford a viscous heterogeneous mixture which was taken up in EtOAc. The resulting precipitated urea was removed by filtration and the filtrate was concentrated to afford an oil which was chromatographed on silica gel (70 g) eluted with 4% methanol in $CH_2Cl_2$. The product containing fractions still contaminated with urea by-product were concentrated and the resulting oil was rechromatographed on silica gel (50 g) eluted with EtOAc to afford 226 mg of a pale yellow oil. The oil was dissolved in ether and treated with excess HCl in ether. The resulting HCl salt was isolated by filtration and solvent remnants were removed by heating in a vacuum oven at 80 °C under full vacuum for 13 h to afford 231 mg (70%) of title compound as an off-white solid; m.p. 181-184 °C.

| Anal. Calcd. for $C_{35}H_{37}N_2O_2Cl$: | | | | |
|---|---|---|---|---|
| | C, 76.00; | H, 6.74; | N, 5.06; | Cl, 6.41 |
| Found: | C, 75.68; | H, 6.81; | N, 5.03; | Cl, 6.11. |

Example 284

2,3-Dihydro-2-[1-(3-oxo-3-phenylpropyl)-4-piperidinyl]-1H-isoindol-1-one (intermediate)

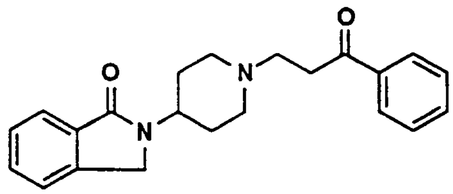

A.

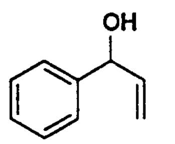

To a solution of benzaldehyde (Aldrich) (4.0 g, 37.7 mmol) in THF (100 mL) at 0 °C was added dropwise a solution of vinylmagnesium bromide (1.0M, 41.5 mL, 41.5 mmol) in THF (100 mL). The reaction was stirred at 0 °C for 1 h then warmed to RT for 2 h. Saturated ammonium chloride solution (10 mL) was added to quench the reaction. Ethyl ether (200 mL) was added and the organic layer was washed with water (2 x 50 mL), brine (2 x 50 mL) and dried over $MgSO_4$. Purification was performed by flash chromatography on silica gel (200 g), loaded and eluted with 10% ethyl acetate in hexane. Pure fractions were combined and evaporated to give title compound (1.3g, 23%) as a colorless oil.

B.

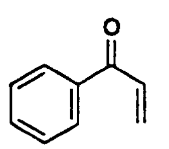

To a solution of oxalyl chloride (5.2 mL, 10.4 mmol) in dichloromethane (40 mL) at -60 °C was added dropwise DMSO (1.5 mL, 20.8 mmol). The reaction was stirred at -60 °C for 1 h. A solution of Part A compound (1.2 g, 8.0 mmol) in dichloromethane (3 mL) was added dropwise. Stirring was continued at -60 °C for 1 h. Triethylamine (6.7 mL, 48 mmol) was added and the reaction was warmed to RT over 1 h. Ethyl ether (200 mL) was added and the organic layer was washed with water (2 x 50 mL), brine (2 x 50 mL) and dried over $MgSO_4$. Evaporation gave title compound(1.1 g, 100%) as a crude oil.

C. 2,3-Dihydro-2-[1-(3-oxo-3-phenylpropyl)-4-piperidinyl]-1H-isoindol-1-one

To a solution of Example 2 Part A compound (1.93 g, 8.92 mmol) in dry ethanol (15 mL) at RT was added a solution of Part B compound (1.1 g, 7.43 mmol) in dry ethanol (2 mL). The reaction was stirred at RT for 4 h. The reaction was evaporated to dryness. Ethyl ether (150 mL) was added, and the solution was washed water (2 x 30 mL), brine (2 x 30 mL) and dried over $MgSO_4$. Purification was perfomed by flash chromatography on silica gel (150 g), loaded and eluted with 4% methanol in dichloromethane. Pure fractions were combined and evaporated to give title compound (1.0 g, 37%) as a yellowish solid.
m.p. 87-90 °C.

| Anal. Calc. for $C_{22}H_{24}N_2O_2$: | | | |
|---|---|---|---|
| | C, 75.83; | H, 6.94; | N, 8.04 |
| Found: | C, 75.40; | H, 6.97; | N, 7.96. |

Example 285

2,3-Dihydro-2-[1-[3-phenyl-3-(4-propylphenyl)propyl]-4-piperidinyl]-1H-isoindol-1-one, monohydrochloride

A.

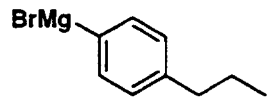

To a suspension of magnesium turnings (1.0 g, 41.2 mmol) in THF (8.2 mL) was added a solution of 1-bromo-4-propylbenzene (Aldrich) (1.64 g, 8.20 mmol) in THF (8.2 mL). The reaction was refluxed for 1.5 h, then cooled to RT. The Grignard solution was cannulated then titrated against 1.0M isopropanol in toluene using 2,2-biquinoline as an indicator to give title compound (0.35M, 70%) as a black solution.

B.

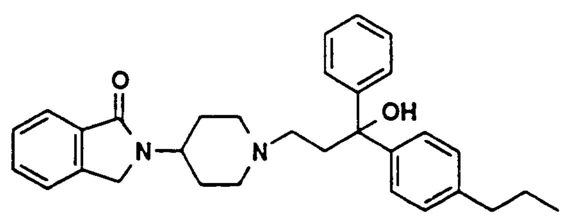

To a solution of Example 284 compound (500 mg, 1.37 mmol) in THF (8 mL) was added dropwise at 0 °C a solution of Part A compound (4.3 mL, 1.51 mmol). The reaction was stirred at 0 °C for 3 h then warmed to RT for 3 h. The reaction was quenched with saturated ammonium chloride solution (3 mL). Ethyl ether (200 mL) was added and the organic was washed with water (2 x 50 mL), brine (2 x 50 mL) and dried over $MgSO_4$. Evaporation gave title compound (400 mg, 63%) as a crude oil.

C. 2,3-Dihydro-2-[1-[3-phenyl-3-(4-propylphenyl)propyl]-4-piperidinyl]-1H-isoindol-1-one, monohydrochloride

Triethylsilane (0.15 mL, 0.94 mmol) was added dropwise at 0 °C to a solution of Part B compound (360 mg, 0.78 mmol) in trifluoroacetic acid (4 mL). After addition, the reaction was stirred for 1 h then evaporated

to dryness. The resulting residue was pumped under high vacuum for 2 h. Purification was perfomed by flash chromatography on silica gel (100 g), loaded and eluted with 2.5% methanol in dichloromethane. Pure fractions were combined and evaporated to give a colorless oil consisting of a mixture of desired deoxygenated product and olefin elimination products.

The resulting residue was dissolved in EtOAc (3 mL) and palladium (10%) on active carbon (40 mg) was added under nitrogen. The slurry was purge with nitrogen twice. A hydrogen balloon was connected to the reaction. Hydrogenation was maintained overnight. The reaction was filtered through Celite and the filtrate was evaporated to give a crude oil. Purification was performed by flash chromatography on silica gel (50 g), loaded and eluted with 5% methanol in dichloromethane. Pure fractions were combined and evaporated to give a colorless oil. The resulting oil was dissolved in methanol (1 mL), and HCl in ethyl ether solution(1 M, 1 mL, 1 mmol) was added at RT. The mixture was evaporated to give title compound (85 mg, 22%) as a white solid.
m.p. 125-130 °C.

| Anal. Calc. for $C_{31}H_{37}ClN_2O \cdot 1.4H_2O$: | | | |
|---|---|---|---|
| | C, 72.39; | H, 7.80; | N, 5.45 |
| Found: | C, 72.11; | H, 7.26; | N, 5.22. |

Example 286

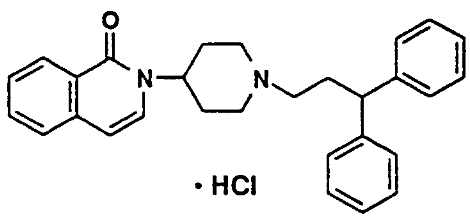

A. N-[1-(3,3-Diphenylpropyl)-4-piperidinyl]-2-methylbenzamide

To a stirred solution of 485 mg (1.46 mmol) of Example 1 Part E compound in 8 mL of methylene chloride at 0 °C under argon was added 336 µL (4.38 mmol) of pyridine and 200 µL (1.54 mmol) of o-toluoyl chloride. After warming to room temperature, the mixture was stirred for one hour and diluted with methylene chloride and water. The organics were separated, and the aqueous layer was basified with 1 M KOH and extracted with methylene chloride. The combined organics were dried (sodium sulfate) and concentrated to provide a yellow oil which was dried under high vacuum. The crude product was purified by flash chromatography on silica gel (80 g) eluted with 98:2 ethyl acetate/methanol. Pure product fractions were combined and concentrated to provide 345 mg (67%) of title compound as a yellow solid.

B.

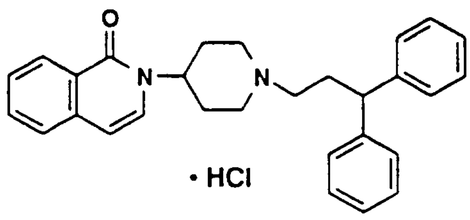

A solution of Part A compound (865mg, 2.1 mmol) in 8.6 ml of dry tetrahydrofuran was cooled to -22°C under an argon atmosphere and 2 equiv. of 1.6 M n-butyl lithium (2.63 ml) in hexanes was added keeping the temperature below -20°C. The reaction was stirred at -20°C for 30 minutes and then dimethylformamide (0.186 ml, 2.4 mmol) was added keeping the temperature below -15°C. When the addition of the DMF was completed the reacton was stirred at -20°C for 30 minutes and then quenched with 6 N HCl (1.66 ml). After stirring for 30 minutes the reaction was made basic by adding 1 N NaOH and extracted with ether (3 x 15 ml). The ether extracts were combined, washed with water (30ml), brine (30ml) and dried over sodium sulfate. The solvents were evaporated and the crude product was purified on a Merck silica column eluting with 5 to 10% MeOH / EtOAc yielding 293 mg (36%) of the "free base" as a colorless solid. The purified product was dissolved in 8 ml of ether and 0.5 ml of a 1 N HCl solution in ether was added. The colorless precipitated HCl salt was collected, washed with additional ether and dried under vacuum at 80°C yielding 320mg (36%) of title compound as a colorless solid, m.p. 188-194°C (dec).

| Analysis Calcd for $C_{29}H_{31}N_2ClO_2$ • 0.25 $H_2O$: | | | | |
|---|---|---|---|---|
| Calc'd: | C, 75.15; | H, 6.85; | N, 6.04; | Cl, 7.65 |
| Found: | C, 74.98; | H, 7.07; | N, 6.21; | Cl, 7.91. |

Example 287

2-[1-(3,3-Diphenylpropyl)-4-piperidinyl]-3,4-dihydro-1(2H)-isoquinolinone, monohydrochloride

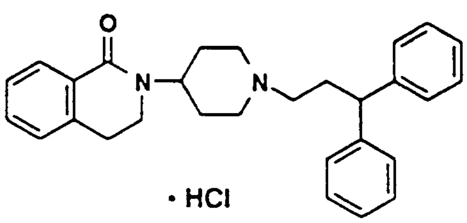

A quantity of Example 286 compound (95 mg, 0.225 mmol) was dissolved in 3 ml of absolute ethanol containing 0.5 ml of 0.1 N HCl. 50 mg of 10% Pd / C was suspended in the reaction and the mixture was stirred under a hydrogen atmosphere (balloon). After stirring for 6 days starting material still remained. The reaction was filtered, fresh catalyst (50 mg) was added and the mixture stirred under 50 psi of hydrogen for 24 hrs. Tlc, silica 5% $MeOH/CH_2Cl_2$ showed some starting material present. The reaction was filtered, and the solvents evaporated yielding the crude product as a colorless oil. Purification by flash chromatography on silica eluting with 1 to 3% $MeOH/CH_2Cl_2$ yielded 22 mg of pure free base as a colorless oil. The oily free base was dissolved in ether and 0.5 ml of 1 N HCl in ether was added forming the product as a white precipitate which was collected, washed with ether and dried under vacuum yielding 22 mg (24%) of title compound as a colorless solid, m.p. 180-184°C.

| Anal. Calc'd for $C_{29}H_{33}N_2ClO$ + 0.75 $H_2O$: | | | | |
|---|---|---|---|---|
| | C, 73.40; | H, 7.32; | N, 5.43; | Cl, 7.47 |
| Found: | C, 73.69; | H, 7.67; | N, 5.13; | Cl, 7.35. |

Example 288

2-[1-[2-(9H-Fluoren-9-yl)ethyl]-4-piperidinyl]-2,3-dihydro-1H-isoindol-1-one

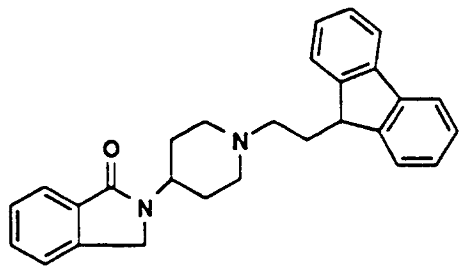

A.

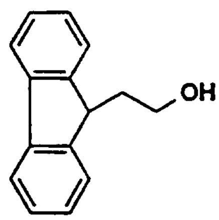

To a solution of 9-fluoreneacetic acid (Aldrich) (2.0 g, 8.92 mmol) in THF (20 mL) at 0 °C was added a solution of borane-tetrahydrofuran complex (1.0M in THF)(13.4 mL, 13.4 mmol). The reaction was stirred at 0 °C for 4 h. Saturated ammonium chloride solution (5 mL) was added to quench the reaction. Ethyl ether (200 mL) was added and the organic layer was washed with water (2 x 50 mL), saturated sodium bicarbonate solution (2 x 50 mL), brine (2 x 50 mL) and dried over $MgSO_4$. Evaporation gave a crude oil. Purification was performed by flash chromatography on silica gel (200 g), loaded and eluted with 20% ethyl acetate in hexane. Pure fractions were combined and evaporated to give title compound (1.82 g, 90%) as a colorless oil.

B.

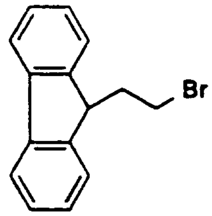

To a solution of Part A alcohol (800 mg, 3.81 mmol) and triphenylphosphine (1.1 g, 4.19 mmol) in dichloromethane (10 mL) at 0 °C was added N-bromosuccinimide (746 mg, 4.19 mmol). The reaction was stirred at 0 °C for 4 h. Hexane (150 mL) was added to dilute the reaction and the organic layer was washed with 10% sodium bisulfite solution (2 x 30 mL), brine (2 x 50 mL) and dried over $MgSO_4$. Purification was performed by flash chromatography on silica gel (100 g), loaded and eluted with 10% dichloromethane in hexane. Pure fractions were combined and evaporated to give title compound (1.02 g, 98%) as a colorless oil.

C.

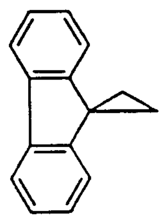

To a solution of Part B compound (680 mg, 2.50 mmol) and Example 2 Part A compound (540 mg, 2.50 mmol) in DMF (10 mL) was added potassium carbonate (380 mg, 2.75 mmol) at RT. The reaction was stirred at 70 °C overnight. Saturated ammonium chloride solution (5 mL) was added to quench the reaction. Ethyl ether (150 mL) was added and the organic layer was washed with water (2 x 30 mL), brine (2 x 30 mL) and dried over $MgSO_4$. Purification was performed by flash chromatography on silica gel (100 g), loaded eluted with 10% ethyl acetate in hexane. Pure fractions were combined and evaporated to give title compound (450 mg, 94%) as a white solid (m.p. 65-68 °C).

D. 2-[1-[2-(9H-Fluoren-9-yl)ethyl]-4-piperidinyl]-2,3-dihydro-1H-isoindol-1-one

A mixture of Part C compound (650 mg, 3.29 mmol) and Example 2 Part A compound (750 mg, 3.47 mmol) was heated until the mixture melted (155 °C). The reaction was maintained at 155 °C for 4.5 h then cooled to RT. Purification was performed by flash chromatography on silica gel (100 g), loaded and eluted with 1% methanol in dichloromethane. Pure fractions were combined and evaporated to give title compound (220 mg, 16%) as a white solid.
m.p. 159-162 °C.

| Anal. Calc. for $C_{28}H_{28}N_2O \cdot 0.6\ H_2O$: | | | |
|---|---|---|---|
| | C, 80.20; | H, 7.02; | N, 6.68 |
| Found: | C, 80.35; | H, 6.83; | N, 6.57. |

Example 289

2-[1-(3,3-Diphenylpropyl)-4-piperidinyl]-2,3-dihydro-3-(phenylmethyl)-1H-isoindol-1-one, monohydrochloride

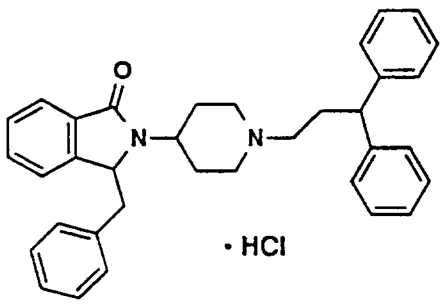

A. 2-[1-(3,3-Diphenylpropyl)-4-piperidinyl]-2,3-dihydro-1H-isoindol-1-one

To a solution of compound A from Example 2 (2.0 g, 9.26 mmol) and compound C from Example 1 (3.40 g, 9.26 mmol) in isopropanol (25 mL) was added potassium carbonate (2.05 g, 14.8 mmol). The reaction was refluxed overnight. The reaction was cooled to room temperature and filtered, and the filtrate was evaporated to dryness. Purification was performed by flash chromatography, loaded and eluted with 2.5% methanol in dichloromethane. Pure fractions were combined and evaporated to give compound A (2.82 g, 74 %) as a colorless oil.

B. 2-[1-(3,3-Diphenylpropyl)-4-piperidinyl]-2,3-dihydro-3-(phenylmethyl)-1H-isoindol-1-one, monohydrochloride

Sodium bis(trimethylsilyl)amide (775 µL, 1.0M in THF, 0.775 mmol) was added dropwise to a solution of Part A compound (265 mg, 0.646 mmol) in THF (4 mL) at -78 °C under argon. Upon addition the reaction color went from yellow to orange. The reaction was stirred at -78 °C for 20 min, then benzyl bromide (92 µL, 0.775 mmol) was added dropwise. The dark yellow reaction was stirred at -78 °C for 30 min, then quenched with saturated $NH_4Cl$ (1 mL). Water (1 mL) was added and the reaction mixture was extracted with $Et_2O$ (2 x 10 mL). The combined organic layers were washed with water (4 mL) and brine (4 mL), then dried over $MgSO_4$. Evaporation gave a yellow oil, which was purified by flash chromatography on silica (50 g) eluting with 50:50 EtOAc/hexane to give 205 mg of a yellow oil.

The free amine prepared above was dissolved in $Et_2O$ (3 mL) and treated with 1N HCl in $Et_2O$ (1 mL, 1 mmol). The resultant white precipitate was filtered, washed with $Et_2O$ (3 x 3 mL), and dried under high vacuum (0.4 torr) at 65 °C for 48 h to give title compound (169 mg, 49%) as a white solid.
mp 130-131 °C.

| Anal. Calcd. for $C_{35}H_{36}N_2O$ • HCl: | | | | |
|---|---|---|---|---|
| | C, 78.26; | H, 6.94; | N, 5.22; | Cl, 6.60. |
| Found: | C, 78.05; | H, 6.92; | N, 5.18; | Cl, 6.77. |

Example 290

2-[1-(3,3-Diphenylpropyl)-4-piperidinyl]-2,3-dihydro-3-methyl-1H-isoindol-1-one, monohydrochloride

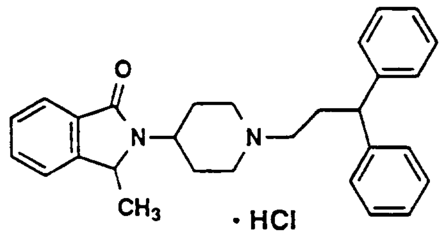

Utilizing the procedure for preparation of the Example 289 compound, the Example 289 Part A compound (276 mg, 0.673 mmol) was reacted with methyl iodide (50 µL, 0.808 mmol) instead of benzyl bromide then subjected to chromatography in 3:97 iPrOH/hexane to provide title compound (91 mg, 29%) as a white solid.
mp 129-131 °C.

| Anal. Calcd. for $C_{29}H_{32}N_2O$ • HCl: | | | | |
|---|---|---|---|---|
| | C, 75.55; | H, 7.21; | N, 6.08; | Cl, 7.69 |
| Found: | C, 75.10; | H, 7.22; | N, 6.06; | Cl, 7.49. |

Example 291

2,3-Dihydro-2-[1-[2-[9-(2-propylenyl)-9H-fluoren-9-yl]ethyl]-4-piperidinyl]-1H-isoindol-1-one

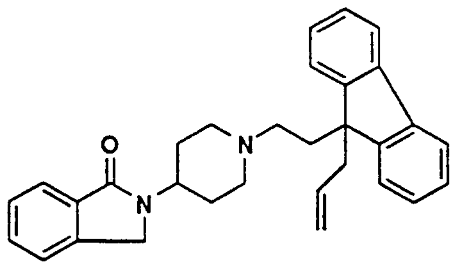

A.

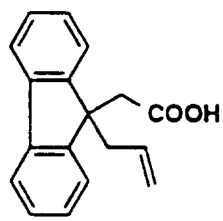

To a solution of 9-fluoreneacetic acid (Aldrich) (500 mg, 2.23 mmol) in THF (8 mL) at -78 °C was added dropwise a solution of butyllithium in hexane (2.5M, 1.78 mL, 4.46 mmol) followed by a solution of allylbromide (0.21 mL, 2.45 mL) in THF (1 mL). The reaction was stirred at -78 °C for 1 h. The reaction was quenched with saturatd ammonium chloride solution (2 mL) and warmed to RT. Ethyl ether (200 mL) was added and the organic layer was washed with water (2 x 50 mL), brine (2 x 50 mL) and dried over $MgSO_4$. Purification was performed by flash chromatography on silica gel (100 g), loaded and eluted with 15% acetone in hexane (4L), then 25% acetone in hexane (4L). Pure fractions were combined and evaporated to give title compound (420 mg, 71%) as a white solid (m.p. 121-124 °C).

B.

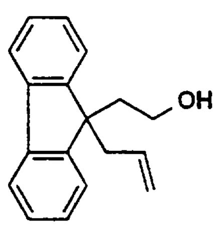

To a solution of Part A compound (420 mg, 1.59 mmol) in THF (10 mL) at 0 °C was added dropwise a solution of lithium aluminum hydride (1.0M in THF)(1.59 mL, 1.59 mmol). The reaction was stirred at 0 °C for 4 h. Methanol (2 mL) was added to quench the reaction. Potassium sodium tartrate solution (1 M, 100 mL) was added and the mixture was stirred at RT for 4 h. Ethyl ether (200 mL) was added and the organic layer was washed with water (2 x 50 mL), saturated sodium bicarbonate solution (2 x 50 mL), brine (2 x 50 mL) and dried over $MgSO_4$. Evaporation gave title compound (350 mg, 88%) as a white solid (m.p. 89-92

°C).

C.

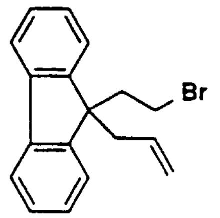

To a solution of Part B compound (350 mg, 1.40 mmol) and triphenylphosphine (403 mg, 1.54 mmol) in dichloromethane (10 mL) at 0 °C was added N-bromosuccinimide (274 mg, 1.54 mmol). The reaction was stirred at 0 °C for 1 h then warmed to RT for 2 h. Hexane (150 mL) was added to dilute the reaction and the organic layer was washed with 10% sodium bisulfite solution (2 x 30 mL), brine (2 x 50 mL) and dried over $MgSO_4$. Purification was performed by flash chromatography on silica gel (100 g), loaded and eluted with 10% dichloromethane in hexane. Pure fractions were combined and evaporated to give title compound (270 mg, 65%) as a colorless oil.

D. 2,3-Dihydro-2-[1-[2-[9-(2-propylenyl)-9H-fluoren-9-yl]ethyl]-4-piperidinyl]-1H-isoindol-1-one

To a solution of Part C compound (270 mg, 0.86 mmol) and Example 2 Part A compound (224 mg, 1.04 mmol) in DMF (5 mL) was added potassium carbonate (144 mg, 1.04 mmol) at RT. The reaction was stirred at 60 °C overnight. Saturated ammonium chloride solution (2 mL) was added to quench the reaction. Ethyl ether (200 mL) was added and the organic layer was washed with water (2 x 50 mL), brine (2 x 50 mL) and dried over $MgSO_4$. Purification was performed by flash chromatography on silica gel (30 g), loaded eluted with 2% methanol in dichloromethane. Pure fractions were combined and evaporated to give title compound (250 mg, 65%) as a white solid.
m.p. 117-120 °C.

| Anal. Calc. for $C_{31}H_{32}N_2O \cdot 0.5\ H_2O$: | | | |
|---|---|---|---|
| | C, 81.37; | H, 7.27; | N, 6.12 |
| Found: | C, 81.25; | H, 7.31; | N, 6.02. |

Example 292

2-[1-(3,3-Diphenylpropyl)-4-piperidinyl]-2,3-dihydro-3-phenyl-1H-isoindol-1-one

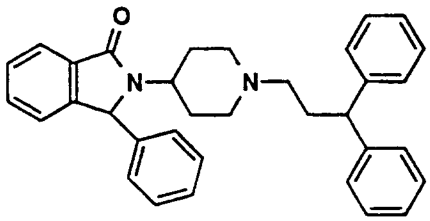

A. 2-[1-(3,3-Diphenylpropyl)-4-piperidinyl]-2,3-dihydro-3-hydroxy-3-phenyl-1H-isoindol-1-one

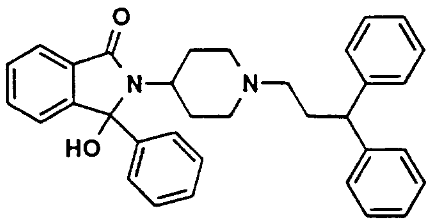

To a stirred solution of 1.06 g (2.88 mmol) of Example 1, Part E compound, 977 mg (4.30 mmol) of 2-benzoylbenzoic acid, 1.8 mL (12.9 mmol) of triethylamine in 1.8 mL (12.9 mmol) of methylene chloride at 0°C under argon was added 1.01 mg (4.30 mmol) of bis(2-oxo-3-oxazolidinyl)phosphinic chloride. After the reaction was allowed to come to room temperature, it was stirred for 16 hours then quenched with water and 4M HCl, and diluted with methylene chloride. The aqueous layer was basified and extracted with methylene chloride. The combined organic layers were dried (sodium sulfate) and concentrated. The crude product was purified by flash chromatography on silica gel eluted with 97:3 methylene chloride/methanol to provide 1.15 mg (88%) of a white solid, which was combined with 261 mg from another reaction and further purified by recrystallization from ethyl acetate/hexanes to provide 700 mg (combined yield for both reactions 47%) of title compound, as a yellow solid.
m.p. 150-153°C.

| Anal. Calc. for $C_{33}H_{34}N_2O_2$ • 0.1 $C_2H_5O_2CCH_3$: | | | |
|---|---|---|---|
| | C, 80.78; | H, 6.86; | N, 5.48 |
| Found: | C, 80,80; | H, 6.87; | N, 5.53. |

B. 2-[1-(3,3-Diphenylpropyl)-4-piperidinyl]-2,3-dihydro-3-phenyl-1H-isoindol-1-one

To a stirred solution of 475 mg (0.94 mmol) of Part A compound in 6 mL (excess) of trifluoroacetic acid under argon was added 386 mL (1.07 mmol) of triethylsilane. After 15 minutes, the bright yellow color dispersed and the reaction was concentrated under vacuum. The residue was dissolved in methylene chloride and washed with saturated sodium bicarbonate, dried (sodium sulfate) and concentrated to 400 mg of a yellow semisolid. The crude product was purified by flash chromatography on silica gel (125 g) eluted with 7:2:1 hexane/ethyl acetate/methanol to provide 343 mg (75%) of title compound as a white solid.
m.p.: 65-69 °C.

| Analysis Calcd. for $C_{34}H_{34}N_2O \cdot 0.43\ H_2O$: | | | |
|---|---|---|---|
| | C, 82.58; | H, 7.11; | N, 5.67 |
| Found: | C, 82.69; | H, 7.05; | N, 5.56. |

Example 293

2,3-Dihydro-2-[1-[4-[phenyl(phenylmethoxy)methyl]phenyl]-4-piperidinyl]-1H-isoindol-1-one

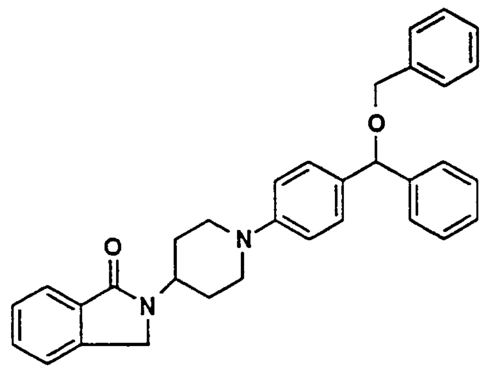

A.

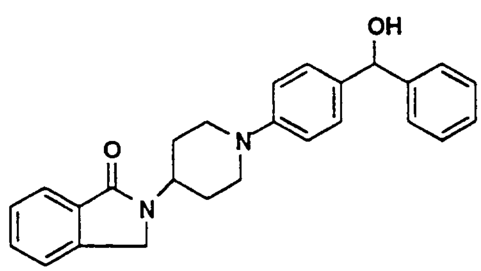

To a solution of Example 280 compound (500 mg, 1.26 mmol) in MeOH/THF (1:1)(30 mL) at 0 °C was added sodium borohydride (48 mg, 1.26 mmol). The reaction was stirred at 0 °C for 2 hours then warmed to RT overnight. Acetic acid was added to quench the reaction until the reaction was pH 6 by pH paper. The resulting mixture was evaporated to dryness. Ethyl ether (200 mL) was added to the residue, and the organic layer was washed with water (2 x 50 mL), saturated sodium bicarbonate solution (2 x 50 mL), brine (2 x 50 mL) and dried over $MgSO_4$. Evaporation gave title compound (355 mg, 71%) as a crude oil.

B. 2,3-Dihydro-2-[1-[4-[phenyl(phenylmethoxy)methyl]phenyl]-4-piperidinyl]-1H-isoindol-1-one

To a suspension of potassium hydride (153 mg, 0.89 mmol) in DMF (5 mL) at 0 °C was added a solution of Part B compound (355 mg, 0.89 mmol) in DMF (1 mL). The reaction was stirred at 0 °C for 30 min. Benzyl bromide (0.13 mL, 1.07 mmol) was added to the reaction at 0 °C. The reaction was stirred at 0 °C for 2 h then warmed to RT for 3 h. The reaction was quenched with saturated ammonium chloride solution (2 mL). Ethyl ether (200 mL) was added to the reaction, and the organic layer was washed with water (2 x 50 mL), brine (2 x 50 mL) and dried over $MgSO_4$. Purification was perfomed by flash chromatography on silica gel (100 g), loaded and eluted with 25% ethyl acetate in hexane. Pure fractions were combined and evaporated to give title compound (110 mg, 31%) as a white solid

m.p. 112-115 °C.

| Anal. Calc. for $C_{33}H_{32}N_2O_2 \cdot 0.4H_2O$: | | | |
|---|---|---|---|
| | C, 79.94; | H, 6.67; | N, 5.65; |
| Found: | C, 80.11; | H, 6.74; | N, 5.54. |

Example 294

2-[1-(3,3-Diphenylpropyl)-4-piperidinyl]-2,3-dihydro-4-hydroxy-1H-isoindol-1-one

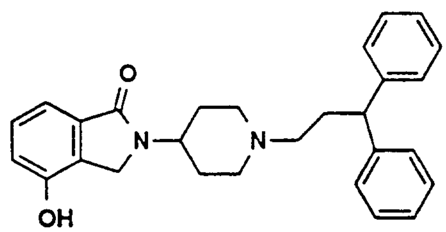

A.

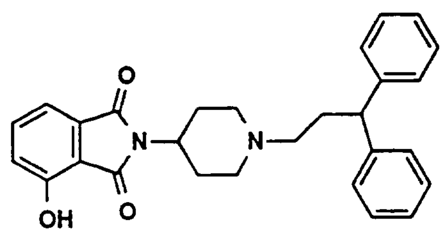

To a suspension of Example 1 Part E compound (4.48 g, 12.2 mmol) and 3-hydroxyphthalic anhydride (Aldrich) (2.0 g, 12.2 mmol) in toluene (10 mL) under argon was added N,N-diisopropylethylamine (4.7 mL, 26.8 mmol). The bright yellow mixture was heated at reflux with azeotropic removal of water with a Dean-Stark trap for 18 h. The biphasic reaction was cooled to RT, diluted with $CH_2Cl_2$ (150 mL), and washed with 1 M $Na_2CO_3$. The aqueous layer was adjusted to pH 7 with glacial acetic acid, and the organic layer was dried over $Na_2SO_4$. Evaporation gave a yellow foamy solid which was purified by flash chromatography on silica gel (300 g) eluting with a step gradient of 3:97 MeOH/$CH_2Cl_2$ to 5:95 MeOH/$CH_2Cl_2$ to afford title compound (4.21 g, 78%) as a yellow solid (mp 114-118 °C).

B. 2-[1-(3,3-Diphenylpropyl)-4-piperidinyl]-2,3-dihydro-4-hydroxy-1H-isoindol-1-one

Zinc dust (654 mg, 10 mmol) was added to a warm solution of Part A compound (440 mg, 1 mmol) in glacial acetic acid (3 mL). The reaction was refluxed under argon for 24 h, then cooled to RT. The reaction was filtered and the solids washed with glacial acetic acid (10 mL). The filtrate was concentrated in vacuo, and the resultant residue was azeotroped with toluene (10 mL). The residue was then dissolved in EtOAc (20 mL) and washed with saturated $NaHCO_3$ (5 mL) and brine (5 mL), then dried over $Na_2SO_4$. Evaporation gave 426 mg of a colorless glass, which was purified by flash chromatography on silica gel (40 g) to give title compound (128 mg, 30%) as a white solid, and the 7-hydroxy isomer (156 mg, 37%) as a colorless glass.
mp 223-25 °C.

| Anal. Calcd. for $C_{28}H_{30}N_2O_2$: | | | |
|---|---|---|---|
| | C, 78.84; | H, 7.09; | N, 6.57 |
| Found: | C, 78.53; | H, 7.16; | N, 6.95. |

Example 295

(Z)-2-[1-[4-(9H-Fluoren-9-yl)-2-butenyl]-4-piperidinyl]-2,3-dihydro-1H-isoindol-1-one

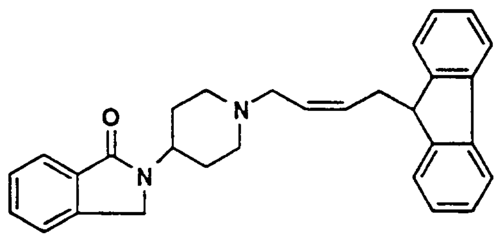

A.

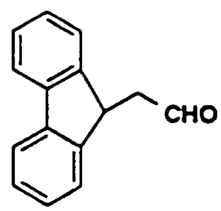

To a solution of oxalyl chloride (2.0M, 1.37 mL, 2.73 mmol) in dichloromethane (30 mL) at -74 °C was added dropwise a solution of DMSO (0.4 mL, 5.46 mmol) in dichloromethane (1 mL). The reaction was stirred at -74 °C for 1 h. A solution of Example 288, Part A compound (440 mg, 5.46 mmol) in dichloromethane (4 mL) was added dropwise. The reaction was stirred at -74 °C for 1.5 h. Triethylamine (1.5 g, 14.7 mmol) was added and the reaction was warmed to RT over 1 h. Ethyl ether (100 mL) was added and the organic layer was washed with 1N HCl solution (2 x 30 mL), water (2 x 30 mL), saturated sodium bicarbonate solution (2 x 30 mL), brine (2 x 30 mL) and dried over $MgSO_4$. Evaporation gave title compound (400 mg, 92%) as a crude oil.

B.

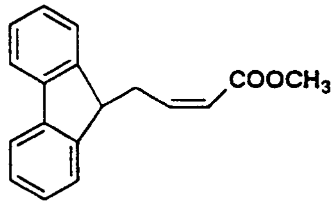

To a solution of bis(2,2,2-trifluoroethyl)(methoxycarbonylmethyl)phosphonate (672 mg, 2.11 mmol) and 18-crown-6 (557 mg, 2.11 mmol) in THF (10 mL) at 0 °C was added a solution of potassium bis-(trimethylsilyl)amide in toluene (0.5M, 4.22 mL, 2.11 mmol). The reaction was stirred at 0 °C for 30 min then cooled to -78 °C. A solution of Part A compound (400 mg, 1.92 mmol) in THF (1 mL) was added. The reaction was stirred at -78 °C for 1 h. The reaction was quenched with saturated ammonium chloride solution (5 mL). Ethyl ether (200 mL) was added to the reaction, and the organic layer was washed with water (2 x 30 mL), brine (2 x 30 mL) and dried over $MgSO_4$. Purification was performed by flash chromatography on silica gel (200 g), loaded and eluted with 20% dichloromethane in hexane. Pure fractions were combined and evaporated to give title compound (390 mg, 77%) as a colorless oil.

C.

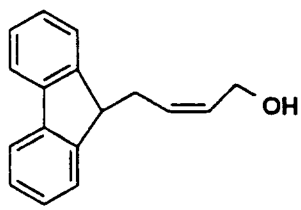

To a solution of Part B compound (390 mg, 1.48 mmol) in THF (20 mL) at 0 °C was added dropwise a solution of diisobutylaluminum hydride in hexane (1.0M, 3.26 mL, 3.26 mmol). The ice bath was removed and the reaction was stirred at RT for 15 min. The reaction was quenched by methanol (2 mL) followed by potassium sodium tartrate solution (1 M, 100 mL). The mixture was stirred at RT overnight. Ethyl ether (150 mL) was added and the organic layer was washed with water (2 x 30 mL), brine (2 x 30 mL) and dried over $MgSO_4$. Purification was performed by flash chromatography on silica gel (100 g), loaded and eluted with 20% ethyl acetate in hexane. Pure fractions were combined and evaporated to give title compound (240 mg, 69%) as a colorless oil.

D.

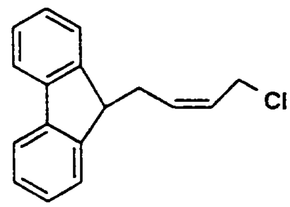

To a solution of N-chlorosuccinimide (155 mg, 1.17 mmol) in dichloromethane (5 mL) at -40 °C was added dropwise methyl sulfide (0.11 mL, 1.52 mmol). The reaction was stirred at -40 °C for 10 min then warmed to RT for 30 min. The reaction was recooled to -40 °C, and a solution of Part C compound (230 mg, 0.97 mmol) in dichloromethane (2 mL) was added dropwise. The reaction was stirred at -40 °C for 2 h then warmed to RT for 30 min. Ethyl ether (120 mL) was added to dilute the reaction and the organic layer was washed with water (2 x 30 mL), brine (2 x 30 mL) and dried over $MgSO_4$. Evaporation gave title compound (210 mg, 85%) as a colorless oil.

E. (Z)-2-[1-[4-(9H-Fluoren-9-yl)-2-butenyl]-4-piperidinyl]-2,3-dihydro-1H-isoindol-1-one

To a solution of Part D compound (216 mg, 0.85 mmol) in DMF (10 mL) was added Example 2 Part A compound (183 mg, 0.85 mmol) followed by anhydrous potassium carbonate (129 mg, 0.94 mmol). The reaction was stirred at RT overnight. Ethyl ether (100 mL) was added to dilute the reaction, and the solution was washed with water (2 x 30 mL), brine (2 x 30 mL) and dried over $MgSO_4$. Evaporation gave a crude oil. Purification was performed by flash chromatography on 100 g of silica gel, loaded and eluted with 2% methanol in dichloromethane. Pure fractions were combined and evaporated to give title compound (221 mg, 60%) as a white solid.
m.p. 112-116 °C.

| Anal. Calc. for $C_{30}H_{30}N_2O$ • 0.2 $H_2O$: | | | |
|---|---|---|---|
| | C, 82.23; | H, 6.99; | N, 6.39 |
| Found: | C, 81.92; | H, 7.00; | N, 6.59. |

Example 296

2-[1-(3,3-Diphenylpropyl)-4-piperidinyl]-2,3-dihydro-7-hydroxy-1H-isoindol-1-one

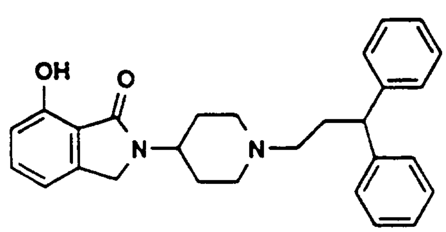

To a solution of Example 294 Part A compound (2.80 g, 6.36 mmol) in THF (30 mL) at -70 °C (internal temp) under argon was added lithium tri-sec-butylborohydride (L-Selectride®) (14.0 mL, 1.0M in THF, 14.0 mmol) dropwise over 45 min via syringe pump. The reaction was stirred at -70 °C for 10 min then allowed to warm to -40 °C over 1.5 h, at which time the reaction gelatinized to a yellow cake. The reaction was quenched with acetic acid (1 mL) and warmed to RT. The reaction was concentrated in vacuo and the

residue was partitioned between $CH_2Cl_2$ (75 mL) and 1M sodium phosphate buffer (pH 7, 25 mL). The organic layer was washed with brine (30 mL) then dried over $Na_2SO_4$. Evaporation gave 3.3 g of a pale yellow solid.

To a suspension of the crude product above in toluene (50 mL) was added trimethylamine N-oxide dihydrate (5.65 g, 50.9 mmol). The reaction was refluxed under argon for 3 h, then cooled to RT. The reaction was diluted with $CH_2Cl_2$ (200 mL) and washed with brine (50 mL) then dried over $Na_2SO_4$. Evaporation gave 2.45 g of a light brown solid.

To a solution of the crude product above in trifluoroacetic acid (30 mL) was added triethylsilane (1.5 mL, 9.54 mmol). The reaction was stirred vigorously under argon (became slightly exothermic) for 10 min, then concentrated in vacuo. The residue was dissolved in $CH_2Cl_2$ (100 mL) and washed with saturated $NaHCO_3$ (2 x 30 mL) and brine (30 mL), then dried over $Na_2SO_4$. Evaporation gave 2.6 g of a brown foam, which was purified by flash chromatography on silica gel (150 g) eluting with 15:85 isopropanol/hexane to afford title compound (890 mg, 33%) as a pale yellow solid. Additional clean product (990 mg, 36%) was obtained upon flushing flash column with 10:90 MeOH/$CH_2Cl_2$ (1 L).

mp 118-122 °C.

| Anal. Calcd. for $C_{28}H_{30}N_2O_2$: | | | |
|---|---|---|---|
| | C, 78.84; | H, 7.09; | N, 6.57 |
| Found: | C, 78.57; | H, 7.23; | N, 6.46. |

Example 297

2-[1-(3,3-Diphenylpropyl)-4-piperidinyl]-2,3-dihydro-7-phenyl-1H-isoindol-1-one

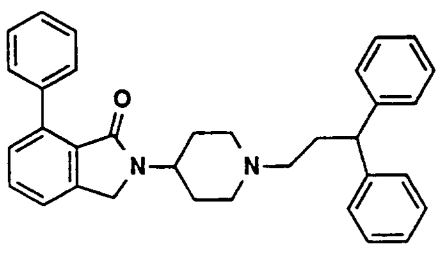

A.

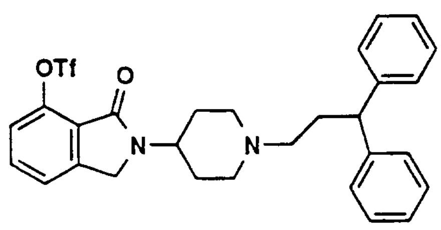

Trifluoromethanesulfonic anhydride (463 µL, 2.76 mmol) was added dropwise to a solution of Example 296 compound (980 mg, 2.30 mmol) in $CH_2Cl_2$ (10 mL) at -20 °C under argon. The reaction was stirred at -20 °C for 15 min, then washed with saturated $NaHCO_3$ (5 mL). The aqueous layer was extracted with $CH_2Cl_2$ (10 mL) and the combined organic layers were dried over $Na_2SO_4$. Evaporation gave title compound (1.38 g, 100%) as a crude brown foam.

B. 2-[1-(3,3-Diphenylpropyl)-4-piperidinyl]-2,3-dihydro-7-phenyl-1H-isoindol-1-one

A mixture of Part A compound (439 mg, 0.787 mmol), phenylboric acid (192 mg, 1.57 mmol), and anhydrous potassium carbonate (163 mg, 1.18 mmol) in toluene (8 mL) at RT was degassed by bubbling argon through the reaction solution. Tetrakis(triphenylphosphine)palladium (45 mg, 0.039 mmol) was added and the reaction was refluxed for 1.5 h. The reaction was cooled to RT, diluted with EtOAc (20 mL), and washed with saturated $NaHCO_3$, water, and brine (5 mL each). The organic layer was dried over $Na_2SO_4$ and evaporated to give 460 mg of a brown foam, which was chromatographed on silica gel (50 g) eluting with 10:90 acetone/$CH_2Cl_2$ to give 250 mg of a brown foam containing the desired product and an unidentified compound. The contaminated product (209 mg) was dissolved in toluene (4 mL) and trimethylamine N-oxide (244 mg, 2.2 mmol) was added. The reaction was refluxed under argon for 3.5 h, then cooled to RT. The reaction mixture was diluted with EtOAc (10 mL) and washed with saturated $NaHCO_3$ (3 mL) and brine (3 mL), then dried over $Na_2SO_4$. Evaporation gave 200 mg of a brown oil, which was purified by flash chromatography on silica gel (50 g) eluting with 1:99 MeOH/EtOAc to give title compound (111 mg, 35%) as a white foam.

| Anal. Calcd. for $C_{34}H_{34}N_2O \cdot 0.4\ H_2O$: | | | |
|---|---|---|---|
| | C, 82.69; | H, 7.10; | N, 5.67 |
| Found: | C, 82.97; | H, 7.11; | N, 5.47. |

Example 298

(Z)-2,3-Dihydro-2-[1-[4-[9-(2-propenyl)-9H-fluoren-9-yl]-2-butenyl]-4-piperidinyl]-1H-isoindol-1-one

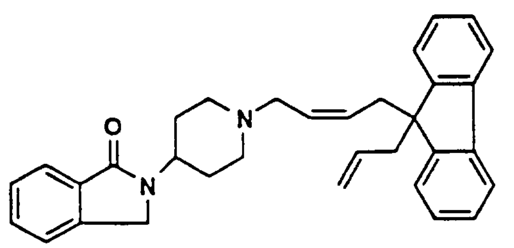

A.

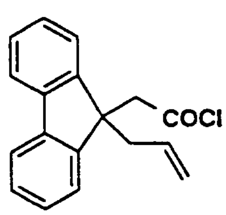

To a solution of Example 291 Part A compound (650 mg, 2.46 mmol) in dichloromethane (10 mL) at RT was added dropwise a solution of oxalyl chloride in dichloromethane (2.0M, 1.85 mL, 3.70 mmol) followed by DMF (3.69 µl, 0.05 mmol). The reaction was stirred at RT for 1 h. The reaction was evaporated to give title compound (650 mg, 100%) as a yellowish oil.

B.

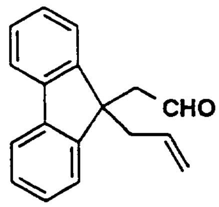

To a solution of Part A compound (650 mg, 2.46 mmol) in THF (10 mL) at -78 °C was added dropwise a solution of lithium tri-*tert*-butoxyaluminohydride (1.0M, 2.60 mL, 2.60 mmol) in THF over 2.5 h. The reaction was stirred at -78 °C overnight. The reaction was quenched with saturated ammonium chloride (5 mL) and warmed to RT. Ethyl ether (150 mL) was added and the organic layer was washed with 1 N potassium hydrogen sulfate solution (30 mL), water (2 x 30 mL), saturated sodium bicarbonate solution (2 x 30 mL), brine (2 x 30 mL) and dried over $MgSO_4$. Evaporation gave title compound (400 mg, 65%) as a crude oil.

C.

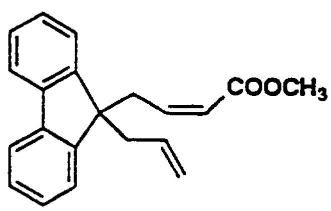

To a solution of bis(2,2,2-trifluoroethyl)(methoxycarbonylmethyl)phosphonate (560 mg, 1.76 mmol), 18-crown-6 (465 mg, 1.76 mmol) in THF (10 mL) at 0 °C was added a solution of potassium bis(trimethylsilyl)-amide in toluene (0.5M, 3.52 mL, 1.76 mmol). The reaction was stirred at 0 °C for 30 min then cooled to -78 °C. A solution of Part B compound (400 mg, 1.60 mmol) in THF (1 mL) was added. The reaction was stirred at -78 °C for 1 h. The reaction was quenched with saturated ammonium chloride solution (5 mL). Ethyl ether (120 mL) was added to the reaction, and the organic layer was washed with water (2 x 50 mL), brine (2 x 50 mL) and dried over $MgSO_4$. Purification was performed by flash chromatography on silica gel (100 g), loaded and eluted with 5% ethyl acetate in hexane. Pure fractions were combined and evaporated to give title compound (280 mg, 58%) as a colorless oil.

D.

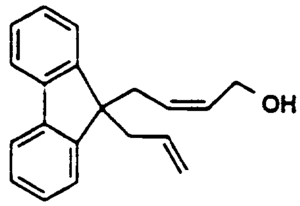

To a solution of Part C compound (280 mg, 0.92 mmol) in THF (8 mL) at 0 °C was added dropwise a solution of diisobutylaluminum hydride in toluene (1.0M, 2.03 mL, 3.26 mmol). The ice bath was removed and the reaction was stirred at RT for 15 min. The reaction was quenched by methanol (2 mL) followed by potassium sodium tartrate solution (1 M, 100 mL). The mixture was stirred at RT overnight. Ethyl ether (150 mL) was added and the organic layer was washed with water (2 x 30 mL), brine (2 x 30 mL) and dried over $MgSO_4$. Purification was performed by flash chromatography on silica gel (100 g), loaded and eluted with 20% ethyl acetate in hexane. Pure fractions were combined and evaporated to give title compound (240 mg, 69%) as a colorless oil.

E.

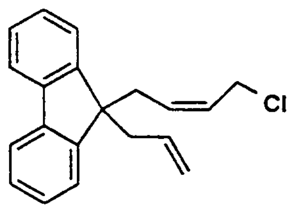

To a solution of N-chlorosuccinimide (122 mg, 0.91 mmol) in dichloromethane (5 mL) at -40 °C was added dropwise methyl sulfide (0.08 mL, 1.14 mmol). The reaction was stirred at -40 °C for 10 min then warmed to RT for 30 min. The reaction was recooled to -40 °C, and a solution of Part D compound(210 mg, 0.76 mmol) in dichloromethane (2 mL) was added dropwise. The reaction was stirred at -40 °C for 2 h then warmed to RT for 30 min. Ethyl ether (120 mL) was added to dilute the reaction and the solution was washed with water (2 x 30 mL), brine (2 x 30 mL) and dried over $MgSO_4$. Evaporation gave title compound (210 mg, 94%) as a colorless oil.

F. (Z)-2,3-Dihydro-2-[1-[4-[9-(2-propenyl)-9H-fluoren-9-yl]-2-butenyl]-4-piperidinyl]-1H-isoindol-1-one

To a solution of Part E compound (210 mg, 0.71 mmol) in DMF (8 mL) was added Example 2 Part A compound (154 mg, 0.71 mmol) followed by anhydrous potassium carbonate (108 mg, 0.78 mmol). The reaction was stirred at RT overnight. Ethyl ether (100 mL) was added to dilute the reaction, and the solution was washed with water (2 x 30 mL), brine (2 x 30 mL) and dried over $MgSO_4$. Evaporation gave a crude oil. Purification was performed by flash chromatography on 100 g of silica gel, loaded and eluted with 1% methanol in dichloromethane. Pure fractions were combined and evaporated to give title compound (219 mg, 65%) as a white solid.
m.p. 117-120 °C.

| Anal. Calc. for $C_{33}H_{34}N_2O \bullet 0.6H_2O$: | | | |
|---|---|---|---|
| | C, 81.65; | H, 7.31; | N, 5.77 |
| Found: | C, 81.69; | H, 7.31; | N, 5.64 |

Example 299

2,3-Dihydro-2-[1-[4-[phenyl(3-phenylpropoxy)methyl]phenyl]-4-piperidinyl]-1H-isoindol-1-one

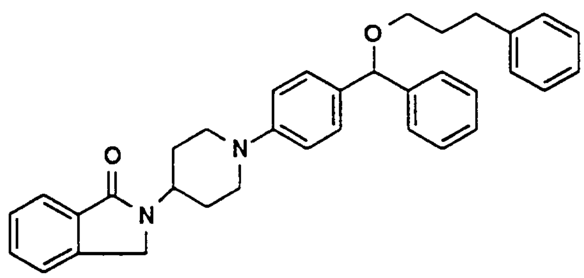

To a suspension of sodium hydride (44 mg, 1.1 mmol) in DMF (5 mL) at 0 °C was added dropwise a solution of Example 293 Part A compound (400 mg, 1.0 mmol) in DMF (1 mL). The reaction was stirred at 0 °C for 30 min. 1-Bromo-3-phenylpropane (219 mg, 1.1 mmol) was added dropwise to the reaction at 0 °C. The reaction was stirred at 0 °C for 1 h then warmed to RT overnight. The reaction was quenched with saturated ammonium chloride solution (2 mL). Ethyl acetate (100 mL) was added to the reaction, and the organic layer was washed with water (2 x 30 mL), brine (2 x 30 mL) and dried over $MgSO_4$. Purification was performed by flash chromatography on silica gel (100 g), loaded and eluted with 50% ethyl acetate in hexane. Pure fractions were combined and evaporated to give title compound (335 mg, 65%) as a white solid.

m.p. 117-120 ° C.

| Anal. Calc. for $C_{35}H_{36}N_2O_2 \bullet 0.3\ H_2O$: | | | |
|---|---|---|---|
| | C, 80.52; | H, 7.07; | N, 5.37 |
| Found: | C, 80.64; | H, 7.01; | N, 5.11. |

Example 300

2,3-Dihydro-2-[1-[4-[(4-pyridinylmethoxy)phenylmethyl]phenyl]-4-piperidinyl]-1H-isoindol-1-one

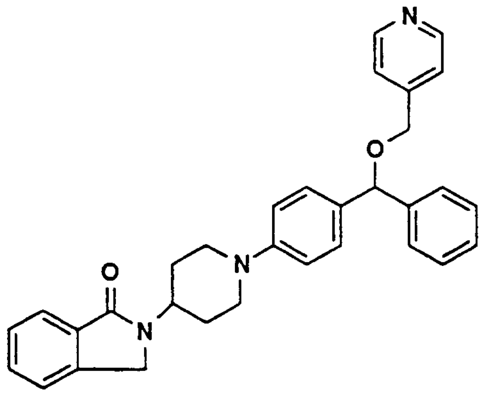

To a solution of 4-picolyl chloride hydrochloride (226 mg, 1.38 mmol) in water (4 mL) was added sodium hydroxide solution (1N, 1.5 mL, 1.50 mmol). The resulting solution was extracted with ethyl ether (3 x 5 mL). The organic layers were combined and evaporated to give 4-picolyl chloride (175 mg, 100%).

To a suspension of sodium hydride (55.3 mg, 1.38 mmol) in DMF (5 mL) at 0 °C was added dropwise a solution of Example 293 Part A compound (500 mg, 1.26 mmol) in DMF (8 mL). The reaction was stirred at 0 °C for 30 min. A solution of 4-picolyl chloride (175 mg, 1.38 mmol) in DMF (1 mL) was added dropwise to the reaction at 0 °C. The reaction was stirred at 0 °C for 1 h then warmed to RT overnight. The reaction was quenched with saturated ammonium chloride solution (2 mL). Ethyl acetate (200 mL) was added to the reaction, and the organic layer was washed with water (2 x 100 mL), brine (2 x 100 mL) and dried over $MgSO_4$. Purification was performed by flash chromatography on silica gel (100 g), loaded and eluted with 70% EtOAc in hexane. Pure fractions were combined and evaporated to give title compound (320 mg, 52%) as a white solid.
m.p. 77-81 °C.

| Anal. Calc. for $C_{32}H_{31}N_3O_2$ • 0.2 $H_2O$: | | | |
|---|---|---|---|
| | C, 77.93; | H, 6.42; | N, 8.52 |
| Found: | C, 77.69; | H, 6.21; | N, 8.71. |

Example 301

2,3-Dihydro-2-[1-[4-[(2-pyridinylmethoxy)phenylmethyl]phenyl]-4-piperidinyl]-1H-isoindol-1-one

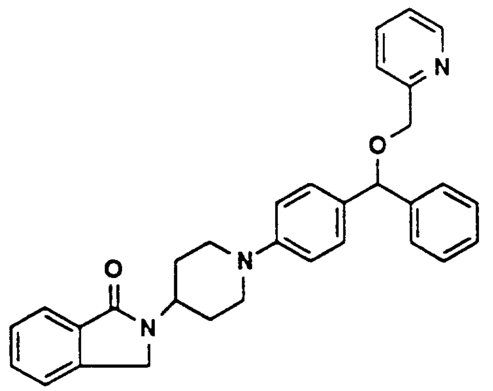

Following the procedure for the preparation of the compound of Example 300, reaction of Example 293 Part A compound (500 mg, 1.26 mmol) and 2-picolyl chloride hydrochloride (316 mg, 1.89 mmol) gave title compound (350 mg, 56%) as a white solid.
m.p. 149-151 °C.

| Anal. Calc. for $C_{32}H_{31}N_3O_2$: | | | |
|---|---|---|---|
| | C, 78.50; | H, 6.38; | N, 8.58 |
| Found: | C, 78.19; | H, 6.32; | N, 8.78. |

Examples 302 to 308

Following the procedures set out in Examples 289 to 314, the following compounds of the invention were prepared.
302.

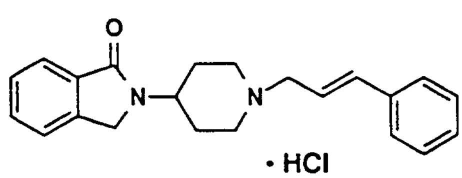

• HCl

m.p.: 260-264 °C.

| Elemental Anal. Calc. for $C_{22}H_{25}ClN_2O \cdot 0.3\ H_2O$: | | | | |
|---|---|---|---|---|
| | C, 70.59; | H, 6.89; | N, 7.48; | Cl, 9.47 |
| Found: | C, 70.77; | H, 6.96; | N, 7.47; | Cl, 9.49. |

303.

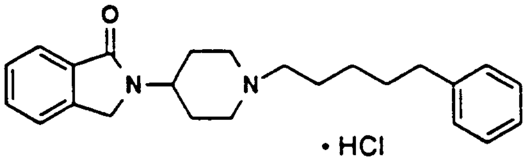

m.p.: 199-201 ° C.

| Elemental Anal. Calc. for $C_{24}H_{31}ClN_2O \cdot 0.2\ H_2O$: | | | | |
|---|---|---|---|---|
| | C, 71.60; | H, 7.86; | N, 6.96; | Cl, 8.81 |
| Found: | C, 71.60; | H, 7.95; | N, 6.93; | Cl, 8.89. |

304.

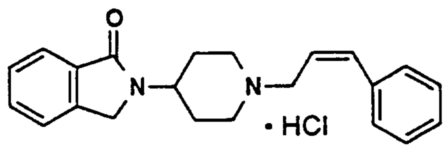

m.p.: 244-247 ° C.

| Elemental Anal. Calc. for $C_{22}H_{25}ClN_2O$: | | | | |
|---|---|---|---|---|
| | C, 71.63; | H, 6.83; | N, 7.59; | Cl, 9.61 |
| Found: | C, 71.61; | H, 6.84; | N, 7.50; | Cl, 9.75. |

305.

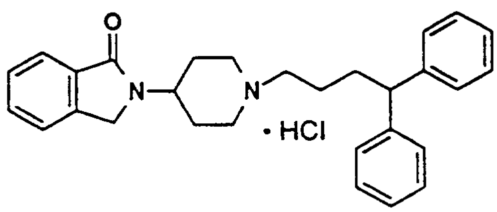

m.p. 212-215 ° C.

| Elemental Anal. Calc. for $C_{29}H_{33}ClN_2O \cdot 0.4\ H_2O$: | | | | |
|---|---|---|---|---|
| | C, 74.39; | H, 7.28; | N, 5.98; | Cl, 7.57 |
| Found: | C, 74.27; | H, 7.28; | N, 6.22; | Cl, 7.66. |

306.

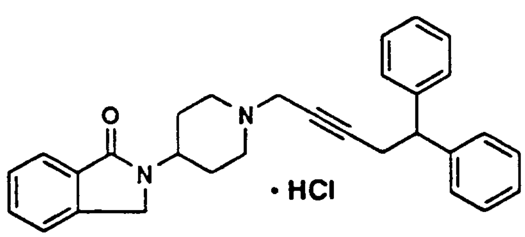

m.p.: 221-225 °C.

| Elemental Anal. Calc. for $C_{30}H_{31}ClN_2O \cdot 1.5\ H_2O$: | | | | |
|---|---|---|---|---|
| | C, 72.35; | H, 6.88; | N, 5.62; | Cl, 7.12 |
| Found: | C, 72.35; | H, 7.02; | N, 5.51; | Cl, 6.79. |

307.

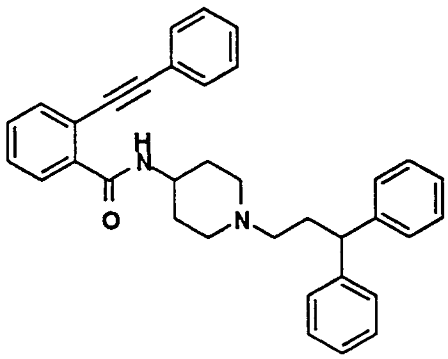

MS (Cl, pos. ions) 497 (M + H)

| Analysis Calcd for $C_{35}H_{34}N_2O \cdot 0.99\ H_2O$: | | | |
|---|---|---|---|
| | C, 81.38; | H, 7.02; | N, 5.42 |
| Found: | C, 81.35; | H, 6.74; | N, 5.45. |

308.

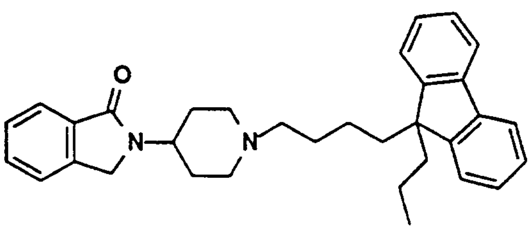

MS (ES) 479 (M + H)

| Anal. Calcd. for $C_{33}H_{38}N_2O \cdot 0.6\ H_2O$: | | | |
|---|---|---|---|
| | C, 80.97; | H, 8.07; | N, 5.72 |
| Found: | C, 80.95; | H, 7.87; | N, 5.65. |

Example 309

(Z)-2,3-Dihydro-2-[1-[4-[9-(3-phenylpropyl)-9H-fluoren-9-yl]-2-butenyl]-4-piperidinyl]-IH-isoindol-1-one

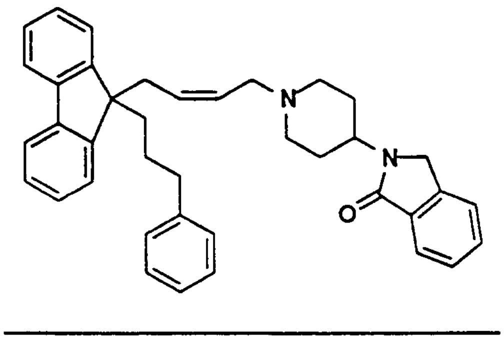

A.

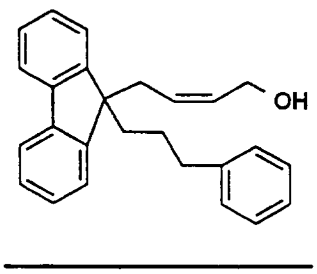

To a solution of the alcohol prepared in Example 295, Part C (330 mg, 1.40 mmol) in THF (8 mL) at -78 °C was added dropwise a solution of n-butyllithium in hexane (1.6M, 1.84 mL, 2.94 mmol) followed by a solution of 1-bromophenylpropane (0.26 mL, 1.68 mmol) in THF (1 mL). The reaction was stirred at -78 °C for 30 min, then warmed to -25 °C for 30 min. The reaction was quenched with saturated ammonium chloride solution (2 mL) and warmed to RT. Ethyl ether (100 mL) was added and the organic layer was washed with water (2 x 50 mL), brine (2 x 50 mL) and dried over $MgSO_4$. Purification was performed by flash chromatography on silica gel (200 g), loaded and eluted with 20% ethyl acetate in hexane. Pure fractions were combined and evaporated to give title compound (410 mg, 81%) as a colorless oil.

B.

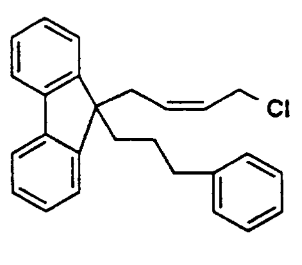

Following the procedure in Example 295, Part D, the above Part A compound (400 mg, 1.13 mmol) was reacted to give title compound (360 mg, 86%) as a colorless oil.

C. (Z)-2,3-Dihydro-2-[1-[4-[9-(3-phenylpropyl)-9H-fluoren-9-yl]-2-butenyl]-4-piperidinyl]-1H-isoindol-1-one

To a solution of Part B compound (360 mg, 0.97 mmol) in DMF (10 mL) was added Example 2, Part A compound (209 mg, 0.97 mmol) followed by anhydrous potassium carbonate (160 mg, 1.16 mmol). The reaction was stirred at 50 °C overnight. Ethyl ether (70 mL) was added to dilute the reaction, and the solution was washed with water (2 x 50 mL), brine (2 x 50 mL) and dried over $MgSO_4$. Evaporation gave a crude oil. Purification was performed by flash chromatography on 100 g of silica gel, loaded and eluted with 2.5% methanol in dichloromethane. Pure fractions were combined and evaporated to give title compound (310 mg, 58%) as a white solid. m.p. 118-122 °C.
MS (Cl, + ion): 553 (M + H).

| Anal. Calc. for $C_{39}H_{40}N_2O$ • 1.2 $H_2O$: | | | |
|---|---|---|---|
| | C, 81.55; | H, 7.44; | N, 4.88 |
| Found: | C, 81.63; | H, 7.51; | N, 4.78. |

**Claims**

**1.** A compound which has the structure

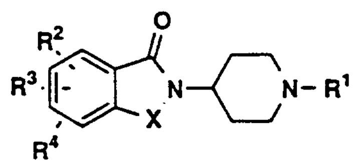 ,

or

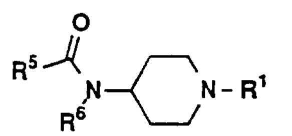 ,

or

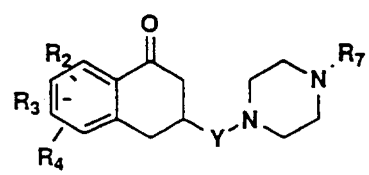

where X is: $CHR^8$,

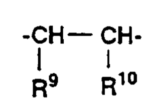 or 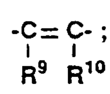

$R^8$, $R^9$ and $R^{10}$ are independently hydrogen, alkyl, alkenyl, alkynyl, aryl, arylalkyl, heteroaryl, heteroarylalkyl, cycloalkyl, or cycloalkylalkyl;

Y is $-(CH_2)_m-$ or

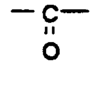

wherein m is 2 or 3;

$R^1$ is alkyl, alkenyl, alkynyl, aryl, heteroaryl, arylalkyl wherein alkyl has at least 2 carbons, diarylalkyl, arylalkenyl, diarylalkenyl, arylalkynyl, diarylalkynyl, diarylalkylaryl, heteroarylalkyl wherein

alkyl has at least 2 carbons, cycloalkyl, or cycloalkylalkyl wherein alkyl has at least 2 carbons, all optionally substituted through available carbon atoms with 1, 2, or 3 groups selected from halo, haloalkyl, alkyl, alkenyl, alkoxy, aryloxy, aryl, arylalkyl, alkylmercapto, arylmercapto, cycloalkyl, cycloalkylalkyl, heteroaryl, fluorenyl, heteroarylalkyl, hydroxy or oxo;

or $R^1$ is a group of the structure

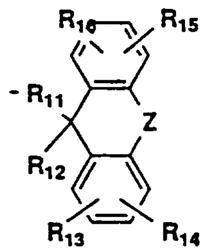

$R^{11}$ is a bond, alkylene, alkenylene or alkynylene of up to 6 carbon atoms; arylene or mixed arylene-alkylene; $R^{12}$ is hydrogen, alkyl, alkenyl, aryl, heteroaryl, haloalkyl, arylalkyl, arylalkenyl, cycloalkyl, aryloxy, alkoxy, arylalkoxy, heteroarylalkyl or cycloalkylalkyl, Z is bond, O, S, N-alkyl, N-aryl, or alkylene or alkenylene from 1 to 5 carbon atoms; $R^{13}$, $R^{14}$, $R^{15}$, and $R^{16}$ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, carboxy, aminocarbonyl, alkylcarbonyloxy, alkylcarbonylamino, arylalkyl, heteroaryl, heteroarylalkyl or aryloxy;

or $R^1$ is a group of the structure

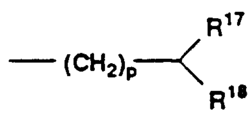

wherein p is 1 to 8 and $R^{17}$ and $R^{18}$ are each independently H, alkyl, alkenyl, aryl, arylalkyl, heteroaryl, heteroarylalkyl, cycloalkyl or cycloalkylalkyl at least one of $R^{17}$ and $R^{18}$ being other than H;

or $R^1$ is a group of the structure

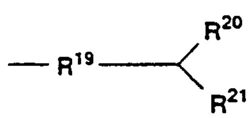

wherein

$R^{19}$ is aryl or heteroaryl;

$R^{20}$ is aryl or heteroaryl;

$R^{21}$ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;

$R^2$, $R^3$, $R^4$ are independently hydrogen, halo, alkyl, haloalkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl;

$R^5$ is independently alkyl of at least 2 carbons, alkenyl, alkynyl, aryl, heteroaryl, arylalkyl, heteroarylalkyl, cycloalkyl, cycloalkylalkyl, polycycloalkyl, polycycloalkylalkyl, cycloalkenyl, cycloalkenylalkyl, polycycloalkenyl, polycycloalkenylalkyl, heteroarylcarbonyl, all optionally substituted through available carbon atoms with 1, 2, or 3 groups selected from hydrogen, halo, alkyl, haloalkyl, alkoxy, haloalkoxy, alkenyl, alkynyl, cycloalkyl, cycloalkylalkyl, cycloheteroalkyl, cycloheteroalkylalkyl, aryl, heteroaryl, arylalkyl, arylcycloalkyl, arylalkenyl, aryloxy, aryloxyalkyl, arylalkoxy, arylazo, heteroaryloxo, heteroarylalkyl, heteroarylalkenyl, heteroaryloxy, hydroxy, nitro, cyano, amino, thiol, alkylthio, arylthio, heteroarylthio, arylthioalkyl, alkylcarbonyl, arylcarbonyl, arylaminocarbonyl, alkoxycarbonyl, aminocarbonyl, alkynylaminocarbonyl, alkylaminocarbonyl, alkenylaminocarbonyl, alkylcarbonyloxy, arylcarbonyloxy, alkylcarbonylamino, arylcarbonylamino, arylsulfinyl, arylsulfinylalkyl, arylsulfonyl, alkylsul-

fonyl, arylsulfonylamino;

$R^6$ is hydrogen or $C_1$-$C_4$ alkyl or $C_1$-$C_4$ alkenyl;

$R^7$ is alkyl, aryl or arylalkyl wherein alkyl by itself or as part of arylalkyl is optionally substituted with oxo

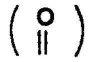;

or

pharmaceutically acceptable salts thereof, or prodrug esters thereof, with the provisos that where in the first formula X is $CH_2$, and $R^2$, $R^3$ and $R^4$ are each H, then $R^1$ will be other than 3,3-diphenylpropyl, and in the third formula, where one of $R^2$, $R^3$ and $R^4$ is 6-fluoro, and the others are H, $R^7$ will be other than 4-O-methoxyphenyl.

**2.** The compound as defined in Claim 1 having the formula

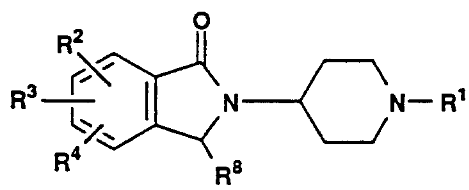

or

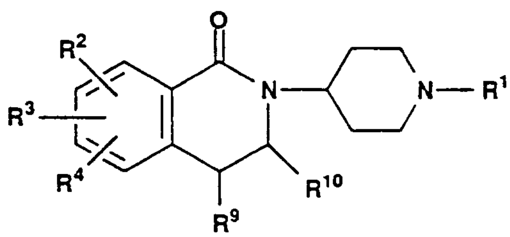

,

or

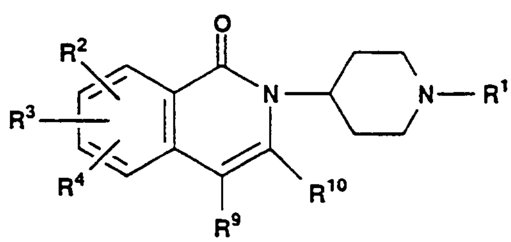

.

**3.** The compound as defined in Claim 2 having the formula

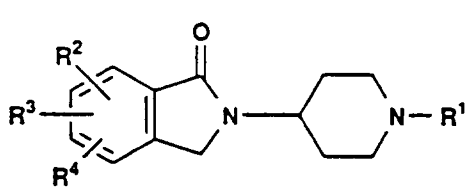

.

**4.** The compound as defined in Claim 1 having the formula

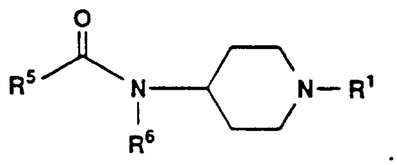

5. The compound as defined in Claim 1 having the formula

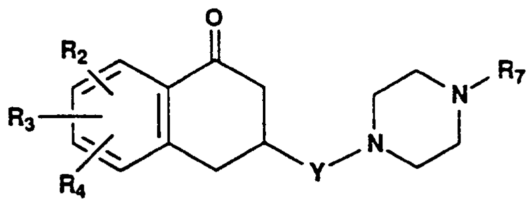

6. The compound as defined in Claim 5 wherein Y is $-CH_2-$.

7. The compound as defined in Claim 1 wherein $R^1$ is arylalkyl, arylalkenyl, heteroarylalkyl, heteroarylalkenyl,

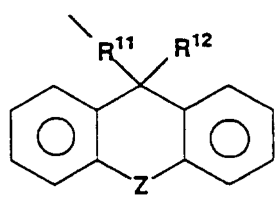

where $R^{11}$ is alkylene or alkenylene, $R^{12}$ is H, alkyl, alkenyl, aralkyl, aralkenyl and Z is O or a bond; or $R^1$ is

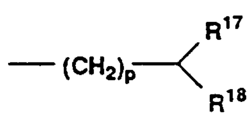

wherein $(CH_2)_p$ represents an alkylene chain or cis alkenylene of up to 6 carbons;

$R^{17}$ and $R^{18}$ are each independently alkyl, alkenyl, aryl, arylalkyl,heteroaryl, heteroarylalkyl, cycloalkyl, cycloalkylalkyl; or $R^1$ is

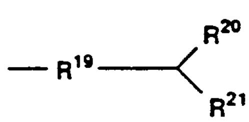

$R^{19}$ is aryl or heteroaryl; $R^{20}$ is aryl or heteroaryl;

$R^{21}$ is H, alkyl, aryl, alkylaryl, arylalkyl, aryloxy, arylalkoxy, heteroaryl, heteroarylalkyl, heteroarylalkoxy, cycloalkyl, cycloalkylalkyl, or cycloalkylalkoxy.

8. The compound as defined in Claim 1 wherein $R^2$ $R^3$ and $R^4$ are each H, X is $CH_2$, $CH=CH$ or $CH_2CH_2$.

9. The compound as defined in Claim 4 wherein $R^6$ is H and $R^5$ is phenyl having an ortho hydrophobic substituent.

**10.** The compound as defined in Claim 5 wherein Y is $CH_2$, $R^2$, $R^3$ and $R^4$ are each H or halo and $R^7$ is aryl.

**11.** The compound as defined in Claim 1 which is:
N-[1-(3,3-diphenylpropyl)-4-piperidinyl]benzamide
2-[1-(3,3-diphenyl-2-propenyl)-4-piperidinyl]-2,3-dihydro-1H-isoindol-1-one;
2,3-dihydro-2-[1-(3-phenylpropyl)-4-piperidinyl]-1H-isoindol-1-one;
2-[1-(5,5-diphenylpentyl)-4-piperidinyl]-2,3-dihydro-1H-isoindol-1-one;
N-[1-(3,3-diphenylpropyl)-4-piperidinyl]cyclohexanecarboxamide;
2-[1-(3-butylheptyl)-4-piperidinyl]-2,3-dihydro-1H-isoindol-1- one;
N-[1-(3,3-diphenylpropyl)-4-piperidinyl]pentamide;
(E)-2,3-dihydro-2-[1-[3-(2-phenoxyphenyl)-2-propenyl]-4-piperidinyl]-1H-isoindol-1-one;
2,3-dihydro-2-[1-[3-(2-methoxyphenyl)propyl]-4-piperidinyl]-1H-isoindol-1-one;
3,4-dihydro-3-[[4-phenyl-1-piperazinyl]methyl]-1(2H)-naphthalenone;
3,4-dihydro-3-[[4-(2-methoxyphenyl)-1-piperazinyl]carbonyl]-1(2H)-naphthalenone;
3,4-dihydro-3-[[4-(2-methoxyphenyl)-1-piperazinyl]methyl]-1(2H)-naphthalenone, dihydrochloride
N-[1-(phenylmethyl)-4-piperidinyl]-1H-indole-3-acetamide;
4-methoxy-α-(4-methoxyphenyl)-N-[1-(2-phenylethyl)-4-piperidinyl]benzeneacetamide;
2,3-dihydro-2-[1-[3-(2-phenoxyphenyl)propyl]-4-piperidinyl]-1H-isoindol-1-one;
(Z)-2,3-dihydro-2-[1-(5,5-diphenyl-2-pentenyl)-4-piperidinyl]-1H-isoindol-1-one;
N-[1-(3,3-diphenylpropyl)-4-piperidinyl]-2-methoxybenzamide;
N-[1-(3,3-diphenylpropyl)-4-piperidinyl]-2-methylbenzamide;
N-[1-(3,3-diphenylpropyl)-4-piperidinyl]-2-pyridinamide;
N-[1-(3,3-diphenylpropyl)-4-piperidinyl]-N-methylbenzamide;
or a hydrochloride salt, or other pharmaceutically acceptable salt thereof.

**12.** A monohydrochloride salt or dihydrochloride salt of the compounds of Claim 1.

**13.** The compound as defined in Claim 1 which is (Z)-2,3-dihydro-2-[1-[3-(2-phenoxyphenyl)-2-propenyl]-4-piperidinyl]-1H-isoindol-1-one or salts thereof;
2,3-dihydro-2-[1-(4-(hydroxyphenylmethyl)phenyl]-4-piperidinyl]-1H-isoindol-1-one or salts thereof;
2,3-dihydro-2-[1-[4-(phenylmethyl)phenyl]-4-piperidinyl]-1H-isoindol-1-one, or salts thereof;
2-[1-(4-benzoylphenyl)-4-piperidinyl]-2,3-dihydro-1H-isoindol-1-one or salts thereof;
2-[1-[4-(diphenylmethyl)phenyl]-4-piperidinyl]-2,3-dihydro-1H-isoindol-1-one or salts thereof;
(Z)-N-[1-(5,5-diphenyl-2-pentenyl)-4-piperidinyl]-2-phenoxybenzamide or salts thereof;
(Z)-N-[1-(5,5-diphenyl-2-pentenyl)-4-piperidinyl]-3-phenoxybenzamide or salts thereof;
2,3-dihydro-2-[1-[3-phenyl-3-(4-propylphenyl)propyl]-4-piperidinyl]-1H-isoindol-1-one, or salts thereof;
2-[1-[3,3-diphenylpropyl]-4-piperidinyl]-1,2-dihydro-1-isoquinolinone, or salts thereof;
2-[1-(3,3-diphenylpropyl)-4-piperidinyl]-3,4-dihydro-1(2H)-isoquinolinone, or salts thereof;
2-[1-(2-(9H-fluoren-9-yl)ethyl]-4-piperidinyl]-2,3-dihydro-1H-isoindol-1-one or salts thereof;
2-[1-(3,3-diphenylpropyl)-4-piperidinyl]-2,3-dihydro-3-(phenylmethyl)-1H-isoindol-1-one, or salts thereof;
2-[1-(3,3-diphenylpropyl)-4-piperidinyl]-2,3-dihydro-3-methyl-1H-isoindol-1-one, or salts thereof;
2,3-dihydro-2-[1-[2-[9-(2-propylenyl)-9H-fluoren-9-yl]ethyl]-4-piperidinyl]-1H-isoindol-1-one or salts thereof;
2-[1-(3,3-diphenylpropyl)-4-piperidinyl]-2,3-dihydro-3-phenyl-1H-isoindol-1-one or salts thereof;
2,3-dihydro-2-[1-[4-[phenyl(phenylmethoxy)methyl]phenyl]-4-piperidinyl]-1H-isoindol-1-one or salts thereof;
2-[1-(3,3-diphenylpropyl)-4-piperidinyl]-2,3-dihydro-4-hydroxy-1H-isoindol-1-one or salts thereof;
(Z)-2-[1-[4-(9H-fluoren-9-yl)-2-butenyl]-4-piperidinyl]-2,3-dihydro-1H-isoindol-1-one or salts thereof;
2-[1-(3,3-diphenylpropyl)-4-piperidinyl]-2,3-dihydro-7-hydroxy-1H-isoindol-1-one or salts thereof;
2-[1-(3,3-diphenylpropyl)-4-piperidinyl]-2,3-dihydro-7-phenyl-1H-isoindol-1-one or salts thereof;
(Z)-2,3-dihydro-2-[1-[4-[9-(2-propenyl)-9H-fluoren-9-yl]-2-butenyl]-4-piperidinyl]-1H-isoindol-1-one or salts thereof;
2,3-dihydro-2-[1-[4-phenyl(3-phenylpropoxy)methyl]phenyl]-4-piperidinyl]-1H-isoindol-1-one or salts thereof;
2,3-dihydro-2-[1-[4-[(4-pyridinylmethoxy)phenylmethyl]phenyl]-4-piperidinyl]-1H-isoindol-1-one or

salts thereof;

2,3-dihydro-2-[1-[4-[(2-pyridinylmethoxy)phenylmethyl]phenyl]-4-piperidinyl]-1H-isoindol- 1-one or salts thereof.

**14.** The compound as defined in Claim 1 having the structures

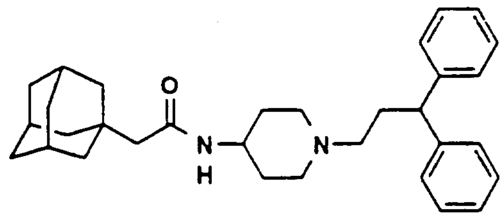

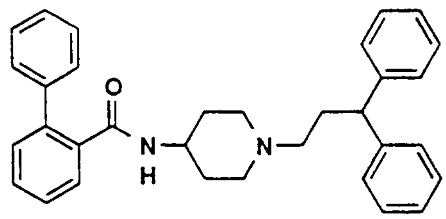

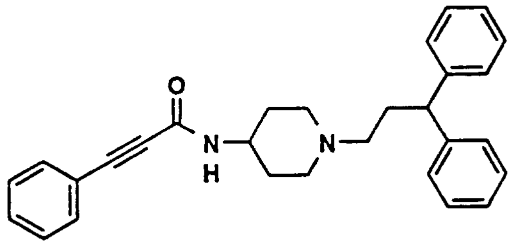

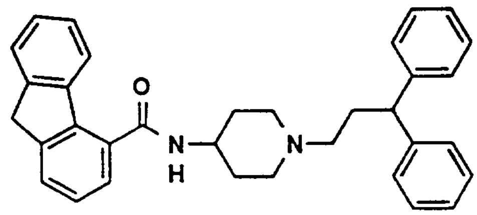

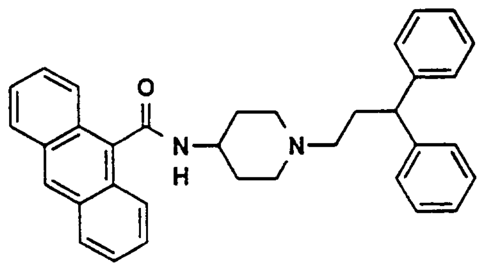

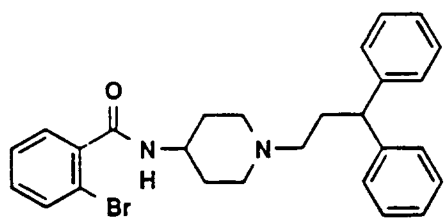

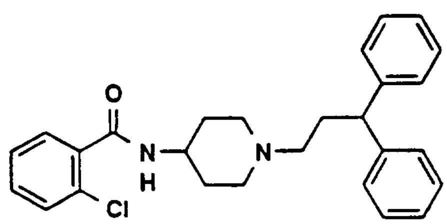

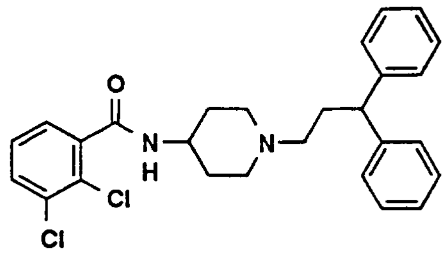

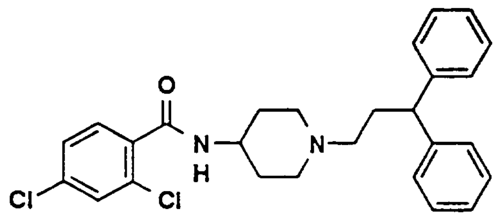

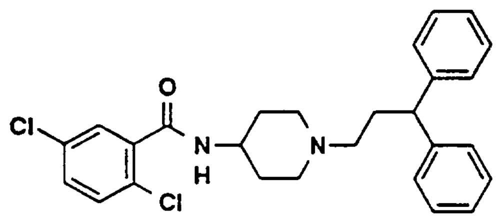

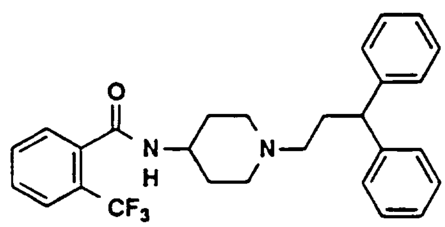

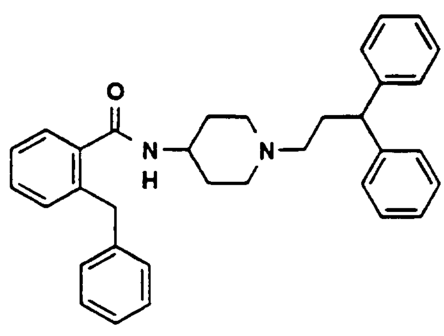

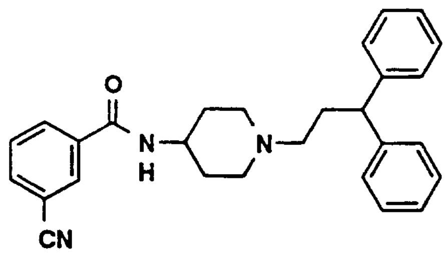

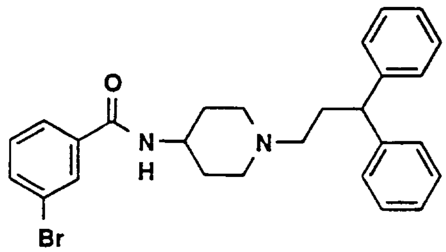

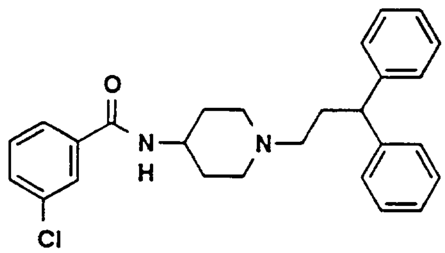

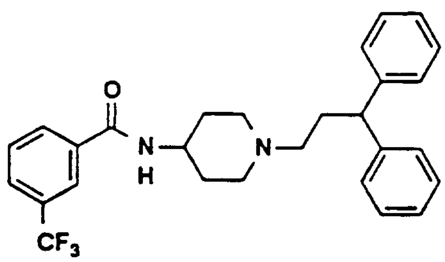

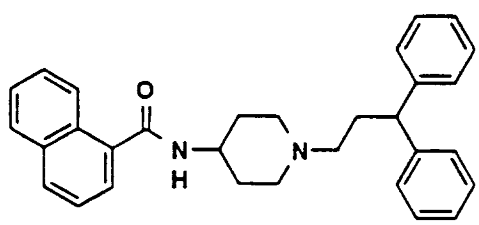

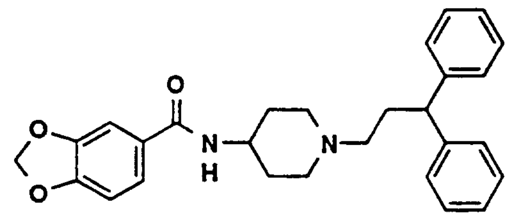

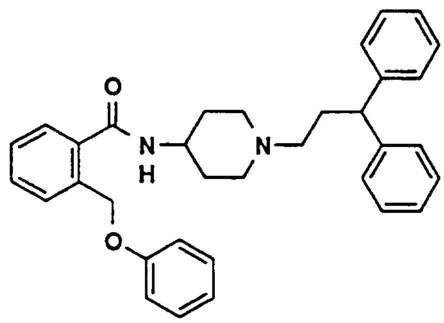

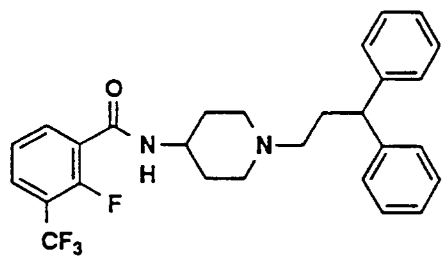

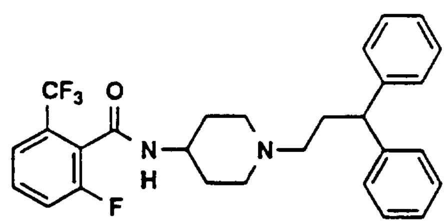

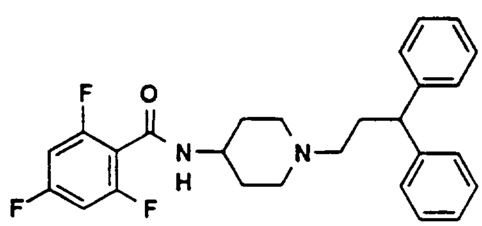

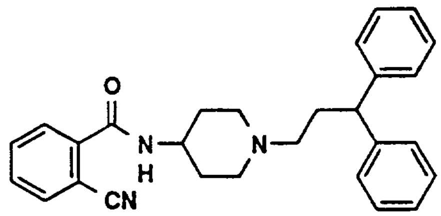

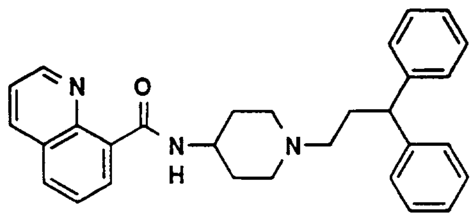

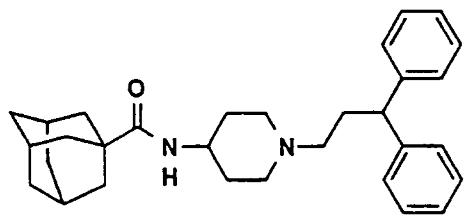

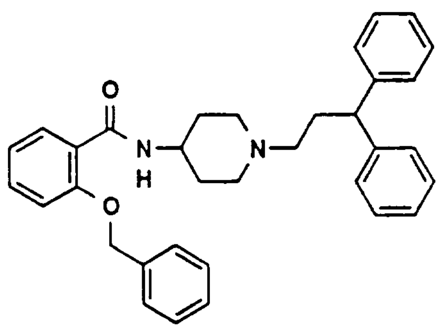

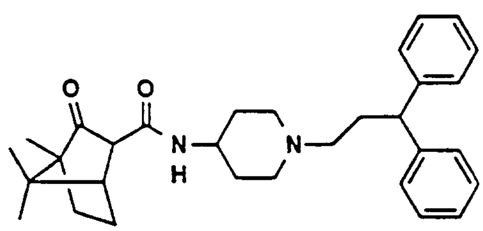

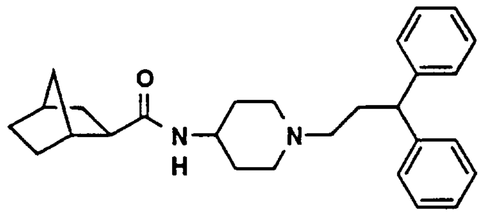

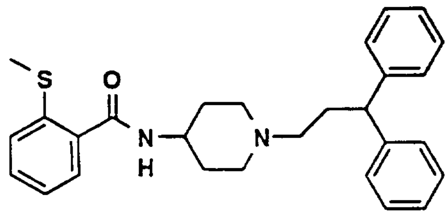

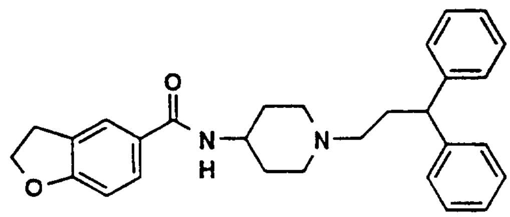

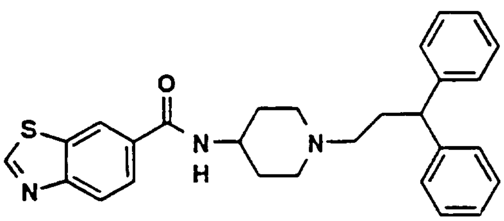

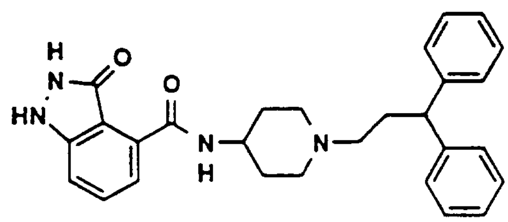

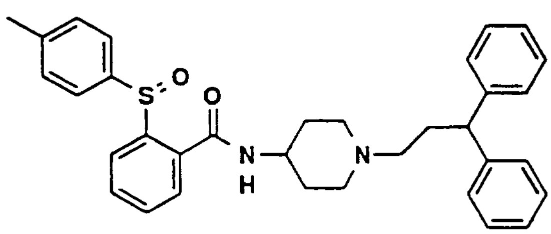

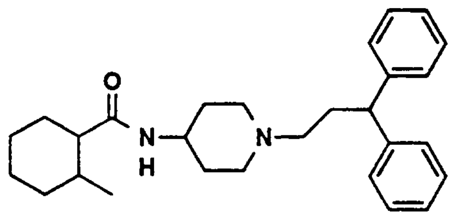

mixture of cis and trans

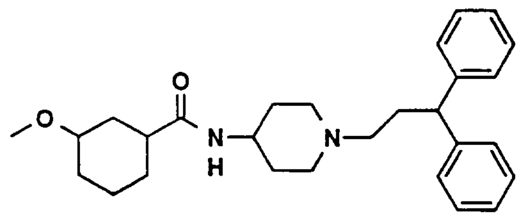

mixture of cis and trans

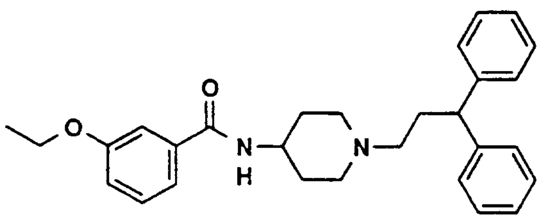

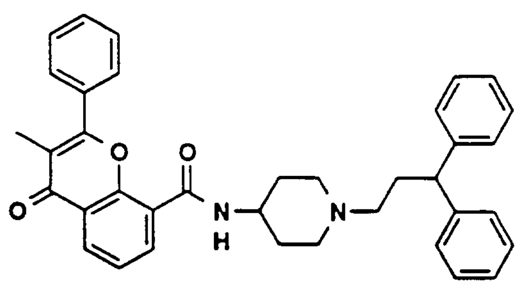

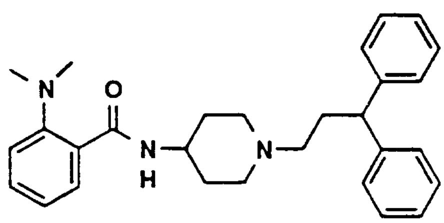

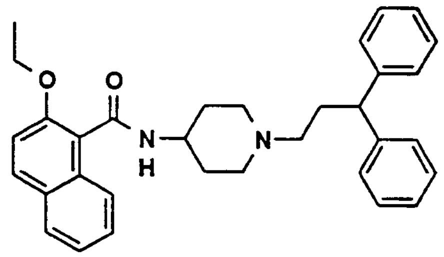

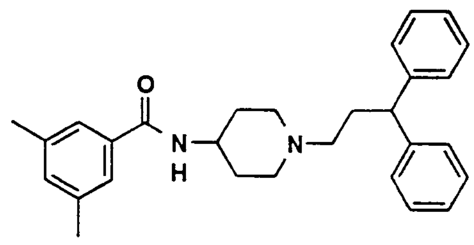

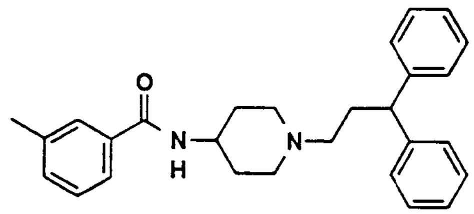

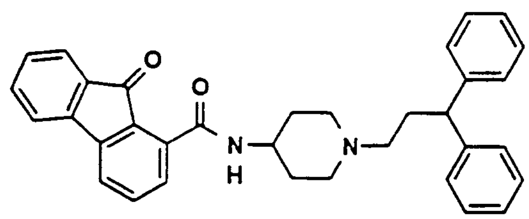

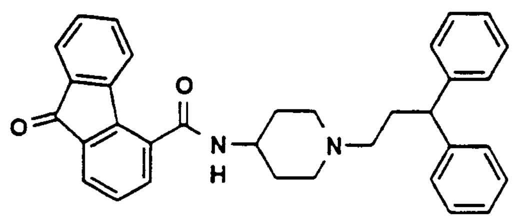

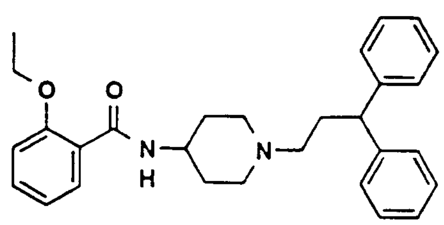

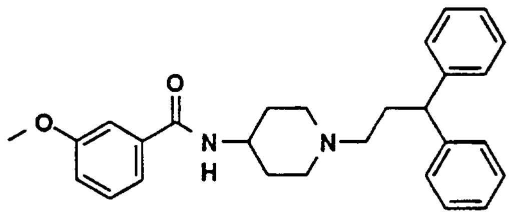

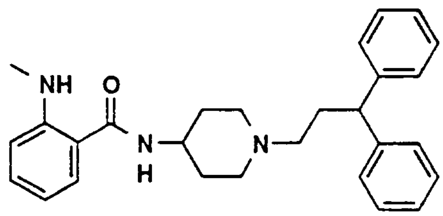

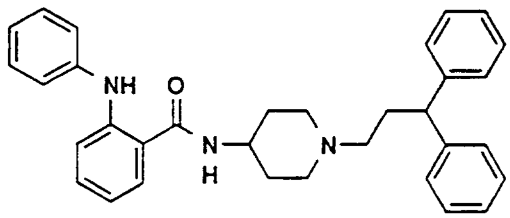

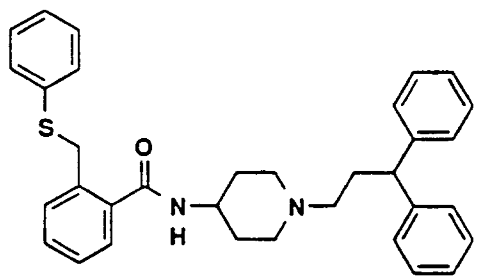

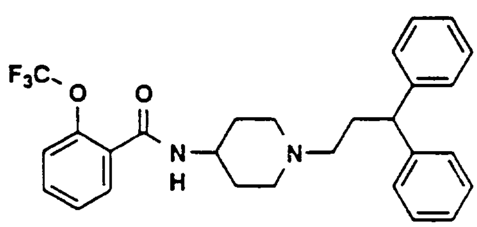

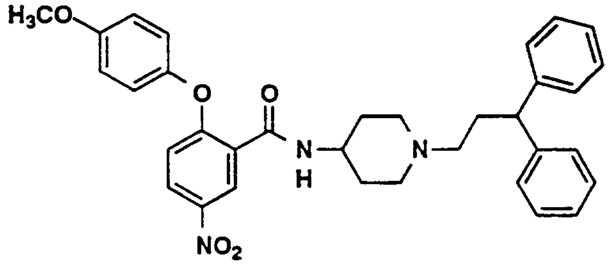

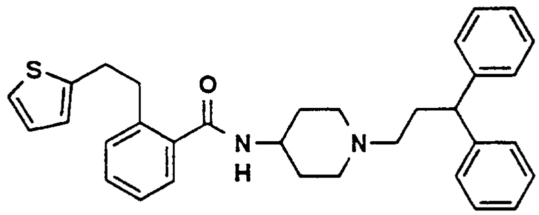

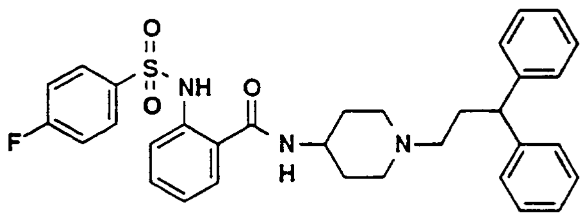

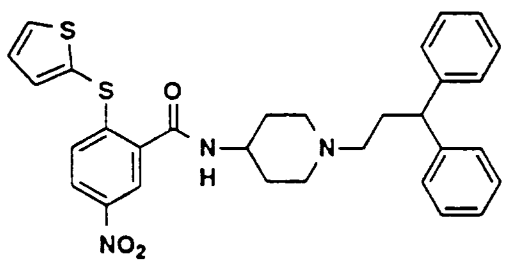

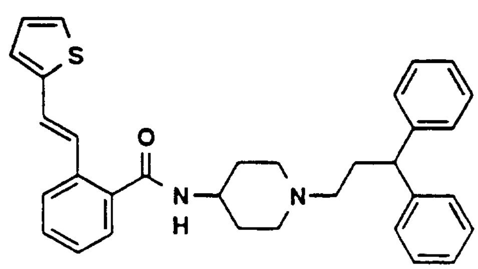

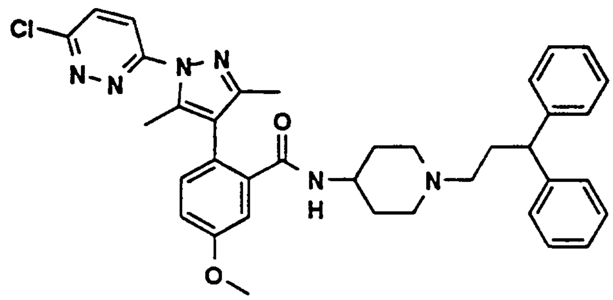

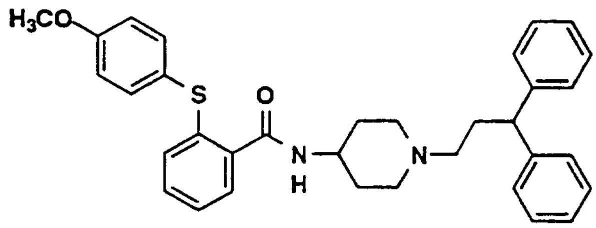

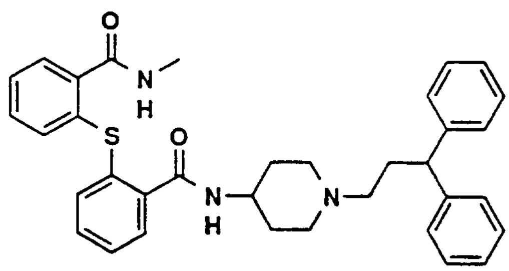

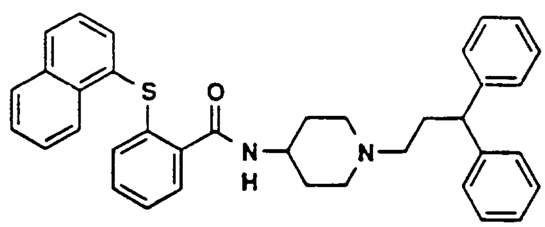

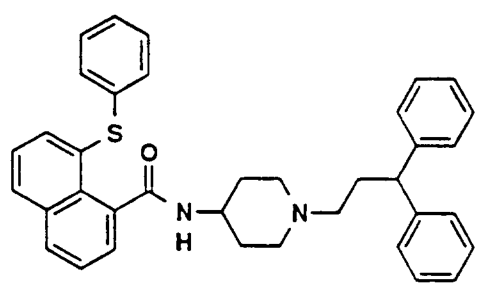

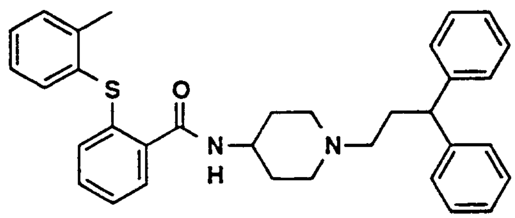

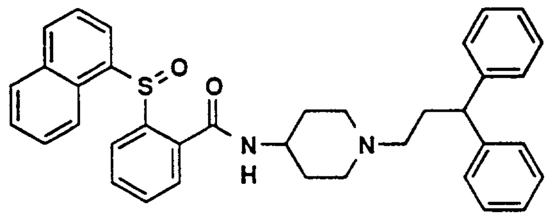

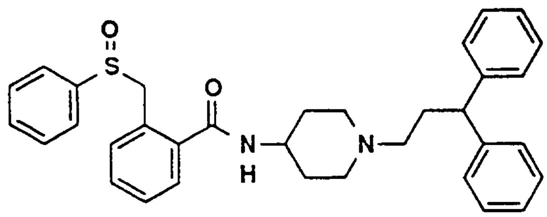

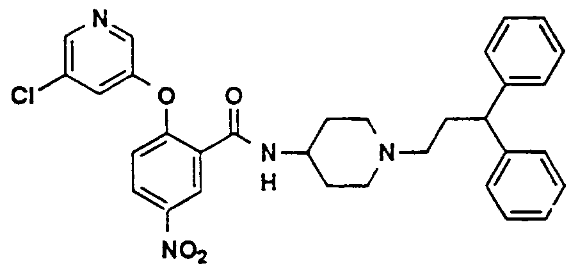

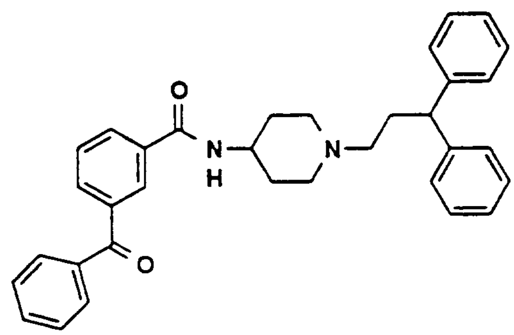

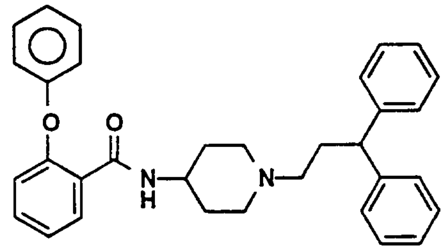

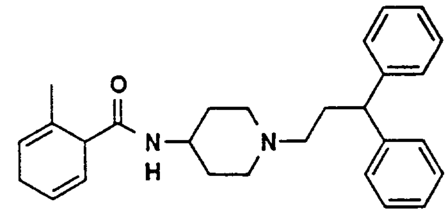

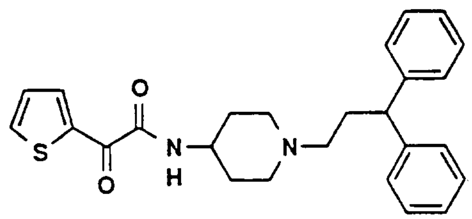

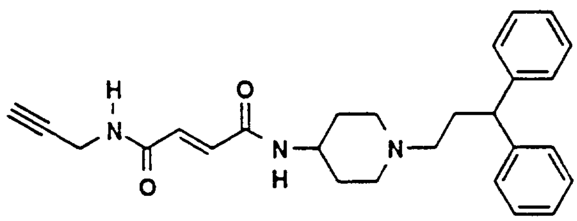

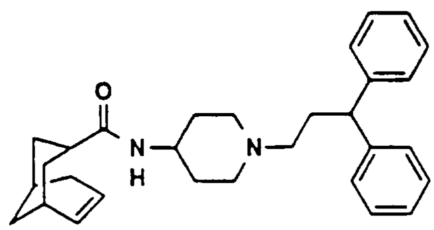

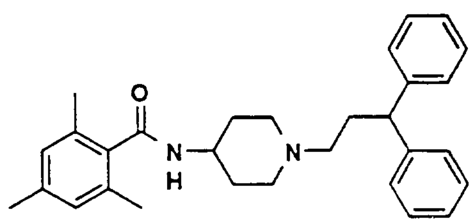

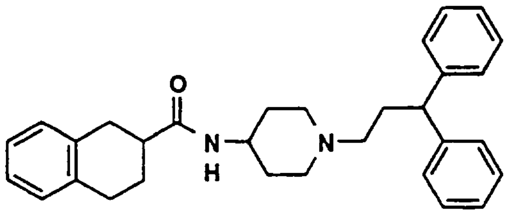

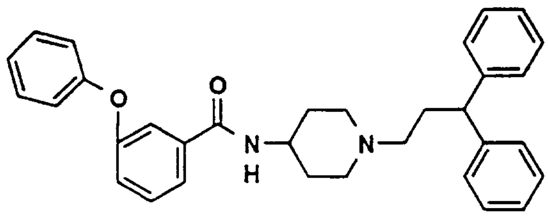

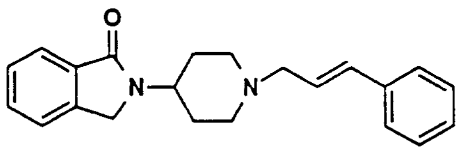

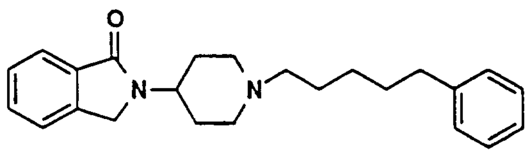

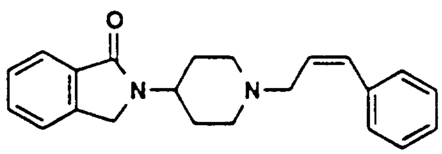

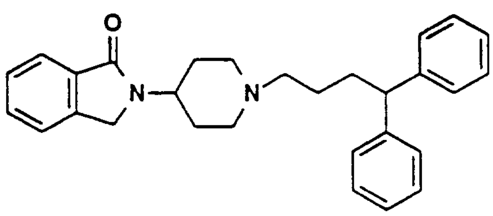

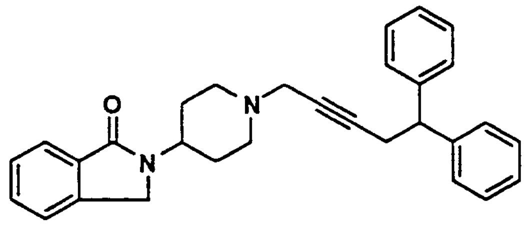

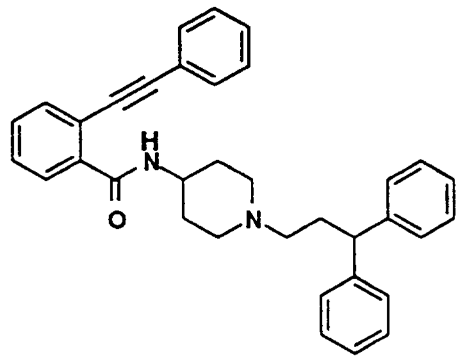

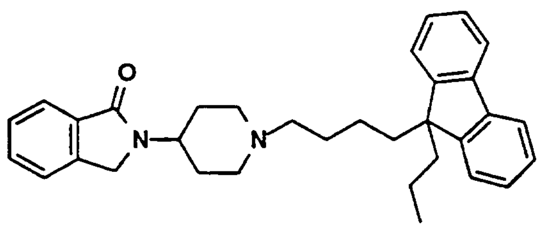

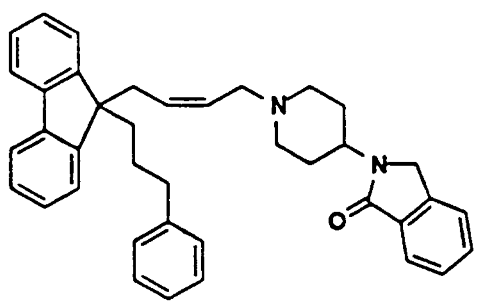

or a pharmaceutically acceptable salt thereof.

**15.** A compound which has the structure

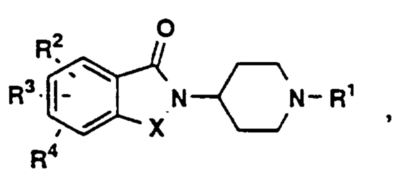

where X is: $CHR^8$,

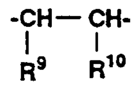 or 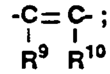

$R^8$, $R^9$ and $R^{10}$ are independently hydrogen, alkyl, alkenyl, alkynyl, aryl, arylalkyl, heteroaryl, heteroarylalkyl, cycloalkyl, or cycloalkylalkyl;

$R^1$ is alkyl, alkenyl, alkynyl, aryl, heteroaryl, arylalkyl wherein alkyl has at least 2 carbons, diarylalkyl, arylalkenyl, diarylalkenyl, arylalkynyl, diarylalkynyl, diarylalkylaryl, heteroarylalkyl wherein alkyl has at least 2 carbons, cycloalkyl, or cycloalkylalkyl wherein alkyl has at least 2 carbons, all optionally substituted through available carbon atoms with 1, 2, or 3 groups selected from halo, alkyl, haloalkyl, alkenyl, alkoxy, aryloxy, aryl, arylalkyl, alkylmercapto, arylmercapto, cycloalkyl, cycloalkylalkyl, heteroaryl, fluorenyl, heteroarylalkyl, hydroxy or oxo;

or $R^1$ is a group of the structure

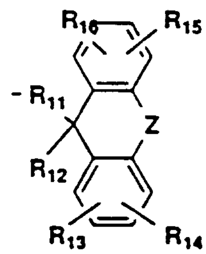

$R^{11}$ is a bond, alkylene, alkenylene or alkynylene of up to 6 carbon atoms; arylene or mixed arylene-alkylene; $R^{12}$ is hydrogen, alkyl, alkenyl, aryl, heteroaryl, haloalkyl, arylalkyl, arylalkenyl, cycloalkyl, aryloxy, alkoxy, arylalkoxy, heteroarylalkyl or cycloalkylalkyl; Z is bond, O, S, N-alkyl, N-aryl, or alkylene or alkenylene from 1 to 5 carbon atoms $R^{13}$, $R^{14}$, $R^{15}$, and $R^{16}$ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, carboxy, aminocarbonyl, alkylcarbonyloxy, alkylcarbonylamino, arylalkyl, heteroaryl, heteroarylalkyl or aryloxy;

or $R^1$ is a group of the structure

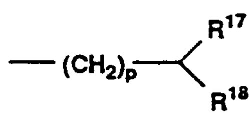

wherein p is 1 to 8 and $R^{17}$ and $R^{18}$ are each independently H, alkyl, alkenyl, aryl, arylalkyl, heteroaryl, heteroarylalkyl, cycloalkyl or cycloalkylalkyl, at least one of $R^{17}$ and $R^{18}$ is other than H;

or $R^1$ is a group of the structure

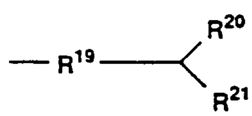

wherein

$R^{19}$ is aryl or heteroaryl;

$R^{20}$ is aryl or heteroaryl;

$R^{21}$ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;

$R^2$, $R^3$, $R^4$ are independently hydrogen, halo, alkyl, haloalkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl;

or pharmaceutically acceptable salts thereof, or prodrug esters thereof, with the proviso that where X is $CH_2$, and $R^2$, $R^3$ and $R^4$ are each H, then $R^1$ will be other than 3,3-diphenylpropyl.

**16.** The compound as defined in Claim 15 having the formula

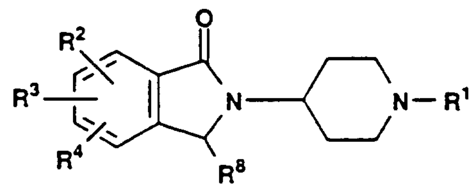

or

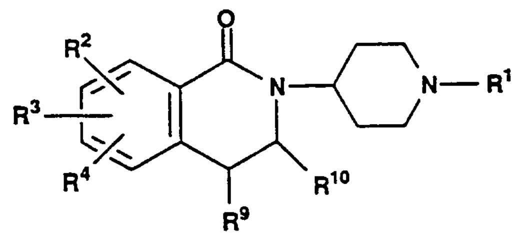

,

or

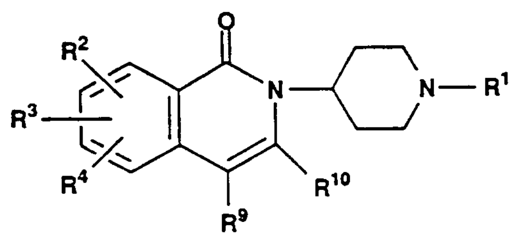

.

**17.** A compound which has the structure

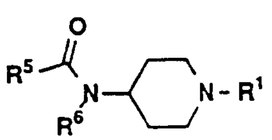

,

wherein $R^1$ is alkyl, alkenyl, alkynyl, aryl, heteroaryl, arylalkyl wherein alkyl has at least 2 carbons, diarylalkyl, arylalkenyl, diarylalkenyl, arylalkynyl, diarylalkynyl, diarylalkylaryl, heteroarylalkyl wherein alkyl has at least 2 carbons, cycloalkyl, or cycloalkylalkyl wherein alkyl has at least 2 carbons, all optionally substituted through available carbon atoms with 1, 2, or 3 groups selected from halo, alkyl, haloalkyl, alkenyl, alkoxy, aryloxy, aryl, arylalkyl, alkylmercapto, arylmercapto cycloalkyl, cycloalkylalkyl, heteroaryl, fluorenyl, heteroarylalkyl, hydroxy or oxo;

or $R^1$ is a group of the structure

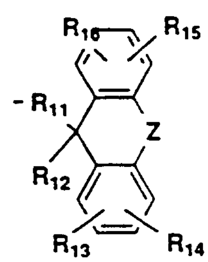

$R^{11}$ is a bond, alkylene, alkenylene or alkynylene of up to 6 carbon atoms; arylene or mixed arylene-

alkylene; $R^{12}$ is hydrogen, alkyl, alkenyl, aryl, heteroaryl, haloalkyl, arylalkyl, arylalkenyl, cycloalkyl, aryloxy, alkoxy, arylalkoxy, heteroarylalkyl or cycloalkylalkyl; Z is bond, O, S, N-alkyl, N-aryl, or alkylene or alkenylene from 1 to 5 carbon atoms $R^{13}$, $R^{14}$, $R^{15}$, and $R^{16}$ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, carboxy, aminocarbonyl, alkylcarbonyloxy, alkylcarbonylamino, arylalkyl, heteroaryl, heteroarylalkyl or aryloxy;

or $R^1$ is a group of the structure

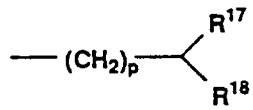

wherein p is 1 to 8 and $R^{17}$ and $R^{18}$ are each independently H, alkyl, alkenyl, aryl, arylalkyl, heteroaryl, heteroarylalkyl, cycloalkyl or cycloalkylalkyl, at least one of $R^{17}$ and $R^{18}$ is other than H;

or $R^1$ is a group of the structure

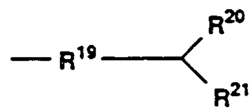

wherein

$R^{19}$ is aryl or heteroaryl;

$R^{20}$ is aryl or heteroaryl;

$R^{21}$ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;

$R^5$ is alkyl of at least 2 carbons, alkenyl, alkynyl, aryl, heteroaryl, arylalkyl, heteroarylalkyl, cycloalkyl, cycloalkylalkyl, polycycloalkyl, polycycloalkylalkyl, cycloalkenyl, cycloalkenylalkyl, polycycloalkenyl, polycycloalkenylalkyl, heteroarylcarbonyl, all optionally substituted through available carbon atoms with 1, 2, or 3 groups selected from hydrogen, halo, alkyl, haloalkyl, alkoxy, haloalkoxy, alkenyl, alkynyl, cycloalkyl, cycloalkylalkyl, cycloheteroalkyl, cycloheteroalkylalkyl, aryl, heteroaryl, arylalkyl, arylcycloalkyl, arylalkynyl, aryloxy, aryloxyalkyl, arylalkoxy, arylazo, heteroaryloxo, heteroarylalkyl, heteroarylalkenyl, heteroaryloxy, hydroxy, nitro, cyano, amino, thiol, alkylthio, arylthio, heteroarylthio, arylthioalkyl, alkylcarbonyl, arylcarbonyl, arylaminocarbonyl, alkoxycarbonyl, aminocarbonyl, alkynylaminocarbonyl, alkylaminocarbonyl, alkenylaminocarbonyl, alkylcarbonyloxy, arylcarbonyloxy, alkylcarbonylamino, arylcarbonylamino, arylsulfinyl, arylsulfinylalkyl, arylsulfonyl, alkylsulfonyl, arylsulfonylamino;

$R^6$ is hydrogen, $C_1$-$C_4$ alkyl or $C_1$-$C_4$ alkenyl;

or pharmaceutically acceptable salts thereof, or prodrug esters thereof.

**18.** A compound which has the structure

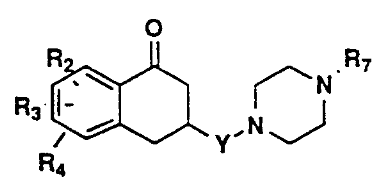

Y is $-(CH_2)_m-$ or

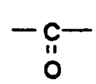

wherein m is 2 or 3;

$R^2$, $R^3$, $R^4$ are independently hydrogen, halo, alkyl, haloalkyl, alkenyl, alkoxy, aryloxy, aryl, arylalkyl,

alkylmercapto, arylmercapto, cycloalkyl, cycloalkylalkyl, heteroaryl, heteroarylalkyl, hydroxy or haloalkyl;

$R^7$ is alkyl, aryl or arylalkyl wherein alkyl by itself or part of arylalkyl is optionally substituted with oxo; or

pharmaceutically acceptable salts thereof, or prodrug esters thereof, with the provisos that where one of $R^2$, $R^3$ and $R^4$ is 6-fluoro, and the others are H, $R^7$ will be other than 4-O-methoxyphenyl.

**19.** A pharmaceutical composition comprising a compound as defined in anyone of claims 1-18 and a pharmaceutically acceptable carrier.

**20.** Use of a compound as defined in Claim 1 where in the compound $R^1$ also includes arylmethyl, heteroarylmethyl and cycloalkylmethyl, and Y also includes $-CH_2-$for the preparation of a pharmaceutical composition for preventing, inhibiting or treating atherosclerosis, pancreatitis or obesity in a mammalian species.

**21.** Use of a compound as defined in Claim 1 where in the compound $R^1$ also includes arylmethyl, heteroarylmethyl and cycloalkylmethyl, and Y also includes $-CH_2-$for the preparation of a pharmaceutical composition for lowering serum lipid levels, cholesterol and/or triglycerides, or inhibiting and/or treating hyperlipemia, hyperlipidemia, hyperlipoproteinemia, hypercholesterolemia and/or hypertriglyceridemia.

European Patent Office

# PARTIAL EUROPEAN SEARCH REPORT

which under Rule 45 of the European Patent Convention shall be considered, for the purposes of subsequent proceedings, as the European search report

Application Number: EP 94 11 3800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE-A-36 00 390 (HOECHST) 16 July 1987<br>* page 6 *<br>see RN 129488-29-5, 2-Propenamide, 3-(3,4-dihydroxyphenyl)-N-[1-(3,3-diphenylpropyl)-4- piperidinyl]-, monohydrochloride<br>--- | 1,4,17 | C07D401/04<br>C07D211/58<br>C07D295/10<br>C07D295/18<br>C07D401/12<br>C07D401/14<br>C07D405/12<br>C07D417/12<br>C07D409/12<br>A61K31/445 |
| X | GB-A-1 404 868 (JOHN WYETH) 3 September 1975<br>see RN 57642-94-1, Benzamide, N-[1-(4,4-diphenylbutyl)-4-piperidinyl]-<br>see RN 57642-93-0, Benzamide, N-[1-[4,4-bis(4-fluorophenyl)butyl]-4-piperidinyl]-<br>--- | 1,4,17 | |
| X | EP-A-0 354 568 (YOSHITOMI) 14 February 1990<br>see RN 128489-40-7, Benzenepropanamide, N-[1-(3,3-diphenylpropyl)-4-piperidinyl]-3,4- dihydroxy-<br>---<br>-/-- | 1,4,17 | |

TECHNICAL FIELDS SEARCHED (Int.Cl.6)

C07D

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 13 December 1994 | Kissler, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application Number
EP 94 11 3800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 445 862 (JANSSEN PHARMACEUTICA) 11 September 1991<br>* claim 1 *<br>see RN 137267-93-7, 7-Benzofurancarboxamide, N-[1-[5,5-bis(4-fluorophenyl)pentyl]-4- piperidinyl]-5-chloro-2,3-dihydro-2,2-dimethyl-, monohydrochloride<br>--- | 1,4,17 | |
| X | PATENT ABSTRACTS OF JAPAN<br>vol. 14, no. 310 (C-736) (4253) 4 July 1990<br>& JP-P-02 104 568 (YOSHITOMI) 17 April 1990<br>see RN 129488-29-5, 2-Propenamide, 3-(3,4-dihydroxyphenyl)-N-[1-(3,3-diphenylpropyl)-4- piperidinyl]-, monohydrochloride<br>--- | 1,4,17 | TECHNICAL FIELDS SEARCHED (Int.Cl.6) |
| D,P, X | EP-A-0 584 446 (SQUIBBS) 2 March 1994<br>* page 17 *<br>--- | 1-3,5,6, 8,10,16, 18-21 | |
| X | JOURNAL OF MEDICINAL CHEMISTRY.,<br>vol.17, no.7, 1974, WASHINGTON US<br>pages 739 - 744<br>see RN 52990-52-0, Benzamide, N-[1-[4,4-bis(4-fluorophenyl)-3-butenyl]-4-piperidinyl]-, monohydrochloride<br>see RN 52990-51-9, Benzamide, N-[1-(4,4-diphenyl-3-butenyl)-4-piperidinyl]-, monohydrochloride<br>* table II *<br>----- | 1,4,17 | |

EP 94 11 3800

-C-

INCOMPLETE SEARCH

Claims searched incompletely: 1-4,7-9,11-17,19-21

Claims not searched: 5,6,10,18

The definition of the following substituent(s) is too general and/or encompasses too broad a range of totally different chemical groups, only partly supported by examples given in the descriptive part of the application:.

R1, R5, R6, R7, X in the three generic formulae of claim 1

The number of theoretically conceivable compounds resulting from the combination of all claimed substituents of above list precludes a comprehensive search.
Guided by the spirit of the application and the inventive concept as disclosed in the descriptive part of the present application the structure search has been limited to the following case(s):
4-Acylamino-1-.omega.-diphenylalkylpiperidines where the 4-acylamino substituent may be part of a ring system.
This limited query yielded 85 compounds in the Registry File and 1105 references in CA.
The search report is limited to above query and is further exclusive of compounds of the Pimozide group (i.e. no cyclic urea structure).

(Cf. Arts. 83,84 EPC, Rule 45 EPC, Guidelines Exam. Part B, Chapt. III, 3.6,3.7).